# EUROPÄISCHE PATENTSCHRIFT

(11) **EP 1 930 396 B1**
(45) Veröffentlichungstag und Bekanntmachung des Hinweises auf die Patenterteilung: **06.07.2011**
(21) Anmeldenummer: 07021758.3
(22) Anmeldetag: 09.11.2007
(51) Int. Cl.: C09K 19/34

(54) **Furochromanderivate**
Furochroman derivatives
Dérivés de furochromane

(30) Priorität: 04.12.2006 EP 06025029
(43) Veröffentlichungstag der Anmeldung: 11.06.2008
(73) Patentinhaber: Merck Patent GmbH, 64293 Darmstadt (DE)
(72) Erfinder: Jansen, Axel, 64293 Darmstadt (DE); Taugerbeck, Andreas, 64285 Darmstadt (DE); Klasen-Memmer, Melanie, 67259 Heuchelheim (DE); Bernatz, Georg, 64289 Darmstadt (DE)

(56) Entgegenhaltungen:
- EP-A- 1 491 612
- DE-A1- 19 900 517
- DE-A1-102005 016 985
- DE-A1-102005 045 848
- DE-A1-102005 045 849

## Beschreibung

Die vorliegende Erfindung betrifft Furochromanderivate, bevorzugt mesogene Furochromanderivate, insbesondere flüssigkristalline Furochromanderivate, sowie diese Furochromanderivate enthaltende flüssigkristalline Medien. Ferner betrifft die vorliegende Erfindung Flüssigkristallanzeigen, insbesondere mittels einer aktiven Matrix angesteuerte Flüssigkristallanzeigen (AMDs oder AM LCDs nach englisch "active matrix addressed liquid crystal displays") und ganz insbesonders sogenannte VAN (englisch "vertically aligned nematics") Flüssigkristallanzeigen, einer Ausführungsform von ECB (von englisch "electrically controlled birefringence") Flüssigkristallanzeigen, bei denen nematische Flüssigkristalle mit einer negativen dielektrischen Anisotropie (Δε) verwendet werden.

In derartigen Flüssigkristallanzeigen werden die Flüssigkristalle als Dielektrika verwendet, deren optische Eigenschaften sich bei Anlegen einer elektrischen Spannung reversibel ändern. Elektrooptische Anzeigen, die Flüssigkristalle als Medien verwenden, sind dem Fachmann bekannt. Diese Flüssigkristallanzeigen verwenden verschiedene elektrooptische Effekte. Die gebräuchlichsten hiervon sind der TN-Effekt (englisch "twisted nematic"), mit einer homogenen, nahezu planaren Ausgangsorientierung des Flüssigkristalldirektors und einer um ca. 90° verdrillten nematischen Struktur), der STN-Effekt (englisch "super-twisted nematic") und der SBE-Effekt (englisch "supertwisted birefringence effect" mit einer 180° oder mehr verdrillten nematischen Struktur). Bei diesen und ähnlichen elektrooptischen Effekten werden flüssigkristalline Medien mit positiver dielektrischer Anisotropie (Δε) verwendet.

Neben den genannten elektrooptischen Effekten, welche Flüssigkristallmedien mit positiver dielektrischer Anisotropie benötigen, gibt es andere elektrooptische Effekte welche Flüssigkristallmedien mit negativer dielektrischer Anisotropie verwenden, wie z.B. der ECB-Effekt und seine Unterformen DAP (englisch "deformation of aligned phases"), VAN und CSH (englisch "color super homeotropics").

Ein elektrooptischer Effekt mit hervorragender, kleiner Blickwinkelabhängigkeit des Kontrasts verwendet axial symmetrische Micropixel (ASM von englisch "axially symmetric micro pixel"). Bei diesem Effekt ist der Flüssigkristall jedes Pixels zylinderförmig von einem Polymermaterial umgeben. Dieser Mode eignet sich besonders zur Kombination mit der Adressierung durch Plasmakanäle. So lassen sich insbesondere großflächige PA (englisch "plasma addressed") LCDs mit guter Blickwinkelabhängigkeit des Kontrasts realisieren.

Der in letzter Zeit verstärkt eingesetzte IPS-Effekt (englisch "in plane switching") kann sowohl dielektrisch positive wie auch dielektrisch negative Flüssigkristallmedien verwenden, ähnlich wie auch "guest/host"-Anzeigen, also Gast/Wirt-Anzeigen, die Farbstoffe je nach verwandtem Anzeigemodus entweder in dielektrisch positiven oder in dielektrisch negativen Medien einsetzen können.

Da bei Flüssigkristallanzeigen im allgemeinen, also auch bei Anzeigen nach diesen Effekten, die Betriebsspannung möglichst gering sein soll, werden Flüssigkristallmedien mit einem großen Absolutwert der dielektrischen Anisotropie eingesetzt, die in der Regel überwiegend und meist sogar weitestgehend aus Flüssigkristallverbindungen mit einer dielektrischen Anisotropie mit dem entsprechenden Vorzeichen bestehen. Also bei dielektrisch positiven Medien aus Verbindungen mit positiver dielektrischer Anisotropie und bei dielektrisch negativen Medien aus Verbindungen mit negativer dielektrischer Anisotropie. Bei den jeweiligen Arten von Medien (dielektrisch positiv bzw. dielektrisch negativ) werden typischer Weise allenfalls nennenswerte Mengen an dielektrisch neutralen Flüssigkristallverbindungen eingesetzt. Flüssigkristallverbindungen mit dem der dielektrischen Anisotropie des Medium entgegengesetzten Vorzeichen der dielektrischen Anisotropie werden in der Regel äußerst sparsam oder gar nicht eingesetzt.

Eine Ausnahme bilden hier flüssigkristalline Medien für MIM-Anzeigen (englisch "metal insultator metal", Simmons, J.G., Phys. Rev. 155, 3, 657-660 und Niwa, J.G. et al., SID 84 Digest, 304-307, Juni 1984) bei denen die Flüssigkristallmedien mit einer aktiven Matrix aus Dünnfilmtransistoren angesteuert werden. Bei dieser Art von Ansteuerung, welche die nicht lineare Kennlinie der Diodenschaltung ausnutzt, kann im Gegensatz zu TFT-Anzeigen kein Speicherkondensator gemeinsam mit den Elektroden der Flüssigkristallanzeigeelemente (Pixeln) aufgeladen werden. Somit ist zur Verminderung des Effekts des Spannungsabfalls während des Ansteuerzyklus ein möglichst großer Grundwert der Dielektrizitätskonstante erforderlich. Bei dielektrisch positiven Medien, wie sie z.B. bei MIM-TN-Anzeigen eingesetzt werden, muß also die Dielektrizitätskonstante senkrecht zur Molekülachse (ε_{⊥}) möglichst groß sein, da sie die Grundkapazität des Pixels bestimmt. Hierzu werden, wie z.B. in WO 93/01253, EP 0 663 502 und DE 195 21 483 beschrieben, in den dielektrisch positiven Flüssigkristallmedien, neben dielektrisch positiven Verbindungen, gleichzeitig auch Verbindungen mit negativer dielektrischer Anisotropie eingesetzt.

Eine weitere Ausnahme bilden STN-Anzeigen in denen z.B. nach DE 41 00 287 dielektrisch positive Flüssigkristallmedien mit dielektrisch negativen Flüssigkristallverbindungen eingesetzt werden um die Steilheit der elektrooptischen Kennlinie zu erhöhen.

Die Bildpunkte der Flüssigkristallanzeigen können direkt angesteuert werden, zeitsequentiell, also im Zeitmultiplexverfahren oder sie können mittels einer Matrix von aktiven Elementen mit nichtlinearen elektrischen Kennlinien angesteuert werden.

Die bislang gebräuchlichsten AMDs verwenden diskrete aktive elektronische Schaltelemente, wie z.B. dreipolige Schaltelemente wie MOS-Transistoren (englisch "metal oxide silicon") oder Dünnfilmtransistoren (TFTs von englisch "thin film transistors) oder Varistoren oder 2-polige Schaltelemente wie z.B. MIM-Dioden(englisch "metal insulator metal"), Ringdioden oder "back to back"-Dioden. Bei den TFTs werden verschiedene Halbleitermaterialien, überwiegend Silizium, aber auch Cadmiumselenid, verwendet. Insbesondere wird amorphes Silizium oder polykristallines Silizium verwendet.

Gemäß der vorliegenden Anmeldung sind Flüssigkristallanzeigen mit zur Flüssigkristallschicht senkrechtem elektrischen Feld und Flüssigkristallmedien mit negativer dielektrischer Anisotropie (Δε < 0) bevorzugt. Bei diesen Anzeigen ist die Randorientierung der Flüssigkristalle homeotrop. Im voll durchgeschalteten Zustand, also bei Anliegen einer entsprechend großen elektrischen Spannung, ist der Flüssigkristalldirektor parallel zur Schichtebene orientiert.

Chromanderivate und ihre Verwendung als Komponente in Flüssigkristallmischungen werden in der Druckschrift EP 1 491 612 A1 beschrieben.

Die Verwendung von Benzofuranen bzw. Dihydrobenzofuranen in Flüssigkristallmischungen wird in der Druckschrift DE 199 00 517 A1 beschrieben.

Tetrahydrofurochromene werden in der Druckschrift DE 102005045849 A1 in ihrer Herstellung beschrieben. Weitere tricyclische O-Heterocyclen als Flüssigkristallkomponente werden in der Druckschrift DE 102005016985 A1 und DE 102005045848 A1 offenbart.

Weiterhin wird in der Literatur [M. Bremer, L. Lietzau, New. J. Chem. 2005, 29, 72-74] darauf hingewiesen, dass in auf der 2,3-Difluorphenyleinheit basierten Flüssigkristallen durch die Einführung einer am aromatischen Ring fixierten Alkoxyseitenkette, wie z.B. in Benzofuranen bzw. Dihydrobenzofuranen, Verbindungen mit einer vergleichsweise höheren Polarität erhalten werden.

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

Eine Aufgabe der vorliegenden Erfindung ist es daher, Verbindungen mit vorteilhaften Eigenschaften für den Einsatz in flüssigkristallinen Medien zur Verfügung zu stellen. Bevorzugt sollen sie über eine negative dielektrische Anisotropie verfügen (Δε < 0), was sie besonders geeignet macht für den Einsatz in flüssigkristallinen Medien für VA-Displays.

Um beispielsweise in VA-TFT-Displays zufriedenstellende Eigenschaften, insbesondere geringe charakteristische Spannungen, zu gewährleisten, werden Substanzen mit einem großen Absolutwert der dielektrischen Anisotropie (Δε), einem der jeweiligen Anwendung entsprechenden Wert der optischen Anisotropie (Δn) und guter Stabilität gegen UV, Wärme und elektrischer Spannung benötigt.

Dies wird erreicht durch den Einsatz der erfindungsgemäßen Verbindungen der Formel I 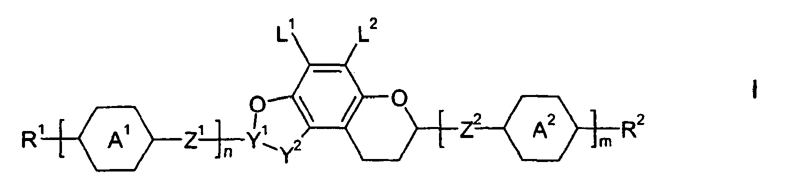 worin
- R¹ und R²: jeweils unabhängig voneinander H, Halogen, -CN, -SCN, -SF₅, -CF₃, -CHF₂, -CH₂F, -OCF₃, -OCHF₂ oder eine Alkylgruppe mit 1 bis 15 C-Atomen, die optional einfach durch CN oder CF₃ oder mindestens einfach durch Halogen substituiert sein kann, wobei jeweils auch eine oder mehrere CH₂-Gruppen, jeweils unabhängig voneinander, durch -O-, -S-, -CH=CH-, -CF=CF-, -CF=CH-, -CH=CF-, 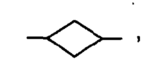 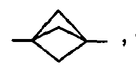 -CO-, -CO-O-, -O-CO- oder -O-CO-O- so ersetzt sein können, dass weder O-noch S-Atome direkt miteinander verknüpft sind, bevorzugt einer der Reste R¹ und R² Alkyl und Alkoxy mit 1 bis 12 C-Atomen, Alkoxyalkyl, Alkenyl oder Alkenyloxy mit 2 bis 12 C-Atomen und der andere, unabhängig vom ersten, ebenfalls Alkyl und Alkoxy mit 1 bis 12 C-Atomen, Alkoxyalkyl, Alkenyl oder Alkenyloxy mit 2 bis 12 C-Atomen oder auch F, Cl, Br, -CN, -SCN, -SF₅, -CF₃, -CHF₂, -CH₂F, -OCF₃ oder -OCHF₂
- >Y¹-Y²-: >C=CH- oder >CH-CH₂-, bevorzugt >C=CH-,
- L¹ und L²: F, 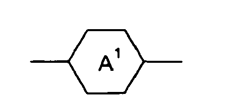
und 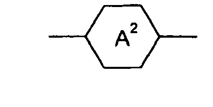 jeweils unabhängig voneinander und wenn mehrfach vorhanden auch diese unabhängig voneinander,
(a) einen trans-1,4-Cyclohexylenrest, worin auch eine oder zwei nicht benachbarte CH₂-Gruppen durch -O- und/oder -S-ersetzt sein können,
(b) einen 1,4-Cyclohexenylenrest,
(c) einen 1,4-Phenylenrest, worin auch eine oder zwei nicht benachbarte CH-Gruppen durch N ersetzt sein können,
(d) einen Rest ausgewählt aus der Gruppe Naphthalin-2,6-diyl, Decahydronaphthalin-2,6-diyl und 1,2,3,4-Tetrahydronaphthalin-2,6-diyl, oder
(e) einen Rest ausgewählt aus der Gruppe 1,4-Bicyclo-[2,2,2]-octylen, 1,3-Bicyclo-[1,1,1]-pentylen und Spiro-[3,3]-heptan-2,6-diyl,
   wobei in
   (a) und (b) eine oder mehrere -CH₂- Gruppen, unabhängig voneinander, jeweils durch eine -CHF- oder eine -CF₂-Gruppe ersetzt sein können und in
   (c) und (d) eine oder mehrere -CH= Gruppen, unabhängig voneinander, jeweils durch eine Gruppe ausgewählt aus der Gruppe -CF=, -CCI=, -CBr=, -C(CN)=, -C(CH₃)=, -C(CH₂F)=, -C(CHF₂)=, -C(OCH₃)=, -C(OCHF₂)= und -C(OCF₃)=, bevorzugt eine -CF= Gruppe, ersetzt sein können und bevorzugt 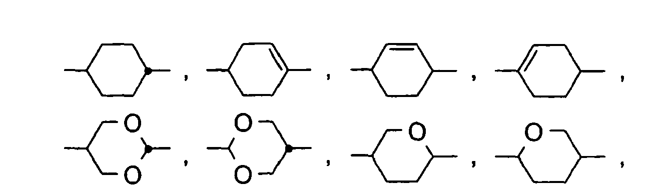 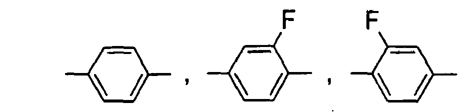 oder 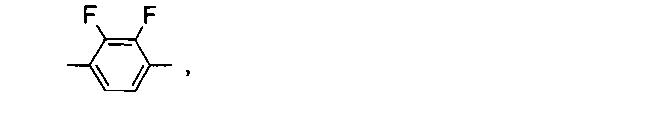 besonders bevorzugt 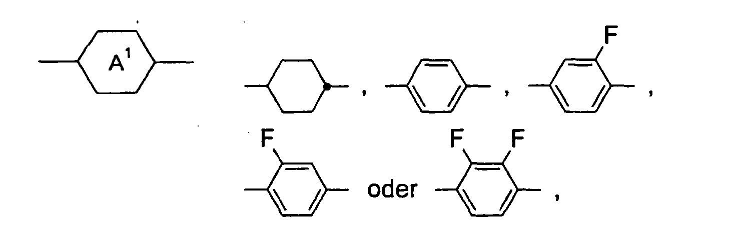 besonders bevorzugt

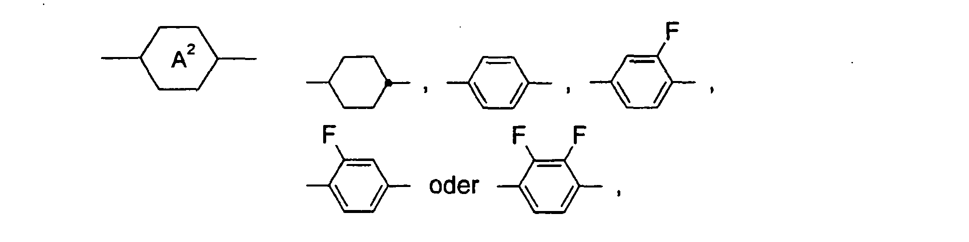

- Z¹ und Z²: jeweils unabhängig voneinander und, wenn mehrfach vorhanden, auch diese unabhängig voneinander, eine Einfachbindung, -CH₂-CH₂-, -CF₂-CH₂-, -CH₂-CF₂-, -CF₂-CF₂-, -CH=CH-, -CF=CF-, -CF=CH-, -CH=CF-, -C=C-, -COO-, -OCO-, -CH₂O-, -OCH₂-, -CF₂O-, -OCF₂-, oder eine Kombination von zweien dieser Gruppen, wobei keine zwei O-Atome miteinander verbunden sind,
- bevorzugt: -(CH₂)₄-, -CH₂-CH₂-, -CF₂-CF₂-, -CH=CH-, -CF=CF-, -C=C-, -CH₂O-, -CF₂O- oder eine Einfachbindung, besonders bevorzugt -CH₂O-, -CH₂-CH₂-, -CF₂-CF₂-, -CF=CF-, -CF₂O- oder eine Einfachbindung, und
- n und m: jeweils 0, 1, 2 oder 3, wobei
- (n + m): 0, 1, 2 oder 3, bevorzugt 0, 1 oder 2, besonders bevorzugt 0 oder 1
bedeuten.

Die erfindungsgemäßen Verbindungen der Formel I sind bevorzugt mesogene Verbindungen und besonders bevorzugt flüssigkristalline Verbindungen.

Besonders bevorzugt sind erfindungsgemäße Verbindungen der Formel I ausgewählt aus den Unterformeln IA und IB (mit IA: >Y¹-Y² = >C=CH-und IB: >Y¹-Y² - = >CH-CH₂-): 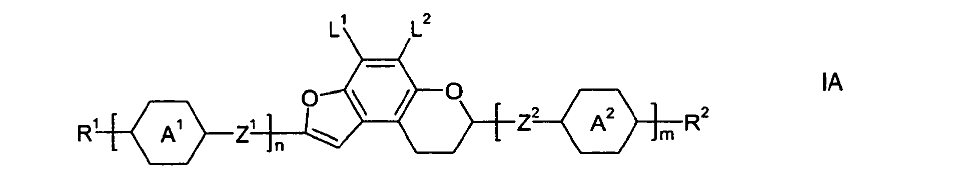 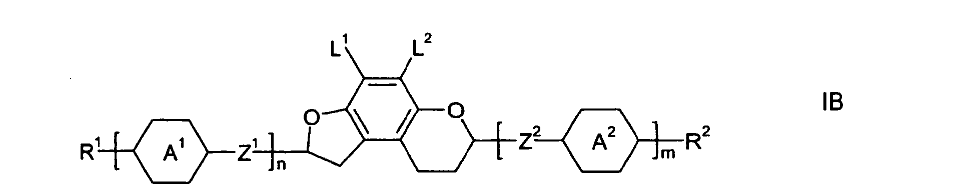 worin die Parameter die jeweiligen oben unter Formel I gegebenen Bedeutungen haben.

Bevorzugt sind Verbindungen der Formel I, bevorzugt ausgewählt aus der Gruppe der Verbindungen der Formeln IA und IB, bei denen die Summe n + m 0, 1 oder 2, besonders bevorzugt 0 oder 1 beträgt.

Eine bevorzugte Ausführungsform stellen die Verbindungen der Formel I dar, bei denen die Summe n + m 1 beträgt und bevorzugt 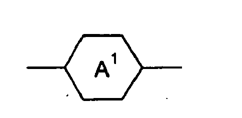 bzw. 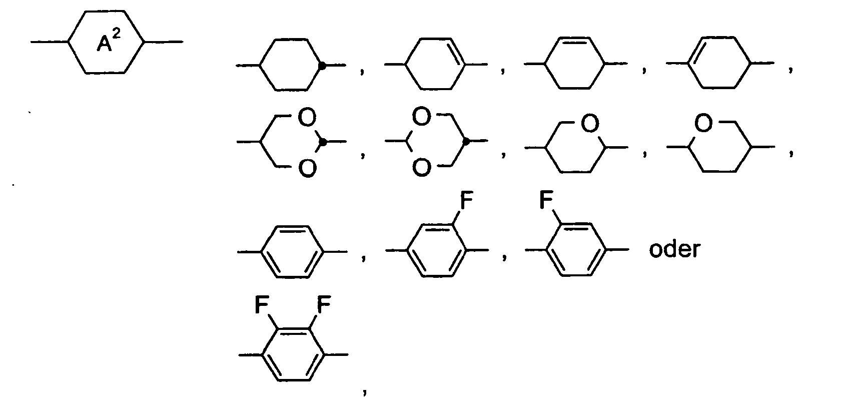 besonders bevorzugt 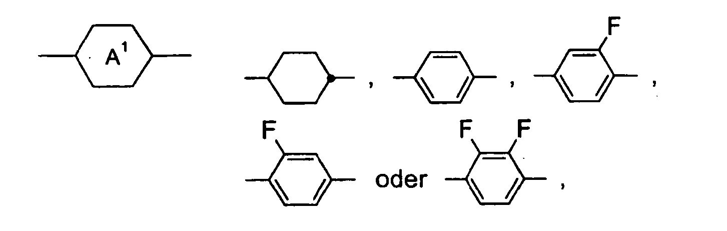 besonders bevorzugt 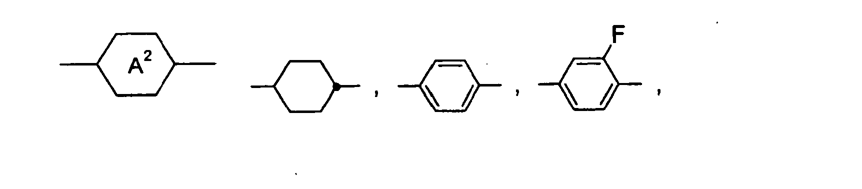 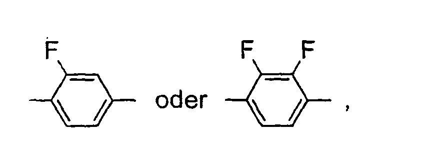
- Z¹, Z²: bevorzugt -(CH₂)₄-, -CH₂-CH₂-, -CF₂-CF₂-, -CH=CH-, -CF=CF-, -C=C-, -O-CH₂-, -O-CF₂- oder eine Einfachbindung, besonders bevorzugt -O-CH₂-, -CH₂-CH₂-, -CF₂-CF₂-, -CF=CF-, -O-CF₂- oder eine Einfachbindung bedeuten und
- L¹, L², R¹ und R²: die oben bei Formel I gegebenen Bedeutungen haben und
- L¹ und L²: bevorzugt F bedeuten.

Besonders bevorzugt sind Verbindungen der Formel I, bevorzugt ausgewählt aus der Gruppe der Verbindungen der Formeln IA und IB, bei denen
- n und m: beide 0 bedeuten und
L¹, L², R¹ und R² die oben bei der entsprechenden Formel gegebenen Bedeutungen haben.

Besonders bevorzugt sind Verbindungen der Formel IA ausgewählt aus der Gruppe der Verbindungen der Formeln IA-1 bis IA-11, bevorzugt der Formeln IA-1 IA-6, besonders bevorzugt der Formeln IA-1 bis IA-3, IA-5 und IA-6, bei denen mindestens eine der Gruppen R¹ und R² direkt mit dem Grundgerüst verknüpft ist: 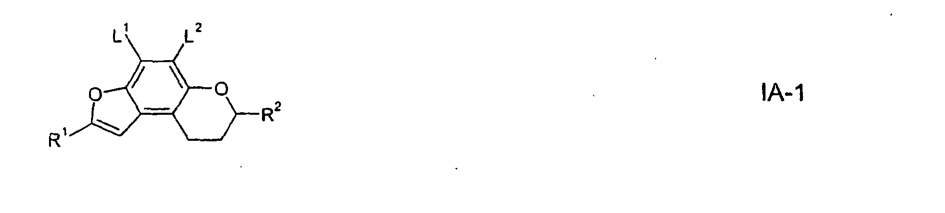 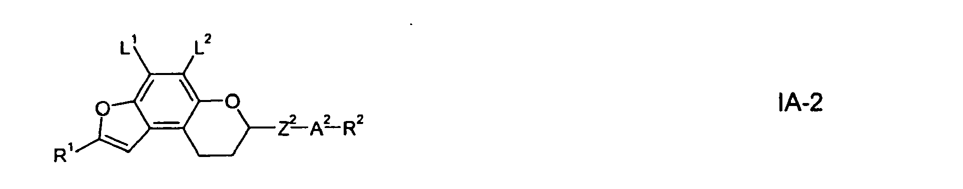 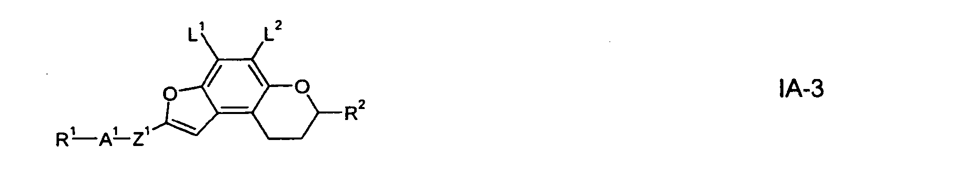 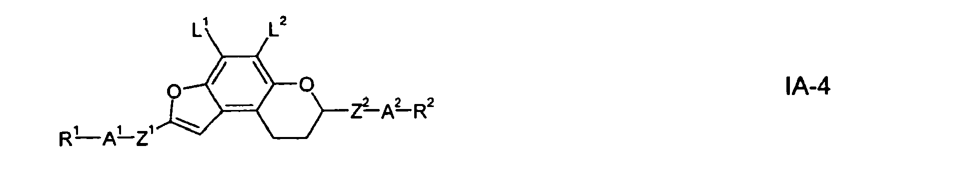 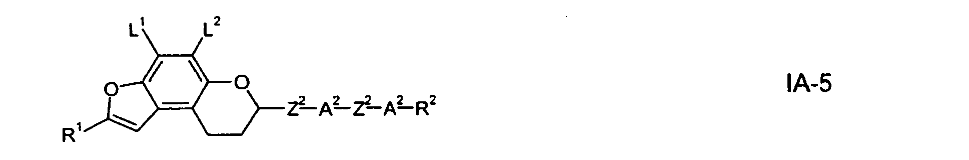 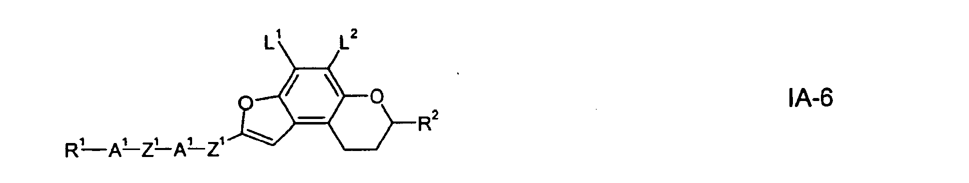 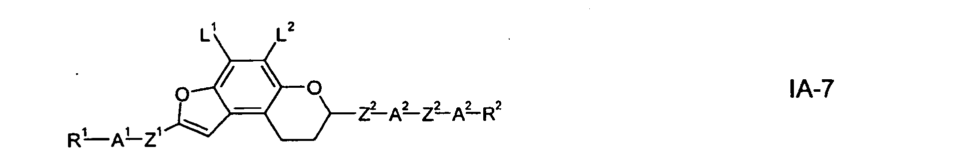 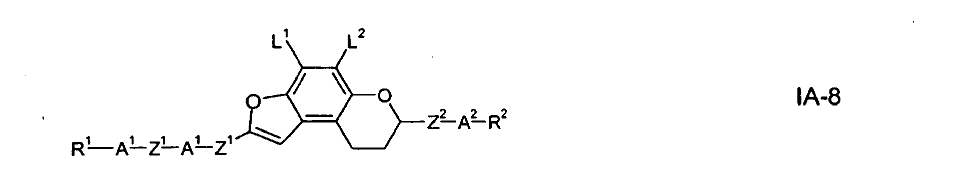 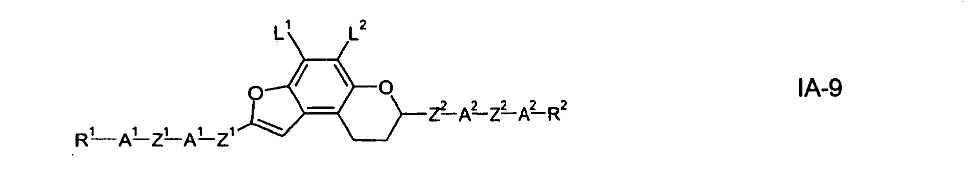 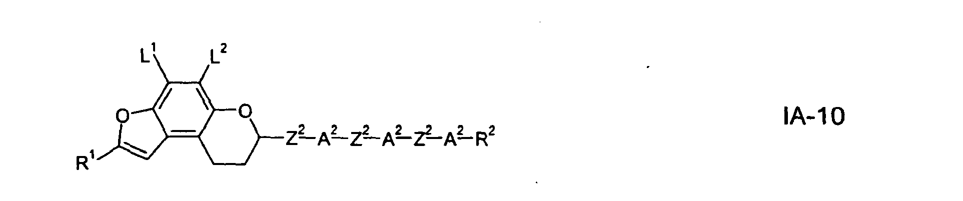 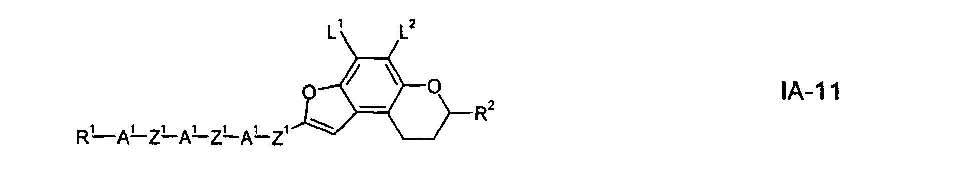 worin die Parameter die jeweilige oben gegebenen Bedeutungen haben.

Hier, wie in der gesamten vorliegenden Anmeldung, wird die Gruppierung der Teilformel I-1 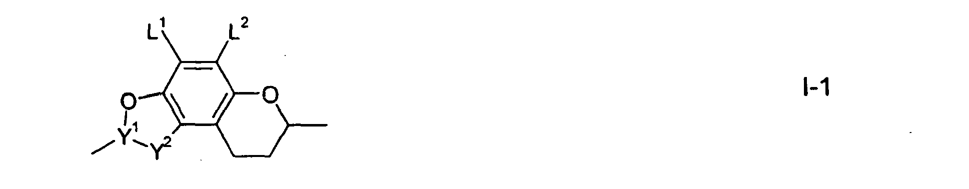 worin die Parameter die oben gegebenen Bedeutungen haben, und bevorzugt
- >Y¹-Y²-: >C=CH- bedeutet,
als das Grundgerüst der Verbindungen der Formel I, oder kurz als das Grundgerüst bezeichet.

Besonders bevorzugt sind Verbindungen der Formel IB ausgewählt aus der Gruppe der Verbindungen der Formeln IB-1 bis IB-11, bevorzugt der Formeln IB-1 bis IB-6, besonders bevorzugt der Formeln IB-1 bis IB-3, IB-5 und IB-6, bei denen mindestens eine der Gruppen R¹ und R² direkt mit dem Grundgerüst verknüpft ist: 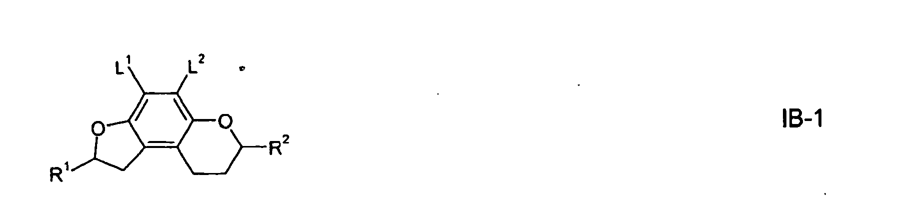 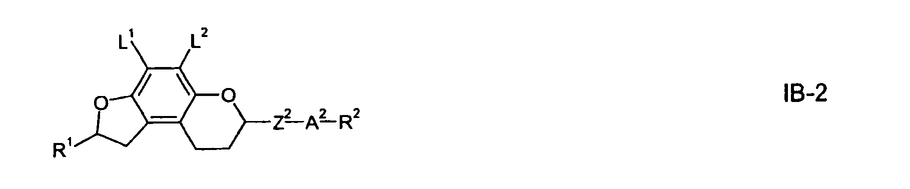 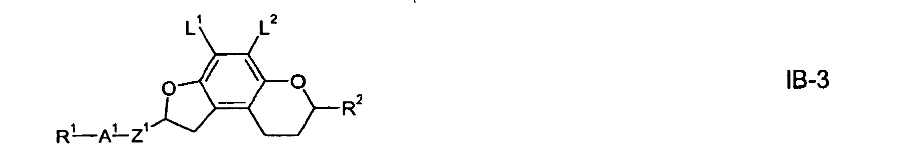 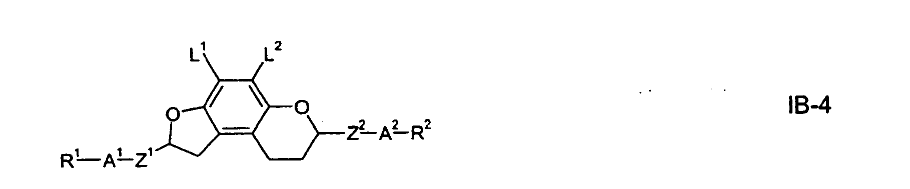 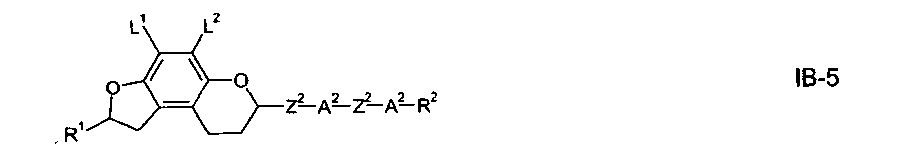 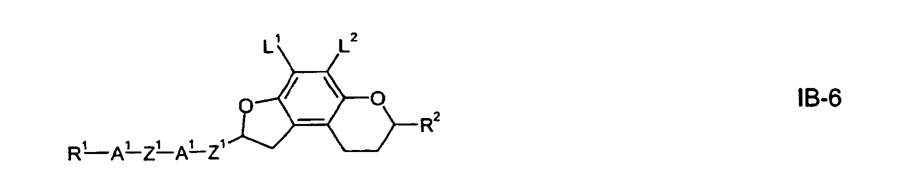 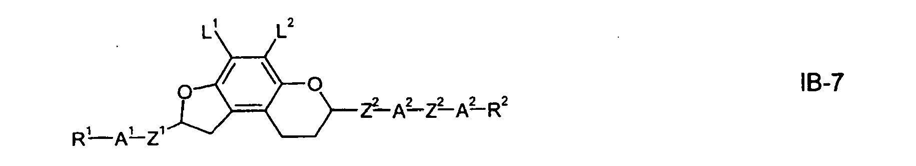 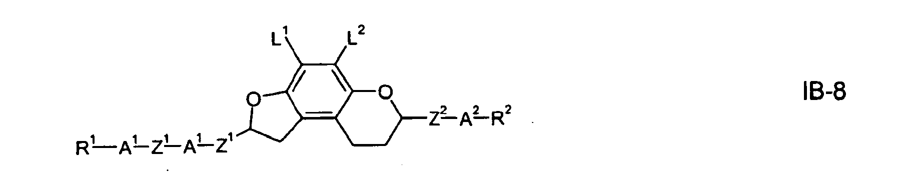 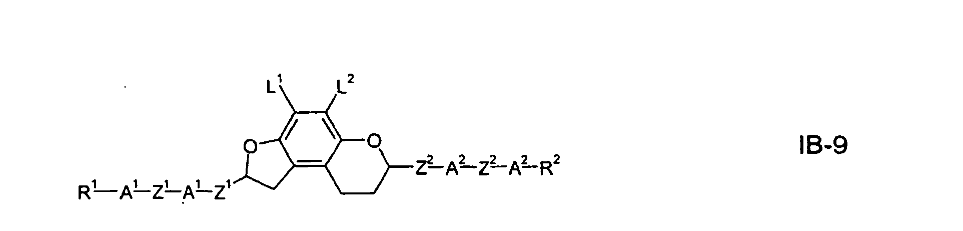 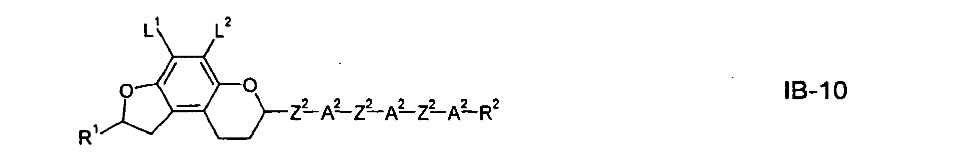 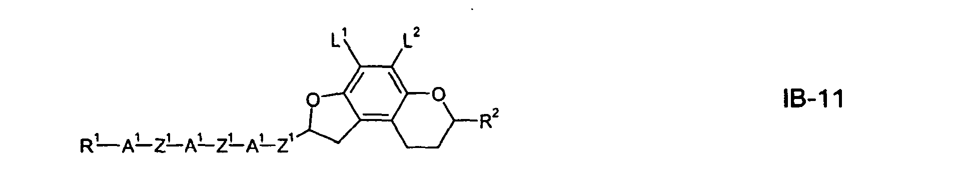 worin die Parameter die jeweilige oben gegebenen Bedeutungen haben.

Besonders bevorzugt sind Verbindungen der Formel I die einen oder mehrere Fluorsubstituenten, ganz besonders bevorzugt zwei Fluorsubstituenten, im Grundgerüst aufweisen.

Verbindungen der Formel I mit verzweigten Flügelgruppen R¹ und/oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien. Verbindungen der Formel I mit S_{A}-Phasen eignen sich beispielsweise für thermisch adressierte Displays.

Falls R¹ und/oder R² einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl, bzw. Alkoxyalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ und/oder R² einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ und/oder R² einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ und/oder R² einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ und/oder R² einen einfach durch CN oder CF₃ substituierten Alkyl-oder Alkenylrest bedeutet, so ist dieser Rest vorzugsweise geradkettig. Die Substitution durch CN oder CF₃ ist in beliebiger Position.

Falls R¹ und/oder R² einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verzweigte Gruppen enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ und/oder R² einen Alkylrest darstellt, in dem zwei oder mehr CH₂-Guppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Insbesondere bevorzugt sind Verbindungen der Formel I bei denen einer der Parameter m und n den Wert 0 und der andere einen Wert wie oben definiert, bevorzugt > 0 besitzt, sowie diese Verbindungen enthaltende Medien. Besonders bevorzugt ist daher m = 0 und n = 0, 1 oder 2 oder n = 0 und m = 0, 1 oder 2, wobei bevorzugt R¹ und/oder R² Methyl, Ethyl, Propyl, Butyl, Pentyl, Vinyl, 1 E-Propenyl, 1 E-Butenyl oder 1 E-Pentenyl bedeuten.

Insbesondere bevorzugt sind auch Verbindungen der Formel I die eine oder mehrere Alkenyl Substituenten tragen.

Die Verbindungen der Formel I können aufgrund asymmetrisch substituierter Kohlenstoffatome im Ring B als Stereoisomere vorliegen. Gegenstand der Erfindung sind sämtliche Isomere, sowohl in reiner Form, als Racemat und auch als Mischung von Diastereomeren oder Enantiomeren. Optisch aktive Verbindungen der Formel I können auch als Dotierstoffe in Flüssigkristallmischungen verwendet werden.

In den folgenden Schemata werden die Verbindungen der Formel IA kurz als Verbindungen 1 und die der Formel IB kurz als Verbindungen **2** bezeichnet. Wegen der besseren Lesbarkeit der Formeln wird jedoch auf die eckigen Klammern und die Parameter n und m verzichtet. Dadurch können die Ringe A¹ und A² auch die Bedeutung einer Einfachbindung haben; sowie ggf. jeweils eine der Gruppen A¹-Z¹ bzw. A²-Z² auch doppelt auftreten, wobei die dann doppelt auftretenden Parameter jeweils unabhängig voneinander eine der angegebenen Bedeutungen haben können.

Die Synthese der Verbindungen der Formel I erfolgt konzeptionell auf zwei verschiedenen Wegen. Bei dem ersten Weg, Weg A, (vgl. Schema I) wird das Chromangerüst vorgegeben, und der Furananteil davon ausgehend aufgebaut. Hierfür werden geeignete substituierte 5-Halo-chroman-6-ole **7** (X = Br, I) als Synthesebausteine verwendet. *Sonogashira*-Kupplung mit entsprechend substituierten terminalen Alkinen **8** verläuft in der Regel mit direkter Cyclisierung und liefert die Verbindungen **1** der Formel I [DE 199 00 517 A1 und G. A. Gfesser, R. Faghih, Y. L. Bennani, M. P. Curtis, T. A. Esbenshade, A. A. Hancock, M, D. Cowart, Biorg. Med. Chem. Lett. 2005, 15, 2559-2563]. Je nach Beschaffenheit des Alkins **8** wird die Umsetzung zu den Verbindungen **1** vorteilhaft zweistufig durchgeführt. Hier werden unter den Bedingungen der *Sonogashira-*Kupplung zunächst die Verbindungen **9** als Zwischenprodukte erhalten. Die Cyclisierung erfolgt dann bei Behandlung mit Diethylzink [M. Nakmura, L. Ilies, S. Otsubo, E. Nakamura, Angew. Chem. 2006, 118, 958-961; Angew. Chem. Int. Ed. 2006, 45, 944-947]. 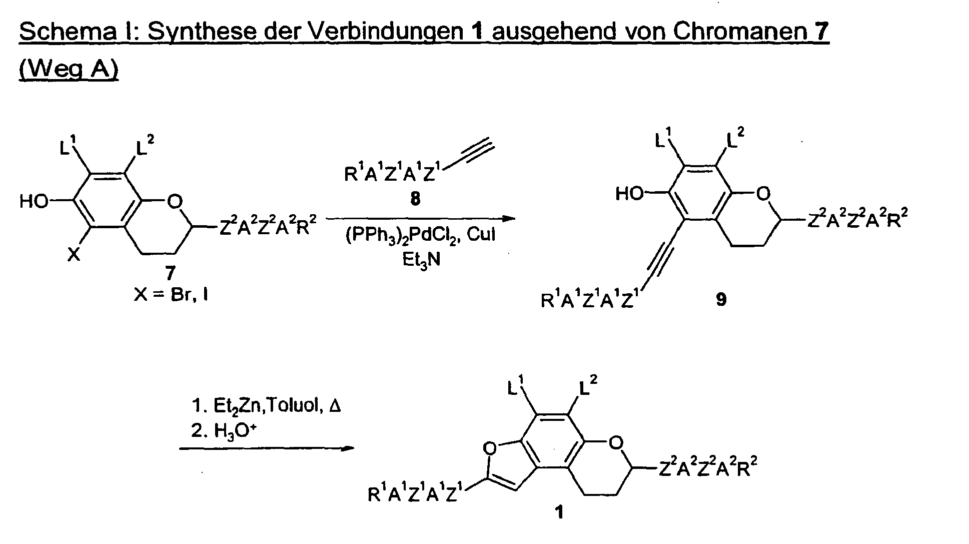 worin, wie in den folgenden Schemata, wenn nicht explizit anders angegeben, die Parameter die jeweilige oben gegebene Bedeutung haben.

Für den zweiten Weg, Weg B, (vgl. Schema II) werden in 2-Position substituierte Benzofuran-5-ole **10** als zentrale Zwischenstufen verwendet. Verbindungen vom Typ **1** werden dann durch Anellierung eines Pyranringes erhalten. Ausgehend von **10** kann dies entweder über eine *Claisen-Umlagerung* [H. Ishii, T. Ishikawa, S. Takeda, S. Ueki, M. Suzuki, Chem. Pharm. Bull. 1992, 40, 1148-1153] oder eine von *Wang* und *Finn* beschriebene Reaktion [Q. Wang, M. G. Finn, Org. Lett. 2000, 2, 4063-4065] erfolgen.

Die Edukte **14** für eine *Claisen-*Umlagerung werden aus **10** durch *Mitsunobu-*Veretherung [O. Mitsunobu, Synthesis 1981**,** 1] mit Propargylalkoholen **12** erhalten, welche z.B. durch Addition von Lithiumacetylid an einen entsprechenden Aldehyd zugänglich sind. Die Aryl-Proparyglether **14** untergehen beim Erhitzen in *N,N*-Diethylanilin eine [3.3]-sigmatrope Umlagerung zu den Chromenderivaten **15.** Diese können alternativ aus den Salicylaldehydderivaten **11** synthetisiert werden. Die Verbindungen **11** werden durch eine geeignete Formylierung (vgl. Schemall) der Benzofuran-5-ole **10** erhalten. Die Umsetzung zum Chroman **15** gelingt über eine Kupplung mit Vinylboronsäuren **13** [Q. Wang, M. G. Finn, Org. Lett. 2000, 2, 4063-4065]. Abschließend wird die Chromendoppelbindung der Verbindungen **15** unter milden Bedingungen selektiv hydriert. 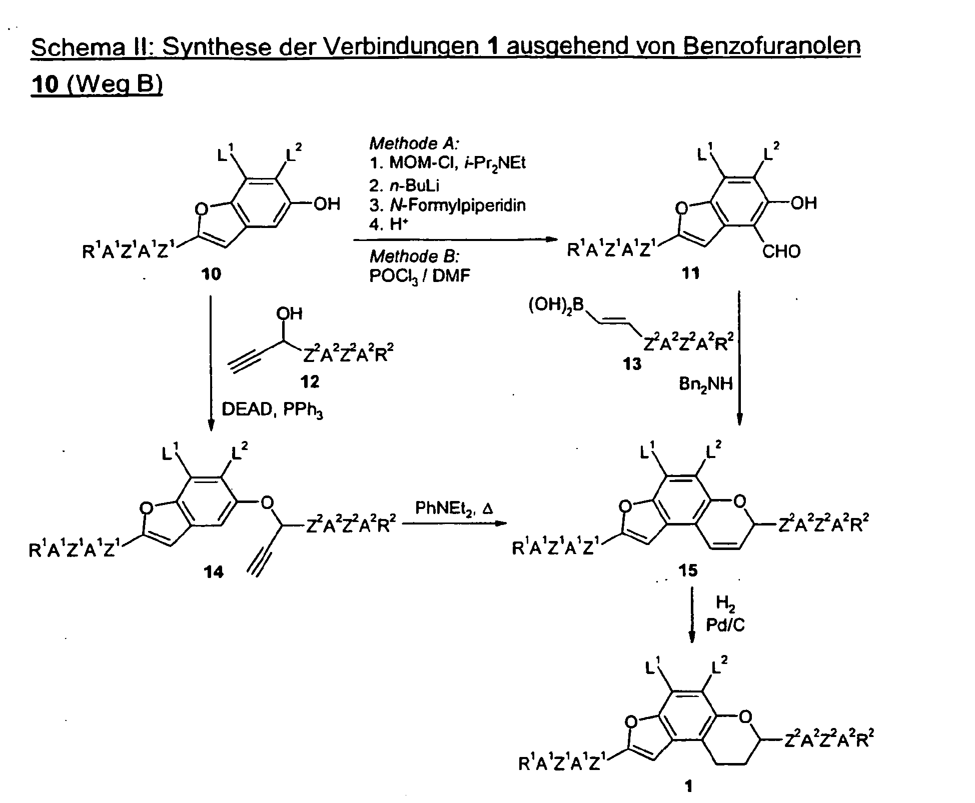 Verbindungen vom Typ **2** werden unmittelbar aus Verbindungen vom Typ **1** durch Hydrierung der 1,2-Doppelbindung erhalten (vgl. Schema III).

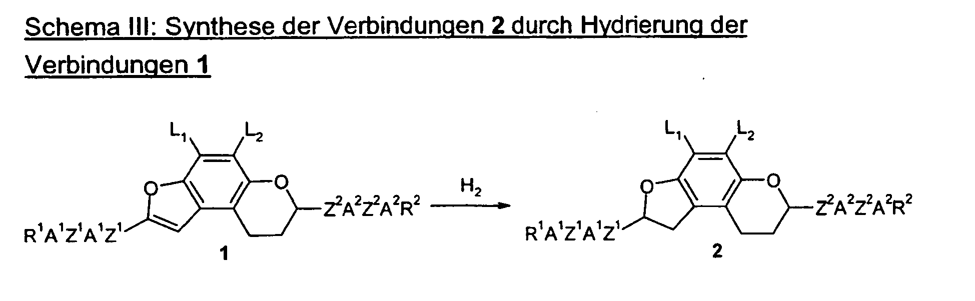

Alternativ können für die Synthese der Verbindungen **2** auch Dihydrobenzofuranole **16** als Ausgangsmaterialien herangezogen werden (vgl. Schema IV).

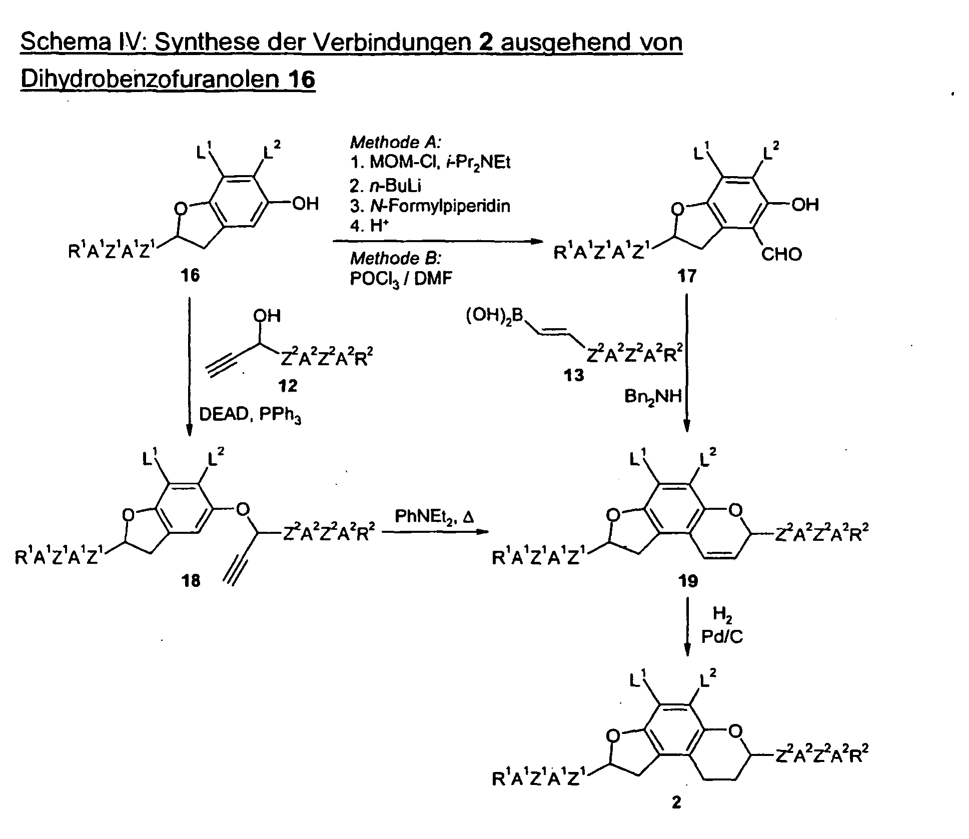

Die Synthese kann durch die Wahl geeigneter Edukte **7** und **8** (Weg A, vgl. Schema 1) bzw. **10** und **12** oder **13** (Weg B, vgl. Schema II) an die jeweils gewünschten Verbindungen der Formeln **1** angepasst werden. Die Verbindungen der Formel **2** werden dann entweder aus den Verbindungen **1** erhalten (vgl. Schema III) oder können durch Wahl geeigneter Edukte **16** und **12** oder **13** (vgl. Schema IV) an die jeweils gewünschten Verbindungen der Formel **2** angepasst werden.

Die Ausgangsmaterialien **8** (Weg A, vgl. Schema II), **12** und **13** (Weg B, vgl. Schema II und Schema IV) sind entweder kommerziell erhältlich, oder sie können bereits veröffentlichten Verfahren [z.B. Methoden der Organischen Chemie (Houben-Weyl), Georg Thieme Verlag, Stuttgart, New, York, 4. Aufl. 1993**]** folgend, synthetisiert werden.

Das Substitutionsmuster bzgl. der Reste L¹ und L² stellt u.U. besondere Anforderungen an die Synthese der Edukte **7** (Weg A, vgl. Schema I), **10** (Weg B, vgl. Schema II) und **16** (vgl. Schema IV).

7,8-Difluor-5-halo-chroman-6-ole **26** (X = Br, I, vgl. Schema VI) werden ausgehend von 2,3-Difluorphenol (**20**) oder 3,4-Difluor-2-hydroxybenzaldehyd (23) [N. J. Lawrence, L. A. Hepworth, D. Rennison, A. T. McGown, J. A. Hadfield, J. Fluorine Chem. 2003, 123,101-108 und E. Marzi, J. Gorecka, M. Schlosser, Synthesis 2004, 1609-161810] synthetisiert (vgl. Schema V).

Dazu wird aus 2,3-Difluorphenol **(20)** und einem Propargylalkohol **12** durch Mitsunobu-Veretherung [O. Mitsunobu, Synthesis 1981, 1-28**]** zunächst ein Propargyl-Arylether **21** gebildet, der unter geeigneten Reaktionsbedingungen eine thermische [3.3]-sigmatrope Umlagerung zu einem *2H-*Chromen eingeht. Diese Chromene können unter schonenden Bedingungen leicht zu den entsprechenden Chromanen **22** hydriert werden.

Alternativ werden diese 7,8-Difluorchromane **22** aus 3,4-Difluor-2-hydroxybenzaldehyd (**23**) [N. J. Lawrence, L. A. Hepworth, D. Rennison, A. T. McGown, J. A. Hadfield, J. Fluorine Chem. 2003, 123, 101-108 und E. Marzi, J. Gorecka, M. Schlosser, Synthesis 2004, 1609-1618] über eine von *Wang* und *Finn* [Q. Wang, M. G. Finn, Org. Lett. 2000, 2, 4063-4065] beschriebene Reaktion erhalten. Dabei werden aus Salicylaldehyden und Vinylboronsäuren in Gegenwart von Dibenzylamin *2H*-Chromene wie **24** mit hoher Ausbeute erhalten. Diese könne dann wiederum leicht zu den entsprechenden Chromanen **22** hydriert werden (s.o.).

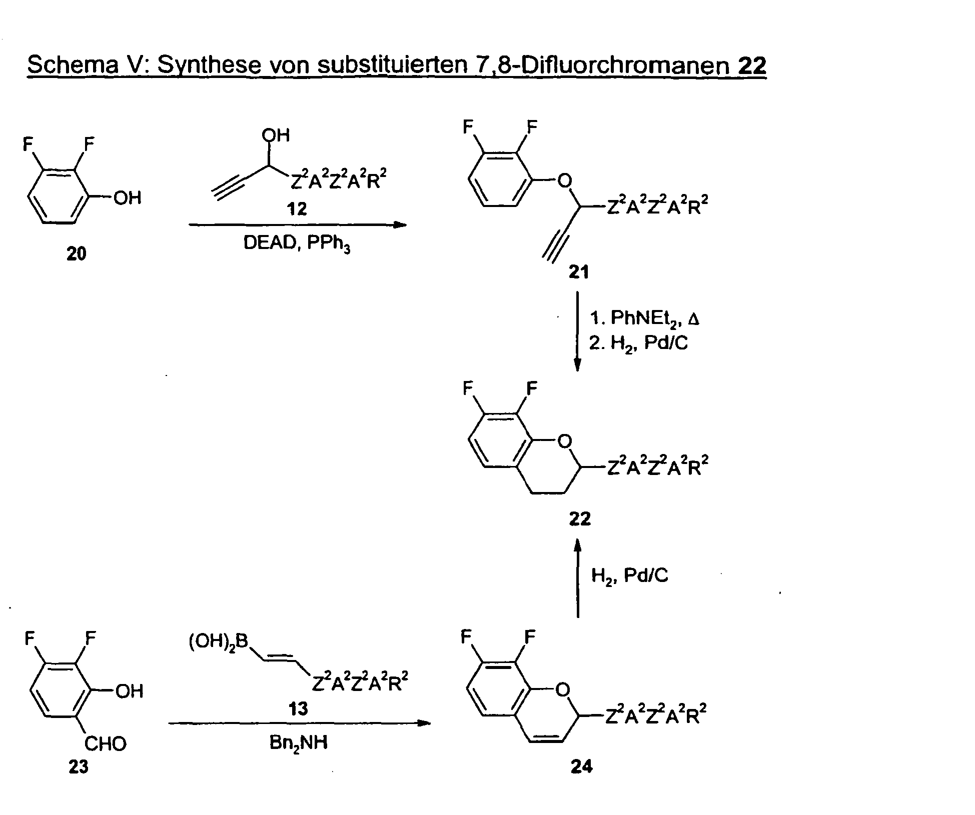

Die so erhaltenen Zwischenprodukte **22** werden durch *ortho*-Metallierung und Hydrolyse und Oxidation des *in situ* gebildeten Boronsäureesters zu Chromanolen **25** funktionalisiert (vgl. Schema VI). Die abschließende Halogenierung der 5-Position gelingt über die in Schema VI dargestellte Reaktionsfolge. Die aus den Verbindungen **25** hergestellten MOM-Ether werden mit *n*-BuLi *ortho*-metalliert und mit lod (bzw. Brom für X = Br) gequencht [E. Marzi, J. Gorecka, M. Schlosser, Synthesis 2004, 1609-1618 sowie R. C. Ronald, M. R. Winkle, Tetrahedron 1983, 39, 2031-2042 und M. Lang, W. Steglich, Synthesis 2005, 1019-1027]. Nach Abspaltung der MOM-Gruppe werden die gewünschten Zwischenprodukte **26** erhalten.

Schema VI: Synthese von 7,8-Difluor-5-halo-chroman-6-olen **26** (X = Br, I) 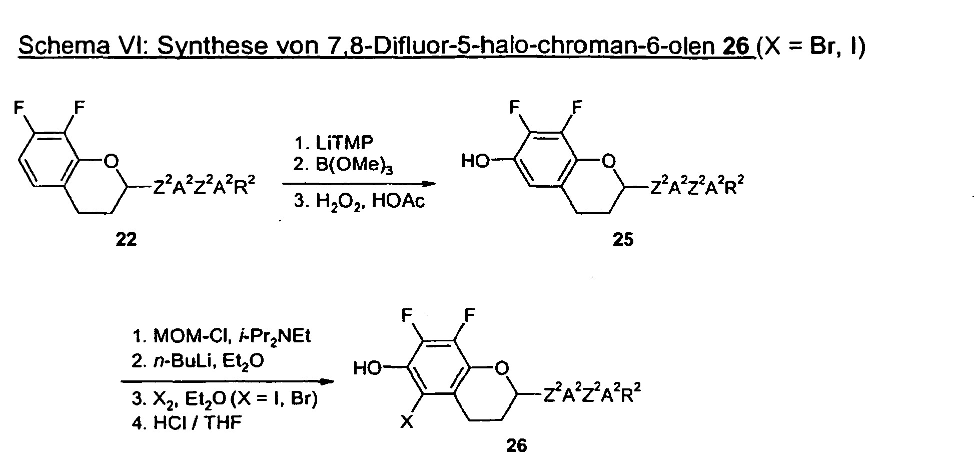 7-Fluor-5-halo-chroman-6-ole **31** (X = Br, I) können aus 5-Brom-4-fluor-2-hydroxy-benzaldehyd (**27**) [J. B. Blair et al., J. Med. Chem. 2000, 43, 4701-4710 und W. A. Caroll et al., J. Med. Chem. 2004, 47, 3163-3179] synthetisiert werden (vgl. Schema VII). Dieses Edukt **27** ist über literaturbekannte Verfahren aus 3-Fluorphenol durch *ortho*-selektive Formylierung [J. B. Blair et al., J. Med. Chem. 2000, 43, 4701-4710] und anschließende Bromierung [W. A. Caroll et al., J. Med. Chem. 2004, 47, 3163-3179] zugänglich.

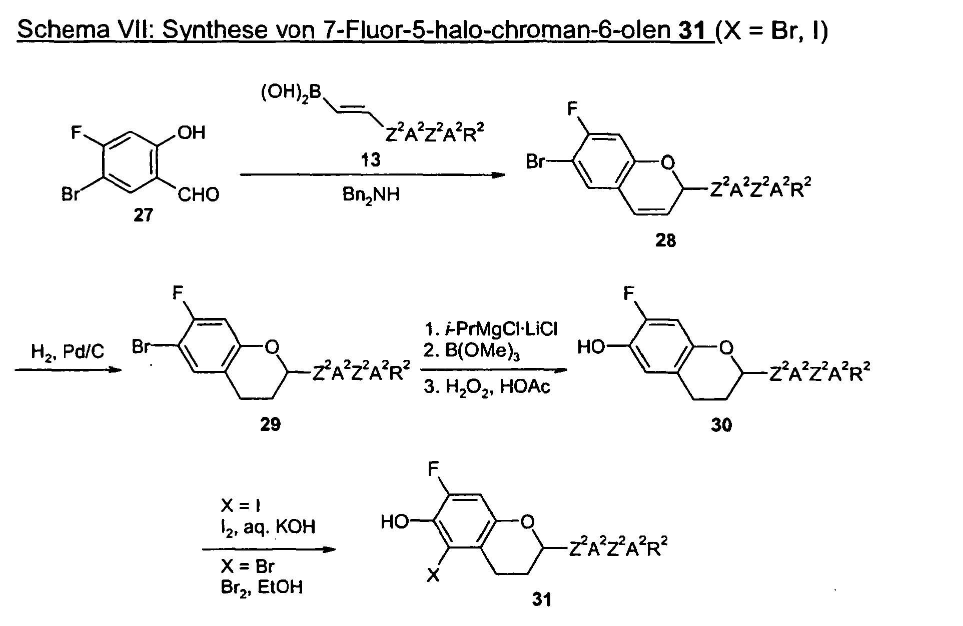

Ausgehend von 5-Brom-4-fluor-2-hydroxy-benzaldehyd **(27)** erfolgt der Aufbau der 7-Fluor-chromane **31** (vgl. Schema VII) wiederum vorteilhaft durch die schon beschriebene Kupplung mit Vinylboronsäuren [Q. Wang, M. G. Finn, Org. Lett. 2000, 2, 4063-4065] und anschließender Hydrierung. Die Funktionalisierung zum Chromanol **30** gelingt wie oben beschrieben, diesmal über die aus **29** hervorgehende *Grignard*-Verbindung. Die abschließende lodierung (bzw. Bromierung) kann wie oben über die MOM-Ether der Verbindungen **30** vorgenommen werden, gelingt aber vorteilhaft auch durch direkte lodierung [G. A. Gfesser, R. Faghih, Y. L. Bennani, M. P. Curtis, T. A. Esbenshade, A. A. Hancock, M, D. Cowart, Biorg. Med. Chem. Lett. 2005, 15, 2559-2563, M. Lang, W. Steglich, Synthesis 2005, 1019-1027 sowie C. W. Holzapfel, D. B. G. Williams, Tetrahedron 1995, 51, 8555-8564, K. J. Edgar, S. N. Falling, J. Org. Chem. 1990, 55, 5287-5291 und R. Johnsson, A. Meijer, U. Ellervik, Tetrahedron 2005, 61, 11567-11663] (bzw. Bromierung [B. F. Bonini, P. Carboni, G. Gottarelli, S. Masiero, G. P., Spada, J. Org. Chem. 1994, 59, 5930-5936]) der Chromanole **30.**

8-Fluor-5-halo-chroman-6-ole **36** (mit X = Br, I) werden ausgehend von 2-Fluor-4-bromphenol (**32**) erhalten. Hier wird der O-Heterocyclus bevorzugt durch eine *Claisen*-Umlagerung über den Propargylarylether **33** anelliert (vgl. SchemaVIII). Die Funktionalisierung zum Chromanol **35** gelingt mit der gleichen Reaktionsfolge wie für das Regioisomer **29.** Die Halogenierung von **35** liefert hauptsächlich die gewünschten Isomere **36** (X = Br, I), welche über labortypische Reinigungsverfahren von unerwünschten Regioisomeren abgetrennt werden können.

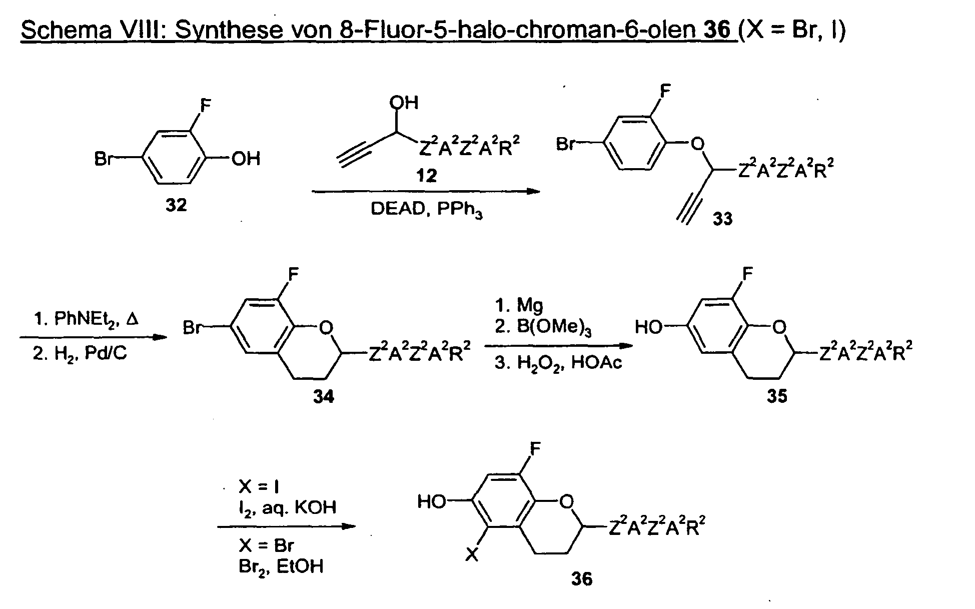

Alternativ können die Zwischenprodukte **34** auch ausgehend von 2-Fluor-4-bromphenol (**32**) über den Salicylaldehyd **37** synthetisiert werden. Dieser ist über eine *Duff*-Reaktion [M. L. Micklatcher, M. Cushman, Synthesis 1999, 1878-1880] aus **32** zugänglich. Die anschließende Synthese des Chromans **34** kann dann über die von *Wang* und *Finn* [Q. Wang, M. G. Finn, Org. Lett. 2000, 2, 4063-4065] beschriebene Prozedur und anschließender Hydrierung erfolgen (Schema IX).

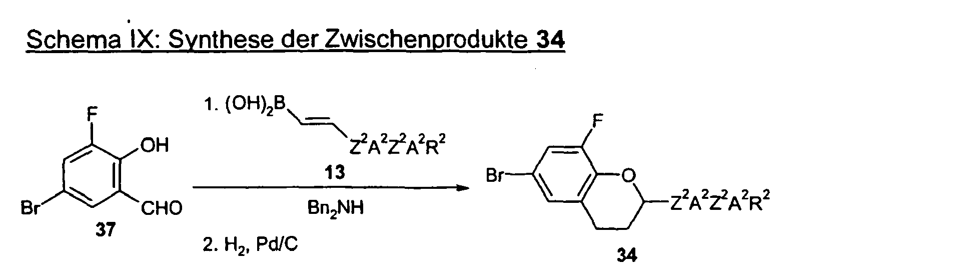

Nicht fluorierte Synthesebausteine **41** können ebenfalls durch die oben beschriebenen Methoden (*Claisen*-Umlagerung bzw. Kupplung mit Vinylboronsäuren) synthetisiert werden. Ein besonders bevorzugtes Verfahren geht aus von 2,5-Dihydroxybenzaldehyd **(38),** welcher nach literaturbekannten Verfahren [Y. Hu, C. Li, B. A. Kulkarni, G. Strobel, E. Lokovsky, R. M. Torczynski, J. A. Porco, Org. Lett. 2001, 3, 1649-1652] zunächst bromiert und selektiv geschützt wird (vgl. Schema X). Nach der zweistufigen Umsetzung zum Chroman **40** wird durch Abspaltung der TBS-Gruppe das Bromid **41** (X = Br) erhalten, welches als Edukt für die folgende *Sonogashira*-Kupplungen dient. In manchen Fällen ist die *Sonogashira*-Kupplung mit entsprechenden Aryliodiden **41** (X = I) besonders vorteilhaft. Diese Verbindungen **41** (X = I) sind ebenfalls aus dem Bromochroman **40** über die, in Schema X dargestellte Reaktionsfolge aus Halogen-Metallaustausch, Abfangen mit Iod und Abspaltung der Schutzgruppe mit Fluorid zugänglich. 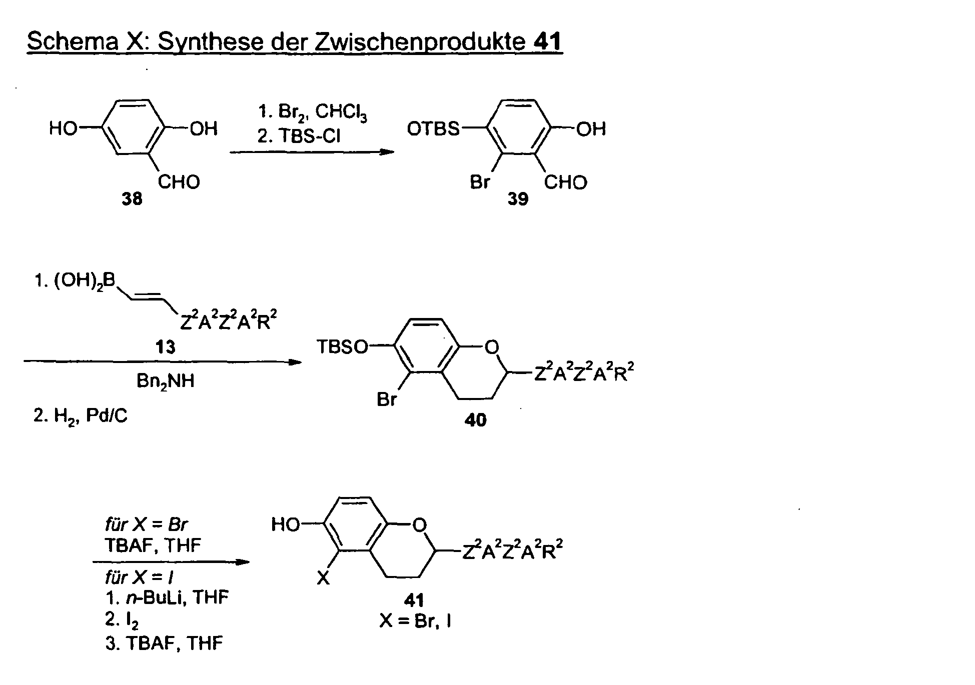 Geeignete, substituierte 6,7-Difluorbenzofuran-5-ole **46** und 6,7-Difluor-2,3-dihydrobenzofuran-5-ole **47** können folgendermaßen erhalten werden (Schema XI).

Schema XI: 6.7-Difluorbenzofuran-5-ole **46** und 6.7.Difluor-2,3-dihydrobenzofuran-5-ole **47** 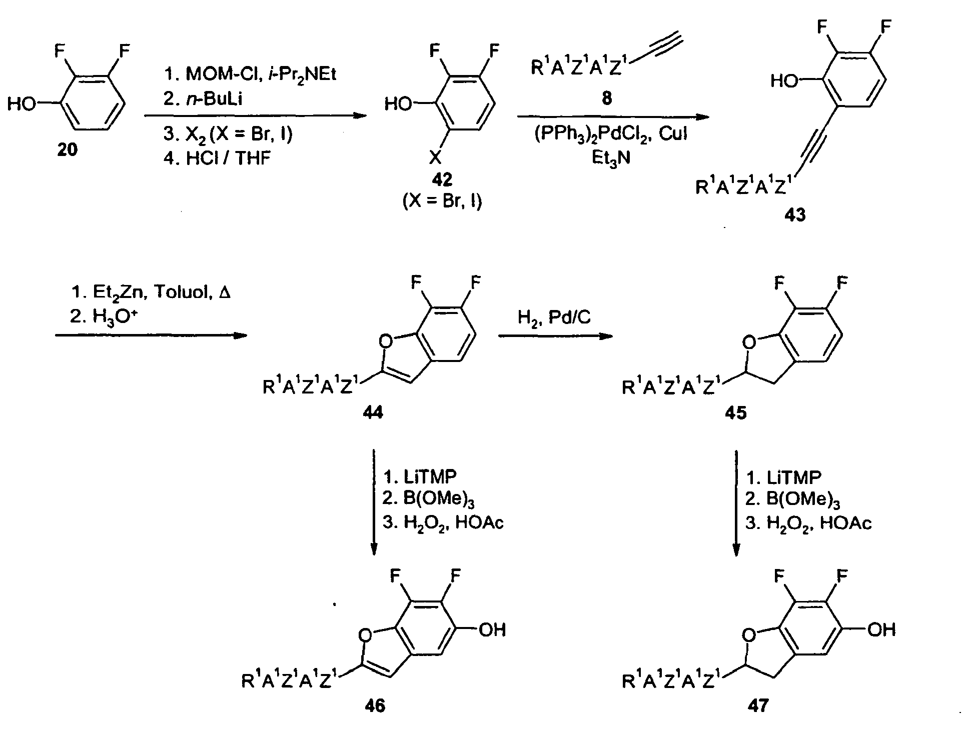 2,3-Difluor-6-halophenole **42** werden vorteilhaft über die schon erläuterte Reaktionsfolge aus 2,3-Difluorphenol (**20**) synthetisiert. Die in der 2-Position substituierten Benzofurane **44** werden entweder direkt in einer *Sonogashira*-Kupplung mit einem geeignten Alkin **8** gebildet, oder über die Zwischenstufe **43** und anschließender Cyclisierung mit Diethylzink (vgl. Schema I) erhalten. Diese Benzofurane **44** können leicht zu den entsprechenden Dihydrobenzofuranen **45** hydriert werden. Die für die in Schema II und Schema IV dargestellten Reaktionsfolgen erforderlichen 6,7-Difluorbenzofuran-5-ole **46** bzw. 6,7-Difluor-2,3-dihydrobenzofuran-5-ole **47** werden aus **44** bzw. **45** durch *ortho*-Metallierung, Hydrolyse und Oxidation des *in situ* gebildeten Boronsäureesters erhalten. 6-Fluorbenzofuran-5-ole **53** (mit L¹ = H und L² = F) bzw. 6-Fluor-2,3-dihydrobenzofuran-5-ole **54** (mit L¹ = H und L² = F), 7-Fluorbenzofuran-5-ole **53** (mit L¹ = F und L² = H) bzw. 7-Fluor-2,3-dihydrobenzofuran-5-ole **54** (mit L¹ = F und L² = H) und Benzofuran-5-ole **53** (mit L¹ = H und L² = H) bzw. 2,3-Dihydrobenzofuran-5-ole **54** (mit L¹ = H und L² = H) können auf konzeptionell ähnlichem Wege hergestellt werden. Im Unterschied zu oben werden entsprechende 4-Bromphenole **48** als Edukte gewählt. Die Funktionalisierung zu den Benzofuranolen **53** und **54** erfogt dann nicht durch *ortho*-Metallierung, sondern über die aus den Verbindungen **51** und **52** erhaltenen *Grignard*-Reagentien (vgl. Schema XII).

Schema XII: Synthese der Zwischenprodukte **53** und **54**

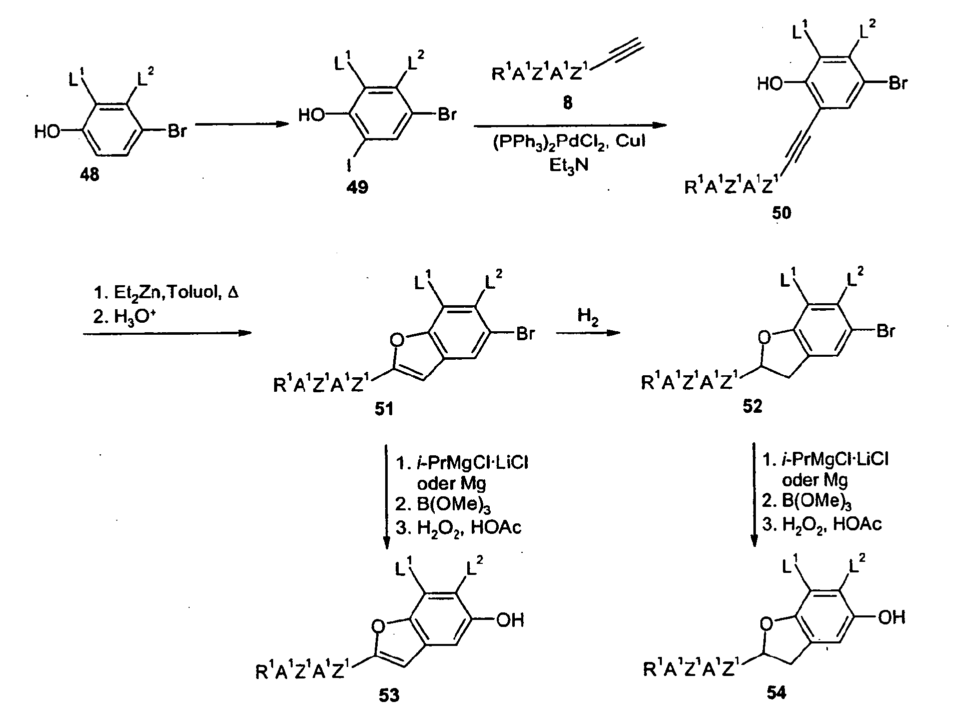

Für den speziellen Fall, dass R¹A¹Z¹A¹Z¹ Methyl und L¹ und L² F bedeuten, kann ein geeignet funktionalisiertes Benzofuran **60** besonders einfach über eine [3.3]-sigmatrope Umlagerung ausgehend von **56** hergestellt werden (vgl. Schema XIII). **56** wird aus 5-Brom-2,3-difluorphenol (**55**) und Propargylbromid hergestellt. Beim Erhitzen in *N,N-*Diethylanilin in Gegenwart von Cäsiumfluorid [H. Ishii, T. Ishikawa, S. Takeda, S. Ueki, M. Suzuki, T. Harayama, Chem. Pharm. Bull. 1990, 38, 1775-1777 und A. Chilin, P. Rodighiero, A. Guiotto, Synthesis 1998, 309-312] wird das Benzofuran **57** erhalten. Die Funktionalisierung zum Salicyaldehyd **60** gelingt dann über eine Kombination von zuvor schon erläuterten Standardverfahren. Anschließend kann wie oben in Schema II dargestellt verfahren werden.

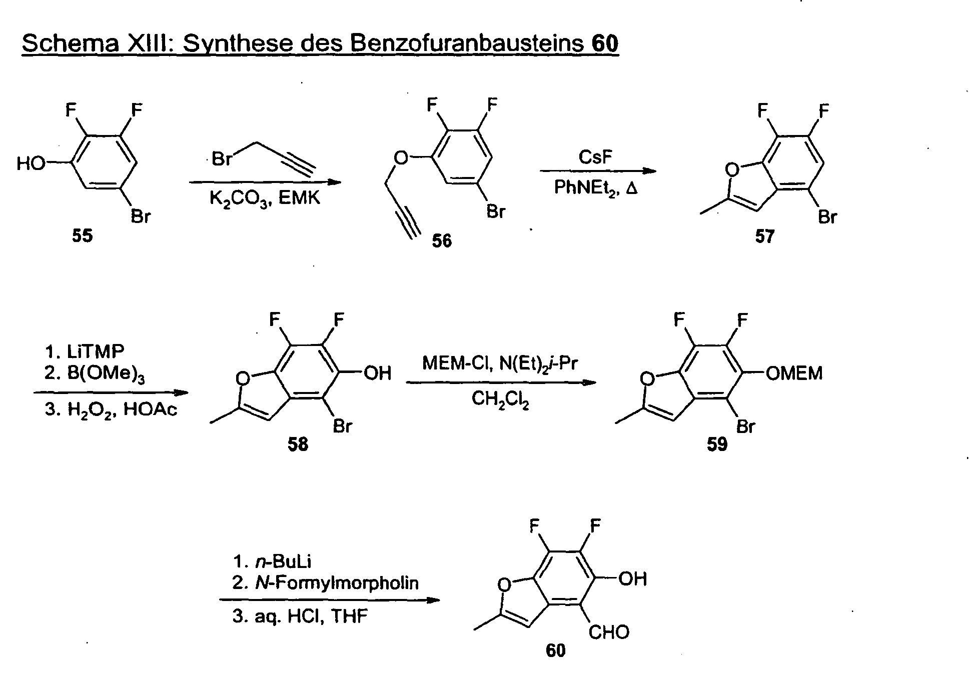

Für Verbindungen der Formel I (**1** und **2**) bei denen R¹A¹Z¹A¹Z¹ und / oder R²A²Z²A²Z² insbesondere einen Alkenylrest oder einen anderen einfach oder mehrfach ungesättigten Rest darstellen, sind folgende Verfahren (Schemata XIV bis XVII) besonders bevorzugt.

Ein erstes bevorzugtes Verfahren, zur Synthese der Verbindungen **1** und **2** bei denen R¹A¹Z¹A¹Z¹ insbesondere einen oder mehrere Alkenylreste oder einen anderen einfach oder mehrfach ungesättigten Rest darstellen, geht wiederum von 5-Halo-chroman-6-olen **7** aus (Schema XIV).

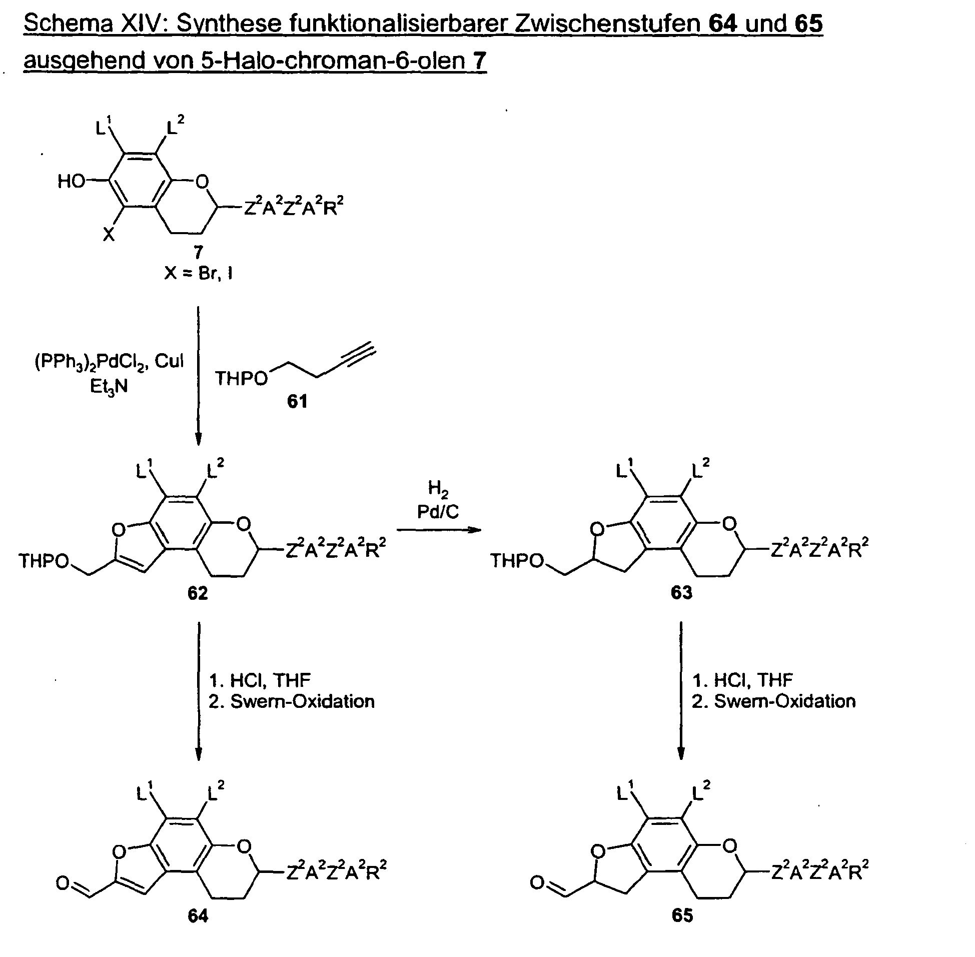

Führt man die Sequenz *Sonogashira*-Kupplung und Ringschluss mit THPgeschütztem Homopropargylakohol **61** [N. G. Kundu, M. Pal, J. S. Mahanty, M. De, J. Chem. Soc. Perkin Trans 1 1997, 19, 2815-2820] durch, so erhält man die Verbindungen **62.** Ausgehend hiervon kann nach Spaltung des THP-Ethers und Oxidation des resultierenden primären Alkohols [L. Capuano, S. Drescher, V. Hammerer, M. Hanisch, Chem. Ber. 1988, 121, 2259-2262] die funktionalisierbare Zwischenstufe **64** erhalten werden. Deren Funktionalisierung zu den Verbindungen **1** kann dann z.B. durch *Wittig*-Olefinierung (vgl. Schema XV) erfolgen. Eine funktionalisierbare Zwischenstufe **65** die auf diese Weise (*Wittig-*Olefinierung etc.) zu den Verbindungen **2** führt, wird nach Hydrierung von **62** zu **63,** anschließender THP-Spaltung und Oxidation erhalten.

Sollen Verbindungen vom Typ **1** synthetisiert werden, in denen R¹Z¹A¹Z¹A¹ *und* R²A²Z²A²Z² ungesättigte Reste, Brücken oder Ringsysteme enthalten, so können entsprechend substituierte 5-Halo-chroman-6-ole **70** eingesetzt werden. Letztere sind über das folgende Verfahren (vgl. Schema XV), mit den Chroman-2-carbaldehyden **69** als zentrale, funktionalisierbare Zwischenstufen zugänglich. Ausgehend von Salicylaldehyden **66** (Y = H, Br, vgl. Schemata V, VII und IX) erfolgt der Aufbau des Chromens **68** mit der Boronsäure **67** [R. A. Batey, A. N. Avinash, A. J. Lough, J. Am. Chem. Soc. 1999, 121, 450-451]. Nach Hydrierung und Oxidation wird die Zwischenstufe **69** erhalten. Hiervon ausgehend kann dann zunächst die Seitenkette R²A²Z²A²Z² aufgebaut werden; die nachfolgende Funktionalisierung zu den 5-Halo-chroman-6-olen **70,** die dann entsprechend Schema XIV zu den Verbindungen **1** umgesetzt werden können wurde schon beschrieben (vgl. Schemata V, VII und IX).

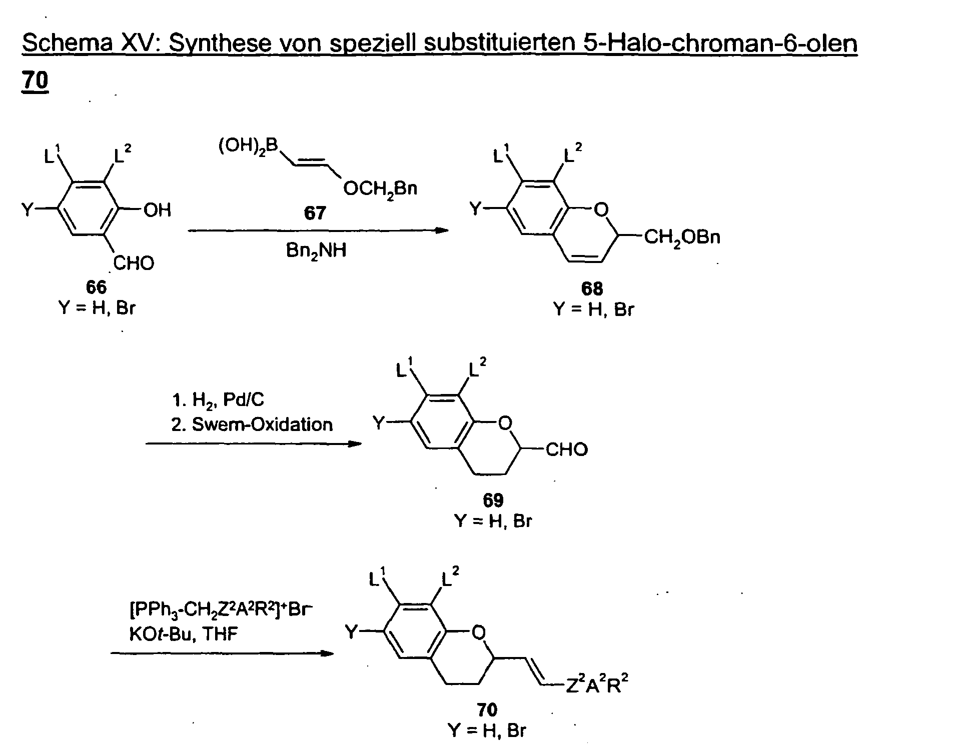

Diese Substanzen sind auch geeignet um Verbindungen **2** zu synthetisieren, in denen R¹Z¹A¹Z¹A *und* R²A²Z²A²Z² ungesättigte Reste, Brücken oder Ringsysteme enthalten. Dazu wird, in Anlehnung an die Literaturvorschrift [J. C. González-Gómez, L. Santana, E. Uriarte, Tetrahedron 2005, 61, 4805-4810 und K. J. Hodgetts, Tetrahedron 2005, 61, 6860-6870], folgendes Verfahren benötigt (vgl. Schema XVI). Schema XVI: Verfahren zur Synthese der Verbindungen **2**

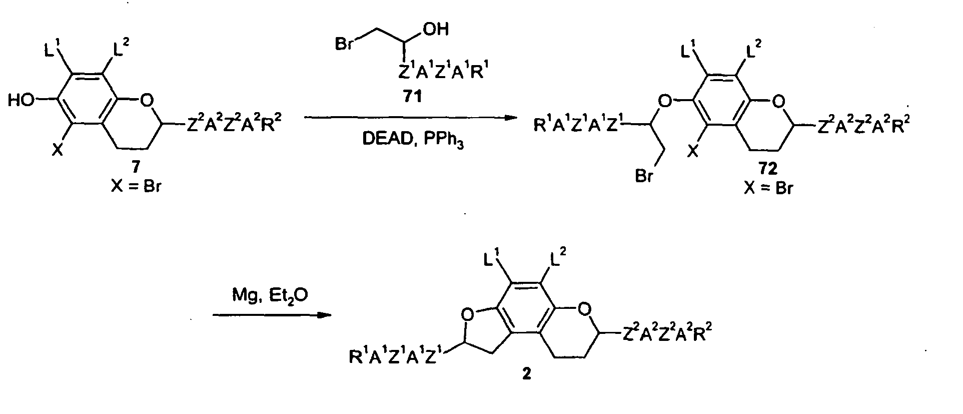

Veretherung von **7** (X = Br ist besonders bevorzugt) mit den Bromethanolderivaten **71** liefert die Verbindungen **72** (X = Br). Das aus den Verbindungen **72** generierte *Grignard*-Reagenz cyclisiert unter den Reaktionsbedingungen spontan unter Bildung eines 5-gliedrigen Heterocyclus.

Sollen Verbindungen **1** und **2** synthetisiert werden, in denen R²A²Z²A²Z² ungesättigte Reste, Brücken oder Ringsysteme enthält, so sind Verfahren, die funktionalisierbare Zwischenstufen wie **74** und **75** verwenden, bevorzugt.

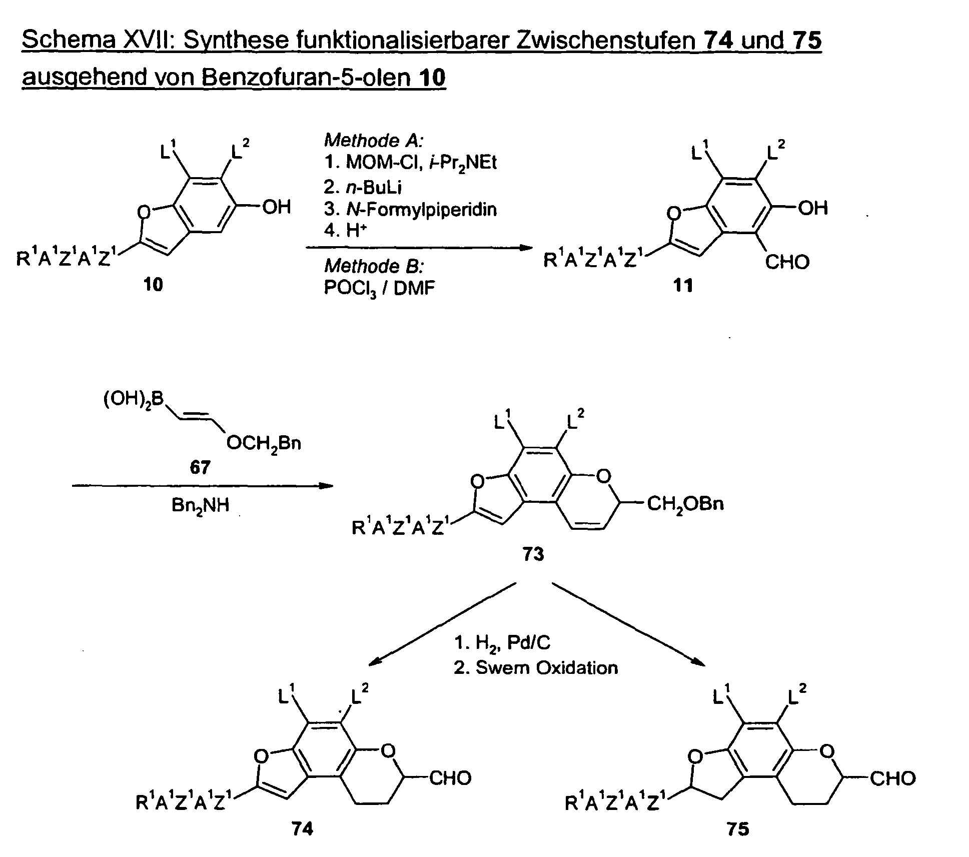

Ausgehend von den bereits zuvor beschriebenen Salicylaldehydderivaten **11** erfolgt der Aufbau des Pyrananteils mit der Boronsäure **67** [R. A. Batey, A. N. Avinash, A. J. Lough, J. Am. Chem. Soc. 1999, 121, 450-45] (vgl. Schema XV). Unter milden Bedingungen (1 atm H₂, Raumtemperatur) wird die Chromen-CC-Doppelbindung hydriert, und der Benzylether wird zum entsprechenden Alkohol gespalten. Erst bei höherem Wasserstoffdruck und erhöhter Temperatur erfolgt auch die Hydrierung der 2,3-Doppelbindung. Die gebildeten Alkohole werden zu den entsprechenden Aldehyden oxidiert, und die Verbindungen **74** und **75** können dann, wie beispielsweise in Schema XV dargestellt, zu den Verbindungen **1** und **2** funktionalisiert werden.

Im Folgenden werden Beispiele für Strukturen bevorzugter Verbindungen der Formel I geordnet nach Unterformel gegeben, worin
- R¹¹ und R²²: die jeweilige unter Formel I für R¹ bzw. R² gegebene Bedeutung haben
- p,: und im Fall, dass p mehrfach auftritt diese unabhängig voneinander, 0, 1, 2, 3 oder 4, bevorzugt 0, 1 oder 2 und bevorzugt 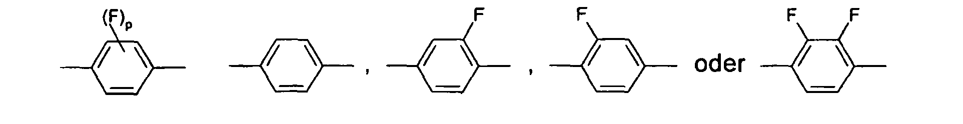
bedeuten.

Bevorzugte Verbindungen der Formeln IA-1 bzw. IB-1 sind:

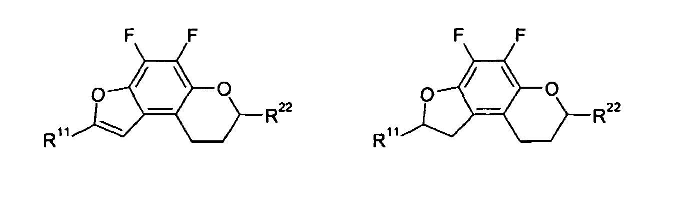

Bevorzugte Verbindungen der Formeln IA-2 bzw. IB-2 sind:

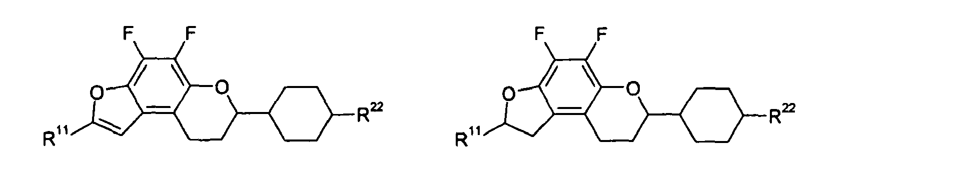

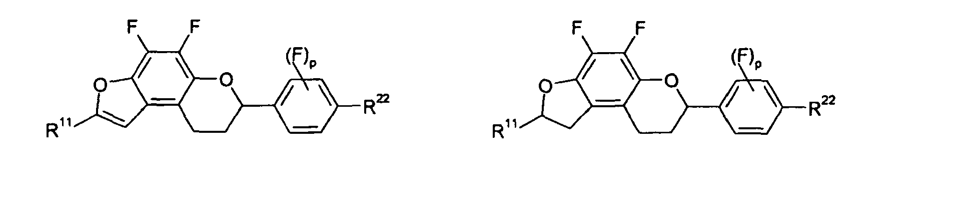

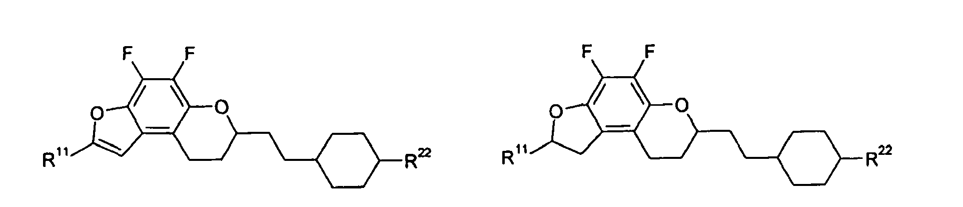

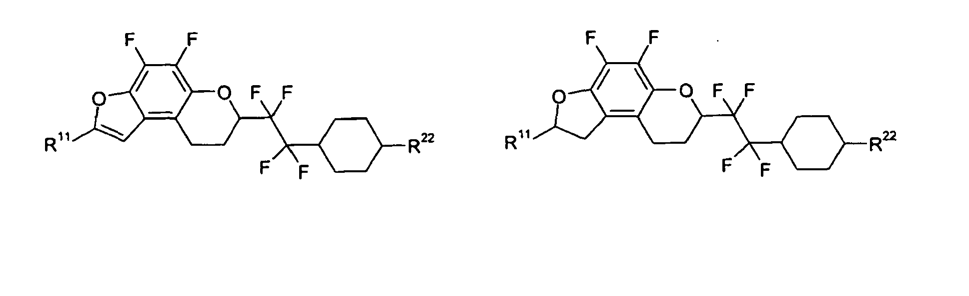

Besonders bevorzugt unter den Verbindungen IA-2 und IB-2 sind solche mit einem Cyclohexylring.

Bevorzugte Verbindungen der Formeln IA-3 bzw. IB-3 sind:

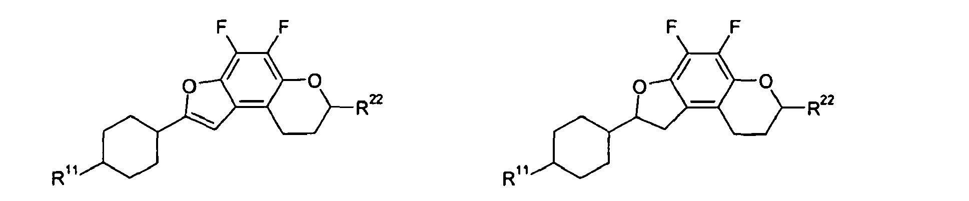

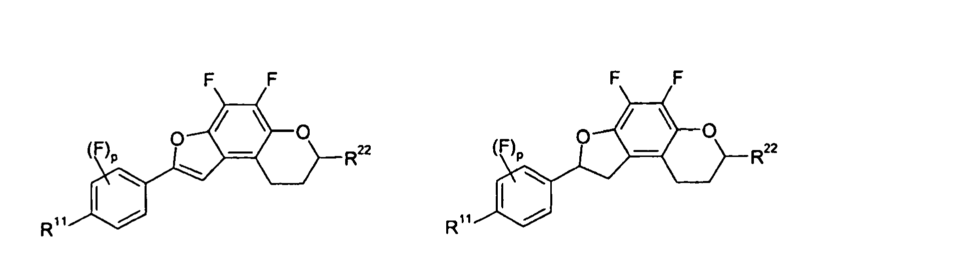

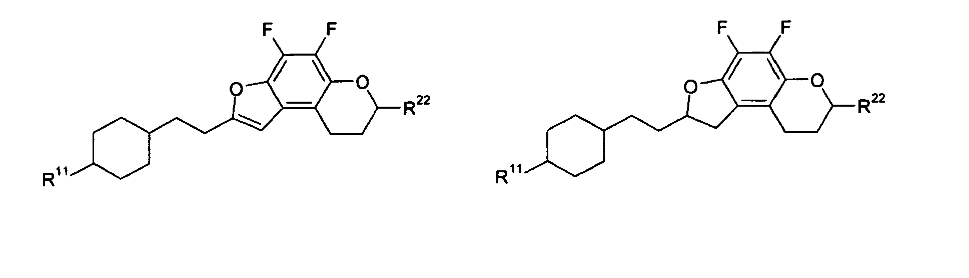

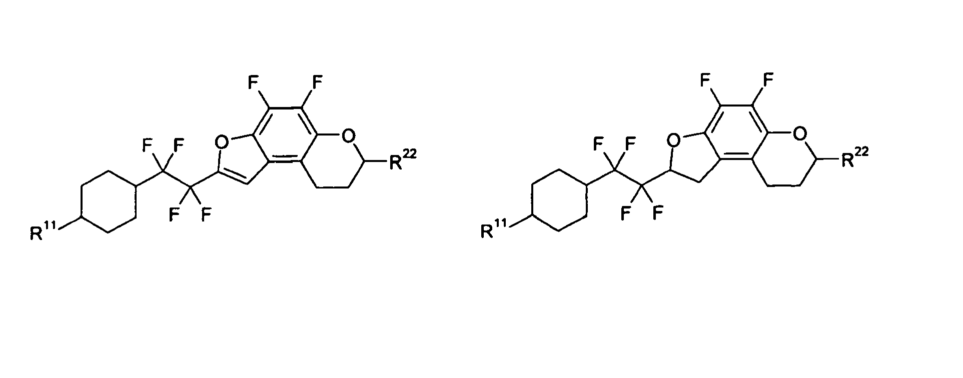

Unter den Verbindungen der Formeln IA-3 sind Verbindungen mit einem unsubstituierten oder substituierten1,4-Phenylen-Ring bevorzugt.

Besonders bevorzugt unter den Verbindungen IB-3 sind solche mit einem Cyclohexylring.

Bevorzugte Verbindungen der Formeln IA-4 bzw. IB-4 sind:

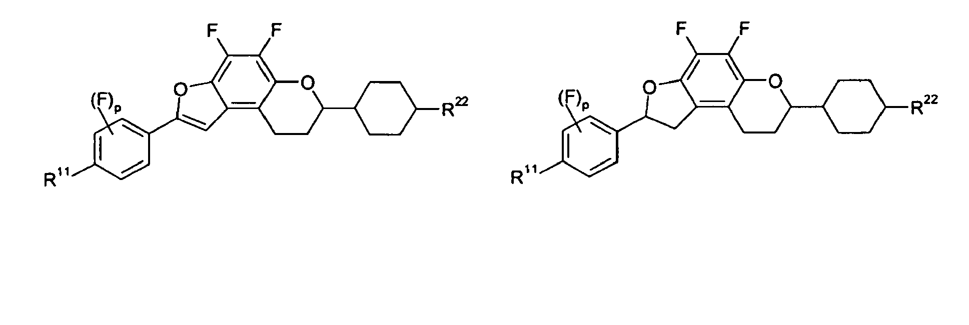

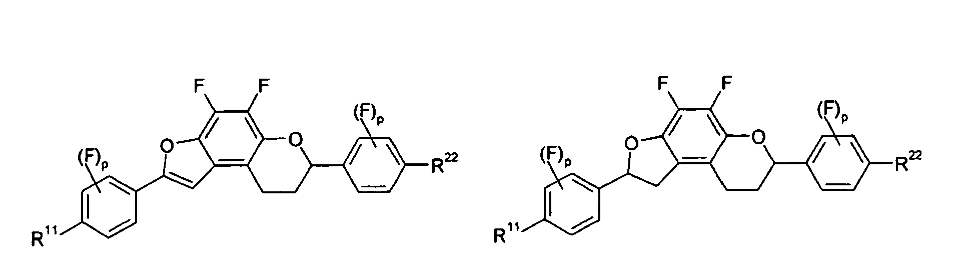

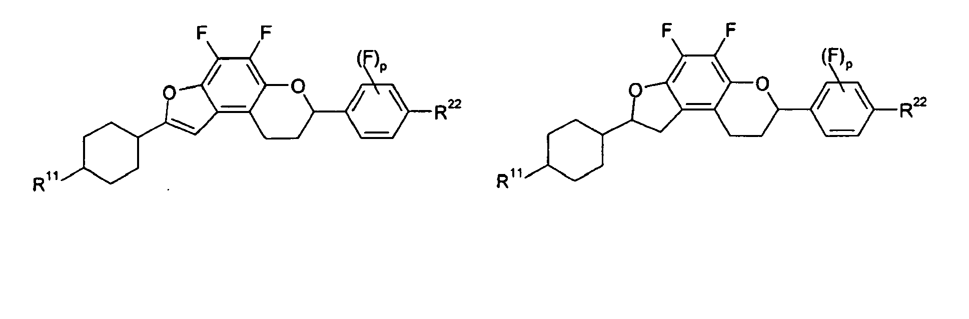

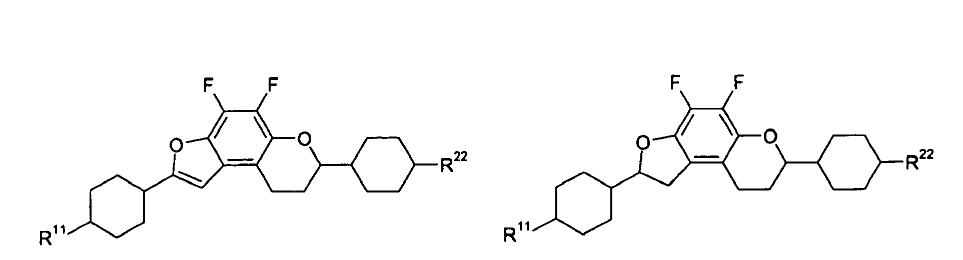

Unter den Verbindungen der Formel IA-4 sind Verbindungen der Formeln besonders bevorzugt, in denen das Grundgerüst linksseitig mit einem substituierten oder unsubstituierten 1,4-Phenylenring substituiert ist und / oder das Grundgerüst rechtsseitig mit einem Cyclohexylrest verknüpft ist.

Unter den Verbindungen der Formel IB-4 sind Verbindungen der Formeln bevorzugt, in denen das Grundgerüst linksseitig mit einem Cyclohexylrest substituiert ist und / oder der Grundkörper rechtsseitig mit einem Cyclohexylrest verknüpft ist.

Bevorzugte Verbindungen der Formeln IA-5 bzw. IB-5 sind:

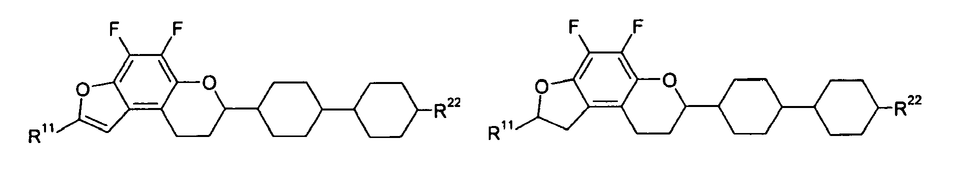

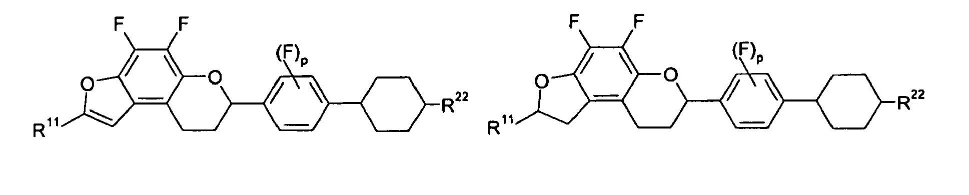

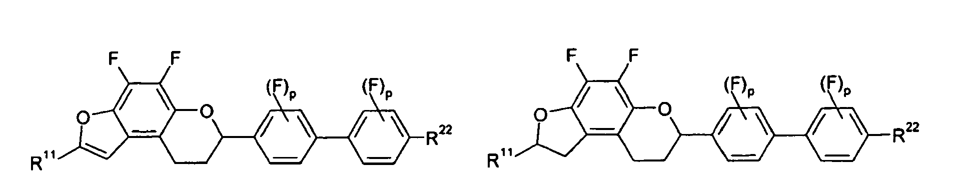

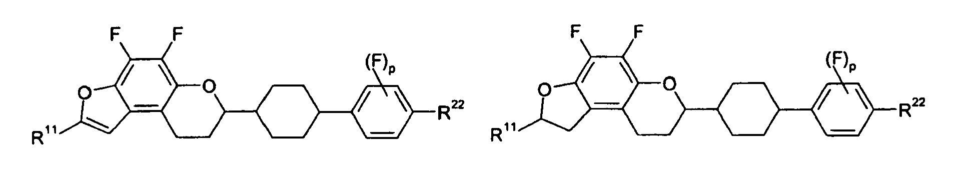

Bevorzugt unter den Verbindungen IA-5 und IB-5 sind solche mit einem Cyclohexylring. Besonders bevorzugt sind dabei Verbindungen bei denen ein Cyclohexylring direkt mit dem Grundgerüst verknüpft ist. Ganz besonders bevorzugt sind Verbindungen mit zwei Cyclohexylringen.

Bevorzugte Verbindungen der Formeln IA-6 bzw. IB-6 sind:

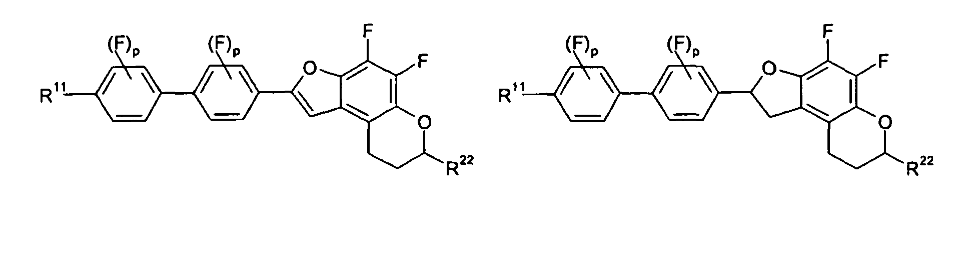

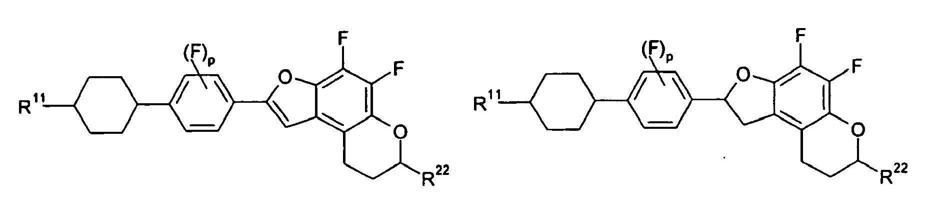

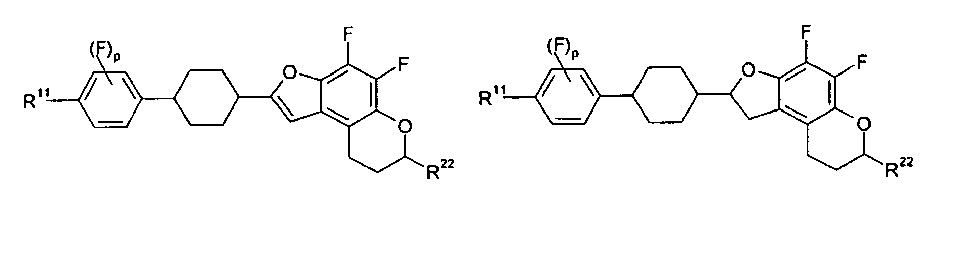

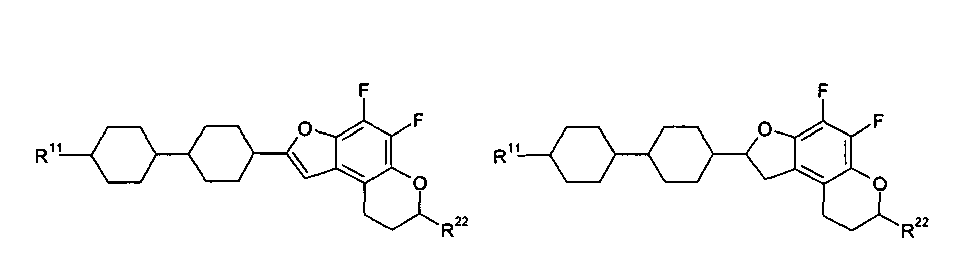

Bevorzugt unter den Verbindungen IA-6 solche mit einem einem unsubstituierten oder substituierten 1,4-Phenylen-Ring. Besonders bevorzugt sind dabei Verbindungen bei denen ein unsubstituierter oder substituierter 1,4-Phenylen-Ring direkt mit dem Grundgerüst verknüpft ist. Bevorzugt unter den Verbindungen IB-6 sind solche mit einem Cyclohexylring. Besonders bevorzugt sind dabei Verbindungen bei denen ein Cyclohexylring direkt mit dem Grundgerüst verknüpft ist.

Unter den Verbindungen der Formel IA-7 sind Verbindungen der Formeln bevorzugt, in denen das Grundgerüst linksseitig mit einem Arylrest substituiert ist und / oder das Grudgerüst rechtsseitig mit einem Cyclohexylrest verknüpft ist.

Unter den Verbindungen der Formel IB-7 sind Verbindungen der Formeln bevorzugt, in denen das Grundgerüst linksseitig mit einem Cyclohexylrest substituiert ist und / oder das Grundgerüst rechtsseitig mit einem Cyclohexylrest verknüpft ist.

Unter den Verbindungen der Formel IA-8 sind Verbindungen der Formeln bevorzugt, in denen das Grundgerüst linksseitig mit einem Arylrest substituiert ist und / oder das Grundgerüst rechtsseitig mit einem Cyclohexylrest verknüpft ist.

Unter den Verbindungen der Formel IB-8 sind Verbindungen der Formeln bevorzugt, in denen das Grundgerüst linksseitig mit einem Cyclohexylrest substituiert ist und / oder das Grundgerüst rechtsseitig mit einem Cyclohexylrest verknüpft ist.

Unter den Verbindungen der Formel IA-9 sind Verbindungen der Formeln bevorzugt, in denen das Grundgerüst linksseitig mit einem Arylrest substituiert ist und / oder das Grundgerüst rechtsseitig mit einem Cyclohexylrest verknüpft ist.

Unter den Verbindungen der Formel IB-9 sind Verbindungen der Formeln bevorzugt, in denen das Grundgerüst linksseitig mit einem Cyclohexylrest substituiert ist und / oder das Grundgerüst rechtsseitig mit einem Cyclohexylrest verknüpft ist.

Bevorzugt unter den Verbindungen IA-10 und IB-10 sind solche mit einem oder mehreren Cyclohexylringen. Besonders bevorzugt sind dabei Verbindungen bei denen der Cyclohexylring direkt mit dem Grundgerüst verknüpft ist.

Bevorzugt unter den Verbindungen IA-11 solche mit einem unsubstituierten oder substituierten1,4-Phenylen-Ring. Besonders bevorzugt sind dabei Verbindungen bei denen der unsubstituierte oder substituierte1,4-Phenylen-Ring direkt mit dem Grundgerüst verknüpft ist.

Bevorzugt unter den Verbindungen IB-11 solche mit einem oder mehreren Cyclohexylringen. Besonders bevorzugt sind dabei Verbindungen bei denen der Cyclohexylring direkt mit dem Grundgerüst verknüpft ist.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Gegenstand der vorliegenden Erfindung sind auch Flüssigkristallmedien, die eine oder mehrere Verbindung(en) der Formel I enthalten.

In einer bevorzugten Ausführungsform enthalten die Flüssigkristallmedien gemäß der vorliegenden Erfindung
a) eine oder mehrere dielektrisch negative Verbindung(en) der Formel 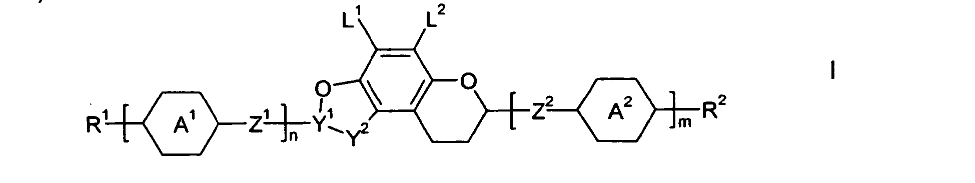 worin die Parameter die oben unter Formel I gegebene Bedeutung haben
b) eine oder mehrere dielektrisch negative Verbindung(en) der Formel II 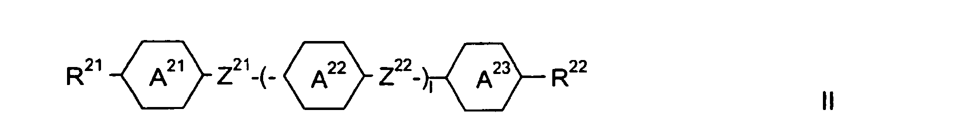 worin
   - R²¹ und R²²: jeweils unabhängig voneinander die oben bei Formel I für R¹ gegebene Bedeutung haben,
   - Z²¹ und Z²²: jeweils unabhängig voneinander die oben bei Formel I für Z¹ gegebene Bedeutung haben,
   mindestens einer der vorhandenen Ringe 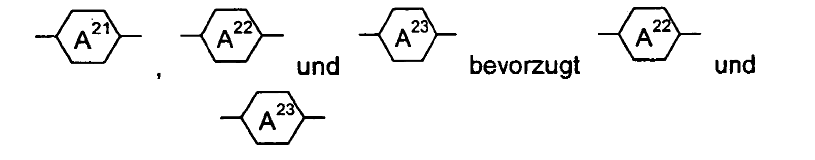 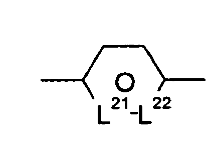 und die anderen, jeweils unabhängig voneinander, 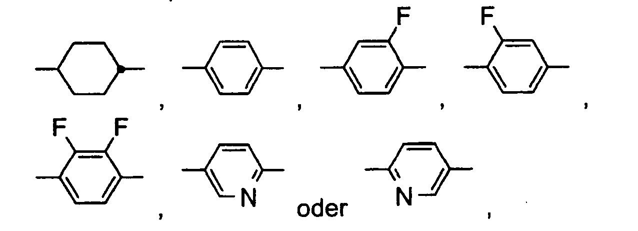 bevorzugt 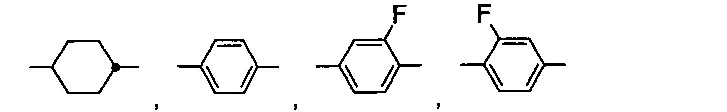 oder 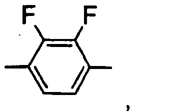 besonders bevorzugt 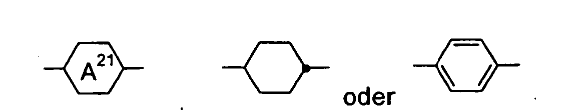 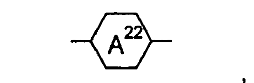 wenn vorhanden, 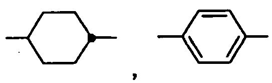 oder 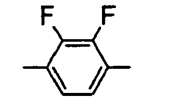
   - L²¹ und L²²: beide C-F oder einer von beiden N und der andere C-F, bevorzugt beide C-F und
   - I: 0, 1 oder 2, bevorzugt 0 oder 1
   bedeuten;
   und optional
c) eine oder mehrere dielektrisch neutrale Verbindung der Formel III 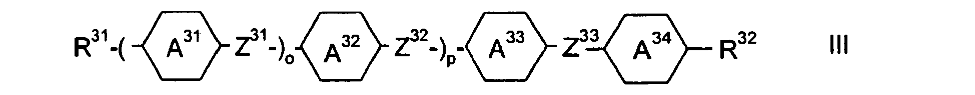 worin
   - R³¹ und R³²: jeweils unabhängig voneinander die oben bei Formel I für R¹ gegebene Bedeutung besitzen und
   - Z³¹, Z³² und Z³³: jeweils unabhängig voneinander -CH₂CH₂-, -CH=CH-, -COO- oder eine Einfachbindung 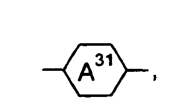 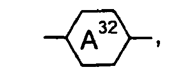 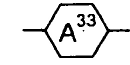 und 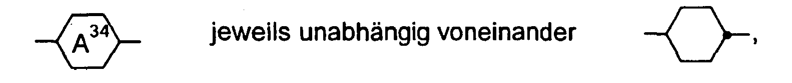 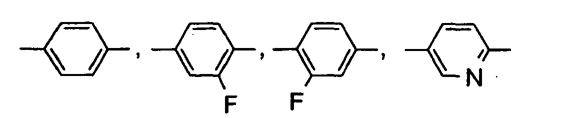 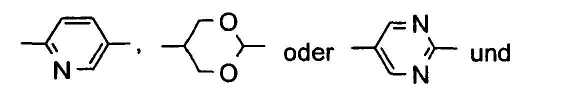
   o und p unabhängig voneinander 0 oder 1 bevorzugt jedoch
   R³¹ und R³² jeweils unabhängig voneinander Alkyl oder Alkoxy mit 1-5 C-Atomen oder Alkenyl mit 2-5 C-Atomen, 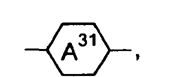 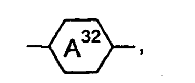 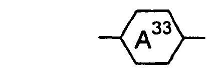 und 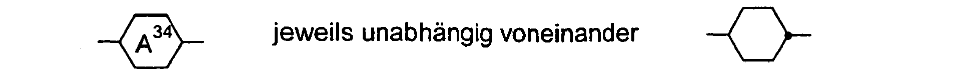 oder 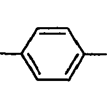 und ganz besonders bevorzugt mindestens zwei dieser Ringe 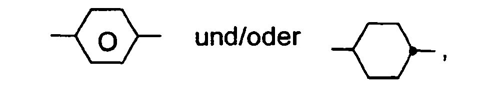 wobei ganz besonders bevorzugt zwei benachbarte Ringe direkt verknüpft sind und zwar bevorzugt 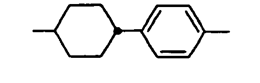 oder 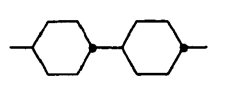 bedeuten, wobei bei dem Phenylenring ein oder mehrere H-Atome, unabhängig voneinander durch F oder CN, bevorzugt durch F und eine oder zwei nicht benacbarte CH₂-Gruppen des Cyclohexylenrings bzw. eines der Cyclohexylenringe durch O-Atome ersetzt sein können.

Bevorzugt enthalten die Flüssigkristallmedien eine oder mehrere Verbindungen der Formel I die keine Biphenyleinheit enthalten.

Besonders bevorzugt enthalten die Flüssigkristallmedien eine oder mehrere Verbindungen der Formel I wobei zwei benachbarte Ringe direkt verknüpft sind und bevorzugt 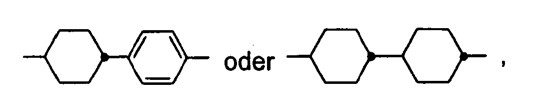 bedeuten,
wobei bei dem Phenylenring ein oder mehrere H-Atome, unabhängig voneinander durch F oder CN, bevorzugt durch F, und eine oder zwei nicht benacbarte CH₂-Gruppen des Cyclohexylenrings bzw. eines der Cyclohexylenringe durch O-Atome ersetzt sein können.

In einer bevorzugten Ausführungsform, die mit den gerade beschriebenen Ausführungsformen identisch sein kann, enthalten die Flüssigkristallmedien eine oder mehrere Verbindungen ausgewählt aus der Gruppe der Verbindungen ausgewählt aus der Gruppe der Verbindungen der Formel I-3.

Bevorzugt enthält das Flüssigkristallmedium eine oder mehrere Verbindungen ausgewählt aus der Gruppe der Verbindungen der Formeln II-1 bis II-3 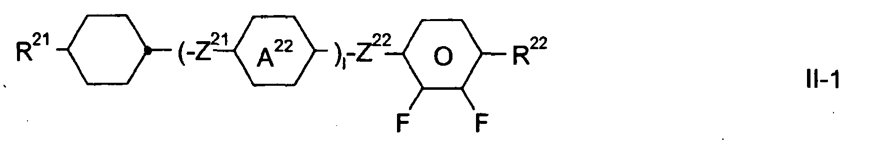 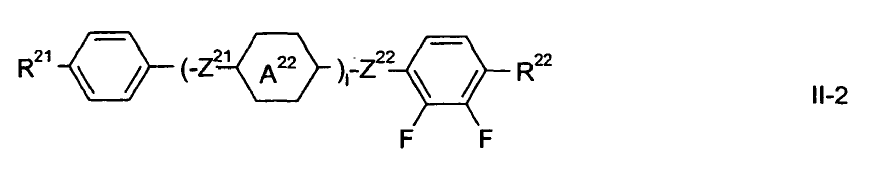 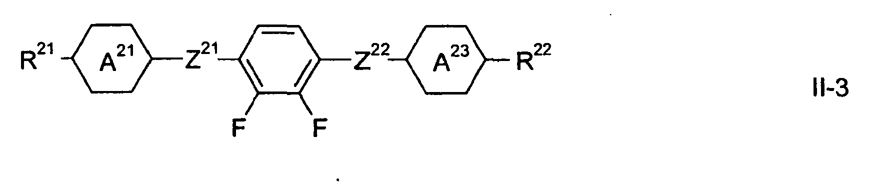 worin
R²¹, R²², Z¹², Z²², 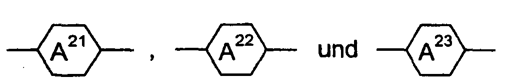 und I
jeweils die oben bei Formel II gegebene Bedeutung besitzen. Bevorzugt ist R²¹ Alkyl, bevorzugt mit 1-5 C-Atomen, R²¹ Akyl oder Alkoxy, bevorzugt jeweils mit 1 bis 5 C-Atomen, und Z²² sowie Z²¹, wenn vorhanden, eine Einfachbindung.

Besonders bevorzugt enthält das Flüssigkristallmedium eine oder mehrere Verbindungen ausgewählt aus der Gruppe der Verbindungen der Formeln III-1 bis III-3: 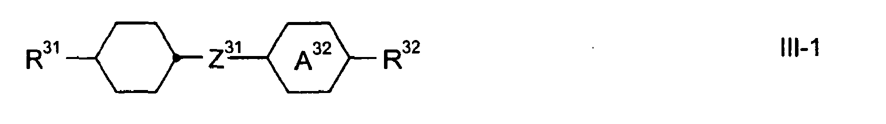 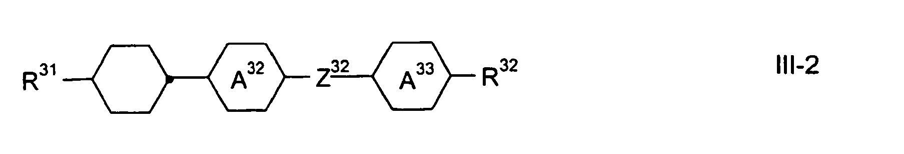 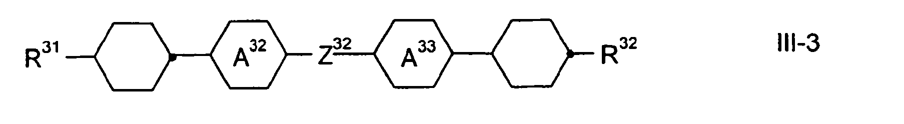 worin R³¹, R³², Z³¹, Z³², 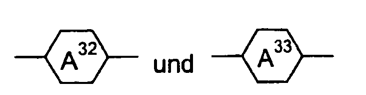 jeweils die oben
bei Formel III angegebene Bedeutung haben.

Insbesondere bevorzugt enthält das Flüssigkristallmedium eine oder mehrere Verbindungen ausgewählt aus der Gruppe der Verbindungen der Formeln III-1a bis III-1d, III-1e, III-2a bis III-2g, III-3a bis III-3d und III-4a: 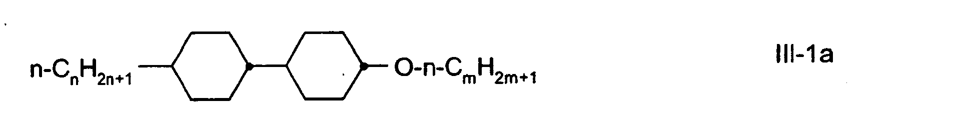 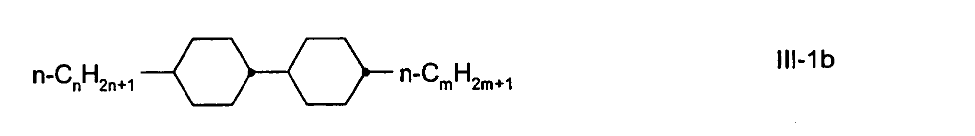 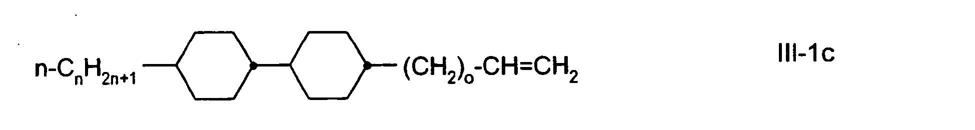 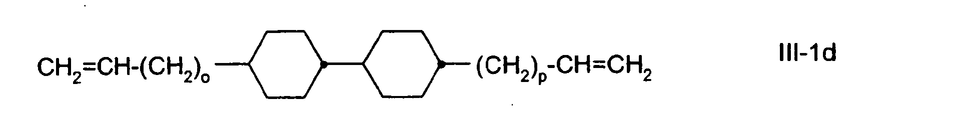 worin n und m jeweils unabhängig voneinander 1 bis 5 und o und p jeweils sowohl davon als auch voneinander unabhängig 0 bis 3 bedeuten, 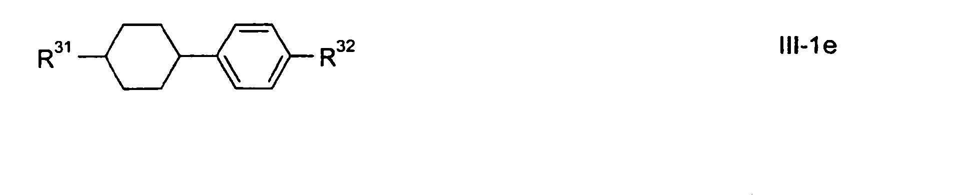 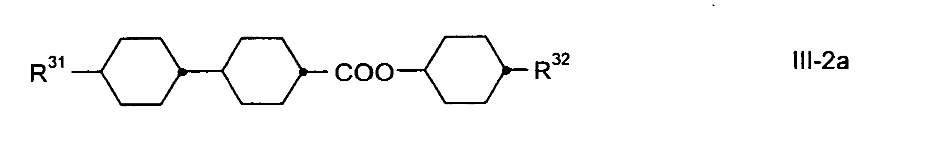 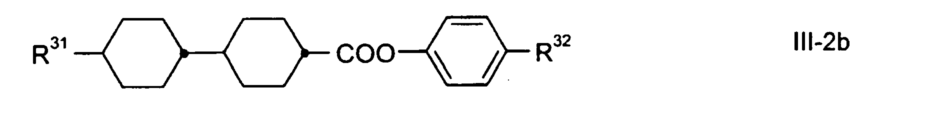 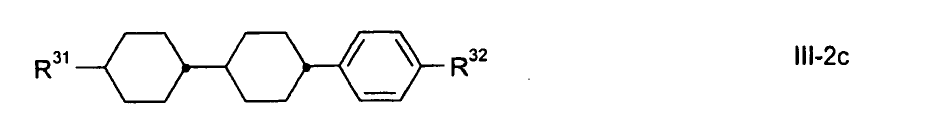 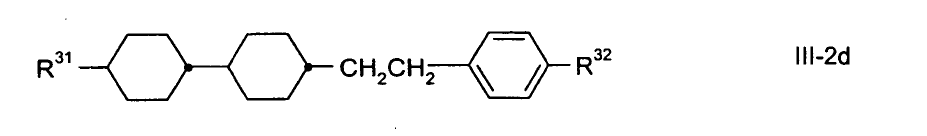 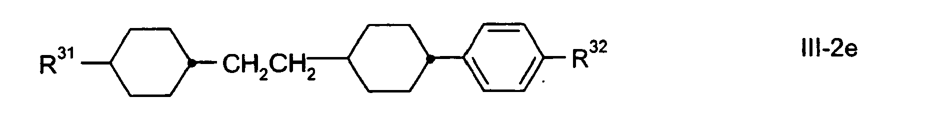 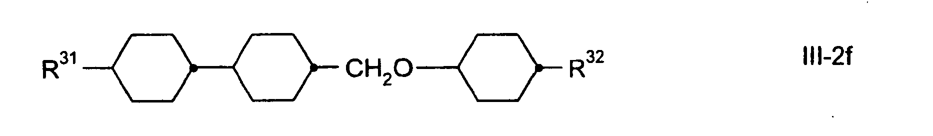 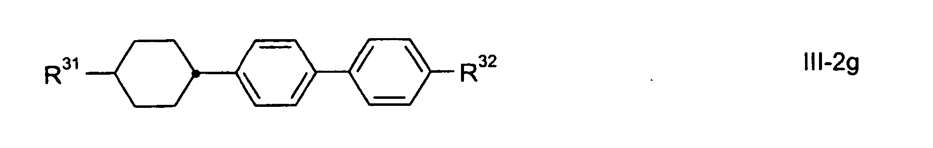 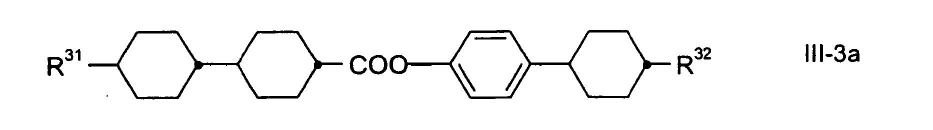 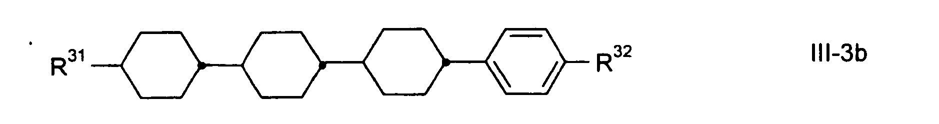 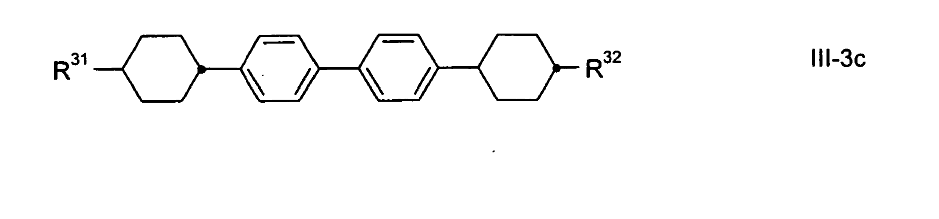 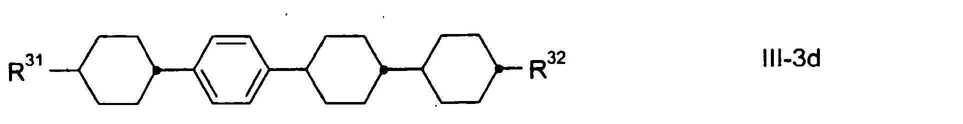 worin R³¹ und R³³ jeweils die oben unter Formel III, bevorzugt die unter Formel III-1, angegebene Bedeutung besitzen und die Phenylringe, insbesondere bei den Verbindungen III-2g und III-3c optional fluoriert sein können, jedoch nicht so, dass die Verbindungen mit denen der Formel II und ihren Unterformeln identisch sind. Bevorzugt ist R³¹ n-Alkyl mit 1 bis 5 C-Atomen, insbesondere bevorzugt mit 1 bis 3 C-Atomen und R³² n-Alkyl oder n-Alkoxy mit 1 bis 5 C-Atomen oder Alkenyl mit 2 bis 5 C-Atomen. Hiervon sind insbesondere Verbindungen der Formeln III-1a bis III-1d bevorzugt.

Bevorzugte fluorierte Verbindungen der Formeln III-2g und III-3c sind die Verbindungen der Formeln III-2g' und III-3c' 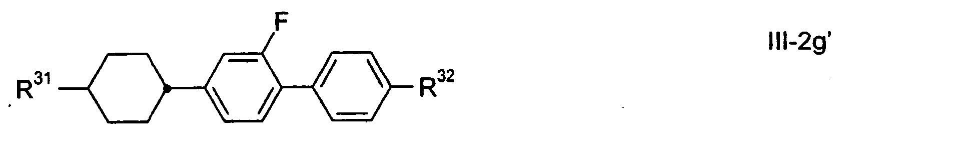 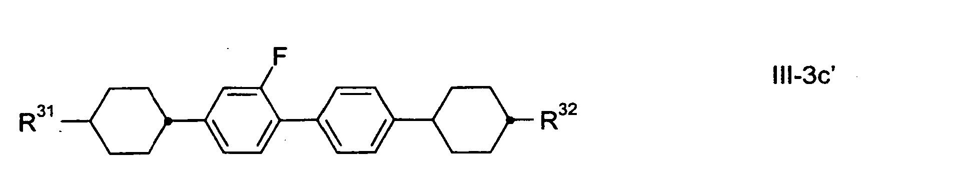 worin R³¹ und R³³ jeweils die oben unter Formel III, bevorzugt die unter Formel III-2g, bzw. III-3c angegebene Bedeutung haben.

In der vorliegenden Anmeldung bedeutet, wenn nicht ausdrücklich anders angegeben, der Begriff Verbindungen sowohl eine Verbindung, als auch mehrere Verbindungen.

Die erfindungsgemäßen Flüssigkristallmedien weisen bevorzugt nematische Phasen von jeweils mindestens von -20°C bis 80°C, bevorzugt von -30°C bis 85°C und ganz besonders bevorzugt von -40°C bis 100°C auf. Hierbei bedeutet der Begriff eine nematische Phase aufweisen einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, einer der elektrooptischen Anwendung entsprechenden Schichtdicke, für mindestens 100 Stunden überprüft. Bei hohen Temperaturen wird der Klärpunkt nach üblichen Methoden in Kapillaren gemessen.

Ferner sind die erfindungsgemäßen Flüssigkristallmedien durch niedrige optische Anisotropien gekennzeichnet.

Der Ausdruck "Alkyl" umfasst vorzugsweise geradkettige und verzweigte Alkylgruppen mit 1 bis 7 Kohlenstoffatomen, insbesondere die geradkettigen Gruppen Methyl, Ethyl, Propyl, Butyl, Pentyl, Hexyl und Heptyl. Gruppen mit 2 bis 5 Kohlenstoffatomen sind im allgemeinen bevorzugt.

Der Ausdruck "Alkenyl" umfast vorzugsweise geradkettige und verzweigte Alkenylgruppen mit 2 bis 7 Kohlenstoffatomen, insbesondere die geradkettigen Gruppen. Besonders bevorzugte Alkenylgruppen sind C₂ bis C₇-1 E-Alkenyl, C₄ bis C₇-3E-Alkenyl, C₅ bis C₇-4-Alkenyl, C₆ bis C₇-5-Alkenyl und C₇ 6-Alkenyl, insbesondere C₂ bis C₇-1 E-Alkenyl, C₄ bis C₇-3E-Alkenyl und C₅ bis C₇-4-Alkenyl. Beispiele weiterer bevorzugter Alkenylgruppen sind Vinyl, 1 E-Propenyl, 1 E-Butenyl, 1E-Pentenyl,1E-Hexenyl,1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl", bzw. Alkoxyalkyl umfasst vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n 1 und m 1 bis 6.

Verbindungen mir einer Vinyl-Endgruppe und Verbindungen mit einer Methyl-Endgruppe haben eine geringe Rotationsviskosität.

In der vorliegenden Anmeldung bedeuten die Begriffe dielektrisch positive Verbindungen solche Verbindungen mit einem Δε > 1,5, dielektrisch neutrale Verbindungen solche mit-1,5 ≤ Δε ≤ 1,5 und dielektrisch negative Verbindungen solche mit Δε < -1,5. Hierbei wird die dielektrische Anisotropie der Verbindungen bestimmt indem 10 % der Verbindungen in einem flüssigkristallinen Host gelöst werden und von dieser Mischung die Kapazität in mindestens jeweils einer Testzelle mit ca. 20 µm Schichtdicke mit homeotroper und mit homogener Oberflächenorientierung bei 1 kHz bestimmt wird. Die Meßspannung beträgt typischerweise 0,5 V bis 1,0 V, jedoch stets weniger als die kapazitive Schwelle der jeweiligen Flüssigkristallmischung.

Als Wirtsmischung für die Bestimmung der anwendungsrelevanten physikalischen Parameter wird ZLI-4792, von von Merck KGaA, Deutschland, verwendet. Als Ausnahme wird bei der Bestimmung der dielektrischen Anisotropie von dielektrisch negativen Verbindungen ZLI-2857, ebenfalls von Merck KGaA, Deutschland, verwendet. Aus der Änderung der Eigenschaften, z.B. der Dielektrizitätskonstanten, der Wirtsmischung nach Zugabe der zu untersuchenden Verbindung und Extrapolation auf 100 % der eingesetzten Verbindung werden die Werte für die jeweilige zu untersuchende Verbindung erhalten.

Die eingesetzte Konzentration der zu untersuchenden Verbindung beträgt 10 %. Ist die Löslichkeit der zu untersuchenden Verbindung hierzu nicht ausreichend wird ausnahmsweise die eingesetzte Konzentration solange halbiert, also auf 5 %, 2,5% usw. verringert, bis die Löslichkeitsgrenze unterschritten ist.

Der Begriff Schwellenspannung bezieht sich üblicherweise auf die optische Schwelle für 10 % relativen Kontrast (V₁₀). In Bezug auf die Flüssigkristallmischungen mit negativer dielektrischer Anisotropie, wird der Begriff Schwellenspannung in der vorliegenden Anmeldung jedoch für die kapazitive Schwellenspannung (V₀), auch Freedericksz-Schwelle genannt, verwendet, sofern nicht explizit anders angegeben.

Alle Konzentrationen in dieser Anmeldung sind, soweit nicht explizit anders vermerkt, in Massenprozent angegeben und beziehen sich auf die entsprechende Gesamtmischung. Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20 °C, sofern nicht explizit anders angegeben. Δn wird bei 589 nm und Δε bei 1 kHz bestimmt.

Bei den Flüssigkristallmedien mit negativer dielektrischer Anisotropie wurde die Schwellenspannung als kapazitive Schwellung V₀ in Zellen mit durch Lecithin homeotrop orientierter Flüssigkristallschicht bestimmt.

Die erfindungsgemäßen Flüssigkristallmedien können bei Bedarf auch weitere Zusatzstoffe und gegebenenfalls auch chirale Dotierstoffe in den üblichen Mengen enthalten. Die eingesetzte Menge dieser Zusatzstoffe beträgt insgesamt 0 % bis 10 % bezogen auf die Menge der gesamten Mischung bevorzugt 0,1 % bis 6 %. Die Konzentrationen der einzelnen eingesetzten Verbindungen betragen jeweils bevorzugt 0,1 bis 3 %. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 bis 30, besonders bevorzugt aus 6 bis 20 und ganz besonders bevorzugt aus 10 bis 16 Verbindungen, die auf herkömmliche Weise gemischt werden. In der Regel wird die gewünschte Menge der in geringerer Menge verwendeten Komponenten in den Komponenten gelöst, die den Hauptbestandteil ausmachen, zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z.B. unter Verwendung von Vormischungen oder aus einem sogenannten "multi bottle" Systemen herzustellen.

Mittels geeigneter Zusatzstoffe können die erfindungsgemäßen Flüssigkristallphasen derart modifiziert werden, dass sie in jeder bisher bekannt gewordenen Art von Anzeige und insbesondere von ECB-Anzeigen, sowie IPS-Anzeigen einsetzbar sind.

Die nachstehenden Beispiele dienen zur Veranschaulichung der Erfindung, ohne sie zu beschränken. In den Beispielen sind der Schmelzpunkt T(C,N), der Übergang von der smektischen (S) zur nematischen (N) Phase T(S,N) und Klärpunkt T(N,I) einer Flüssigkristallsubstanz in Grad Celsius angegeben. Die verschiedenen smektischen Phasen werden durch entsprechende Suffixe gekennzeichnet.

Die Prozentangaben sind, soweit nicht explizit anders gekennzeichnet, vor-und nachstehend Massenprozente und die physikalischen Eigenschaften sind die Werte bei 20 °C, sofern nicht explizit anders angegeben.

Alle angegebenen Werte für Temperaturen in dieser Anmeldung sind °C und alle Temperaturdifferenzen entsprechend Differenzgrad, sofern nicht explizit anders angegeben.

In der vorliegenden Anmeldung und insbesondere bei den Synthesebeispielen und -schemata bedeuten:
- Bn: Benzyl,
- Clp: Klärpunkt,
- DEAD: Diethylazodicarboxylat,
- DIAD: Diisopropylazodicarboxylat,
- DMF: Dimethylformamid,
- ges.: gesättigt,
- Lsg.: Lösung,
- MEM: 2-Methoxy-ethoxy-methyl,
- MOM: Methoxy-methyl,
- MTBE: Methyl-*tert*-butylether,
- Ph: Phenyl,
- Schmp.: Schmelzpunkt,
- SiO₂: Kieselgel,
- TBS: Dimethyl-*tert*-butylsilyl,
- THF: Tetrahydrofuran und
- TMEDA: Tetramethylethylendiamin.

In der vorliegenden Anmeldung und in den folgenden Beispielen sind die Strukturen der Flüssigkristallverbindungen durch Abkürzungen, auch Akronyme genannt, angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Akronym für den Grundkörper angegeben. Im Einzelfall folgt getrennt vom Akronym für den Grundkörper mit einem Strich ein Code für die Substituenten R¹, R², L¹, L² und L³:

| Code für R¹, | R¹ | R² | L¹ | L² | L³ |
|---|---|---|---|---|---|
| R², L¹, L², L³ | | | | | |
| nm | CₙH₂ₙ₊₁ | CₘH₂ₘ₊₁ | H | H | H |
| nOm | CₙH₂ₙ₊₁ | OCₘH₂ₘ₊₁ | H | H | H |
| nO.m | OCₙH₂ₙ₊₁ | CₘH₂ₘ₊₁ | H | H | H |
| nmFF | CₙH₂ₙ₊₁ | CₘH₂ₘ₊₁ | F | H | F |
| nOmFF | CₙH₂ₙ₊₁ | CₘH₂ₘ₊₁ | F | H | F |
| nO.mFF | OCₙH₂ₙ₊₁ | CₘH₂ₘ₊₁ | F | H | F |
| nO.OmFF | OCₙH₂ₙ₊₁ | OCₘH₂ₘ₊₁ | F | H | F |
| n | CₙH₂ₙ₊₁ | CN | H | H | H |
| nN.F | CₙH₂ₙ₊₁ | CN | F | H | H |
| nN.F.F | CₙH₂ₙ₊₁ | CN | F | F | H |
| nF | CₙH₂ₙ₊₁ | F | H | H | H |
| nF.F | CₙH₂ₙ₊₁ | F | F | H | H |
| nF.F.F | CₙH₂ₙ₊₁ | F | F | F | H |
| nCl | CₙH₂ₙ₊₁ | Cl | H | H | H |
| nCl.F | CₙH₂ₙ₊₁ | Cl | F | H | H |
| nCl.F.F | CₙH₂ₙ₊₁ | Cl | F | F | H |
| nmF | CₙH₂ₙ₊₁ | CₘH₂ₘ₊₁ | F | H | H |
| nCF₃ | CₙH₂ₙ₊₁ | CF₃ | H | H | H |
| nOCF₃ | CₙH₂ₙ₊₁ | OCF₃ | H | H | H |
| nOCF₃.F | CₙH₂ₙ₊₁ | OCF₃ | F | H | H |
| nOCF₃.F.F | CₙH₂ₙ₊₁ | OCF₃ | F | F | H |
| nOCF₂ | CₙH₂ₙ₊₁ | OCHF₂ | H | H | H |
| nOCF₂.F.F | CₙH₂ₙ₊₁ | OCHF₂ | F | F | H |
| nS | CₙH₂ₙ₊₁ | NCS | H | H | H |
| rVsN | CᵣH₂ᵣ₊₁-CH=CH-C₅H₂ₛ- | CN | H | H | H |
| nEsN | CᵣH₂ᵣ₊₁-O-CₛH₂ₛ- | CN | H | H | H |
| nAm | CₙH₂ₙ₊₁ | COOCₘH₂ₘ₊₁ | H | H | H |
| nF.Cl | CₙH₂ₙ₊₁ | F | Cl H | | H |

### Tabelle A:

| | |
|---|---|
| 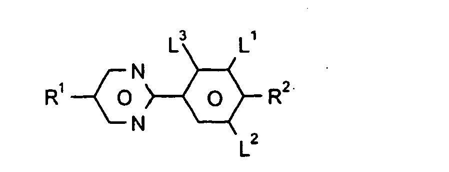 | 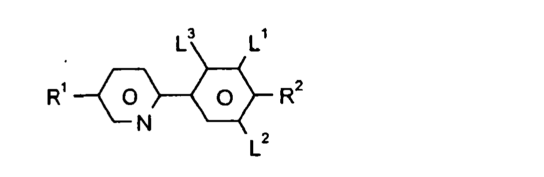 |
| **PYP** | **PYRP** |
| 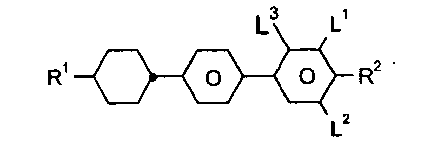 | 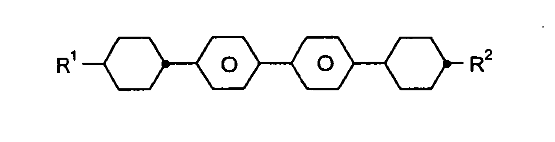 |
| **BCH** | **CBC** |
| 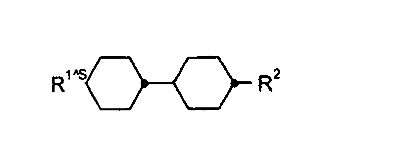 | 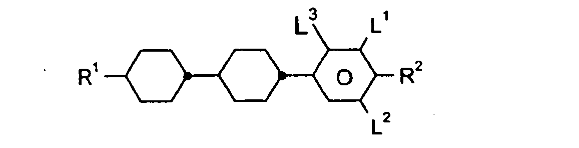 |
| **CCH** | **CCP** |
| 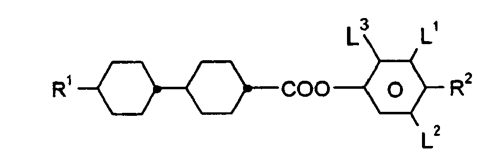 | 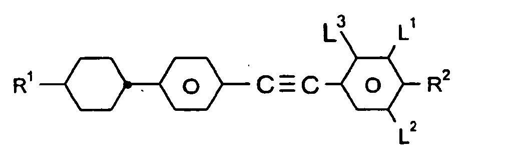 |
| **CP** | **CPTP** |
| 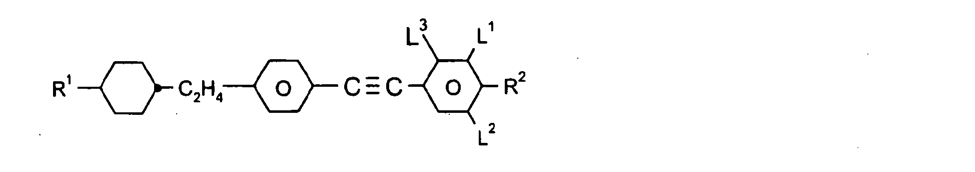 | |
| **CEPTP** | |
| 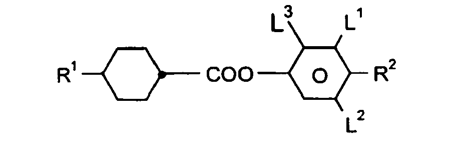 | 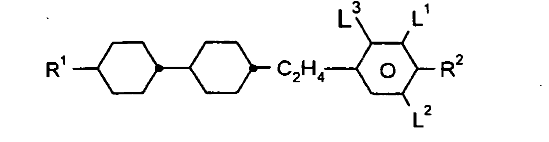 |
| D | **ECCP** |
| 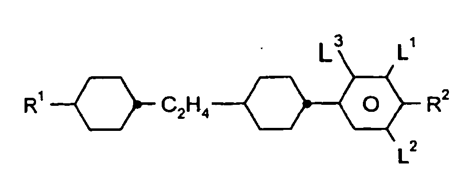 | 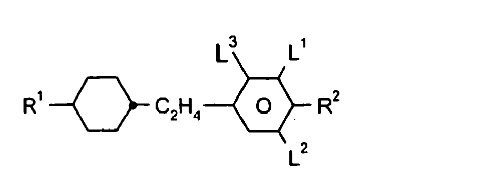 |
| **CECP** | **EPCH** |
| 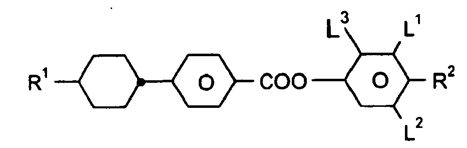 | 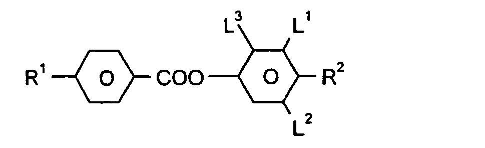 |
| **HP** | **ME** |
| 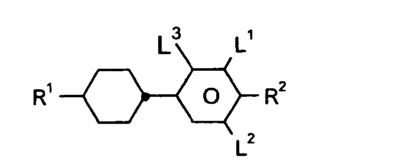 | 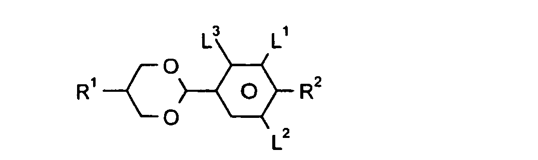 |
| **PCH** | **PDX** |
| 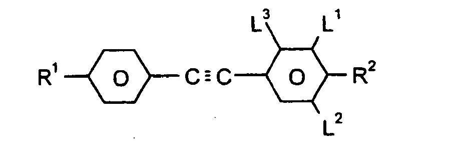 | 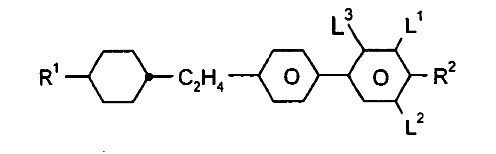 |
| **PTP** | **BECH** |
| 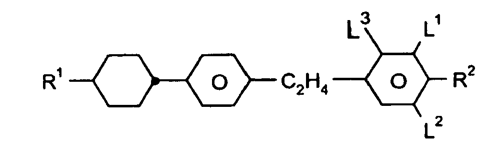 | 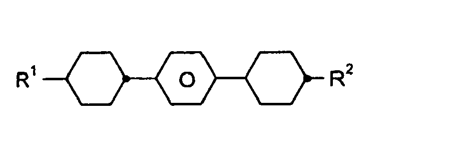 |
| **EBCH** | **CPC** |
| 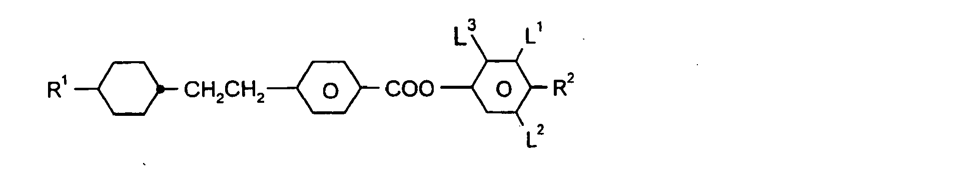 | |
| **EHP** | |
| 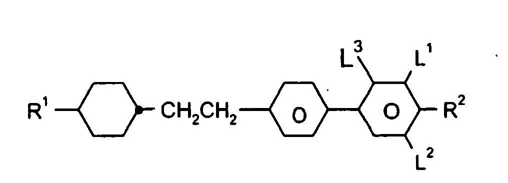 | |
| **BEP** | |
| 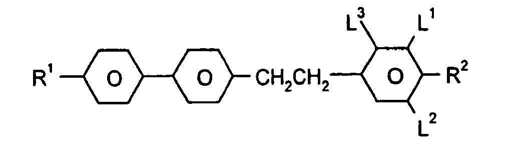 | |
| **ET** | |

**Tabelle B:**

| | |
|---|---|
| 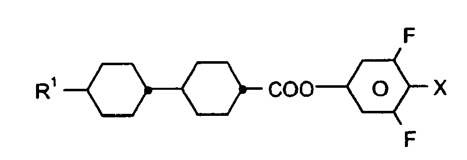 | 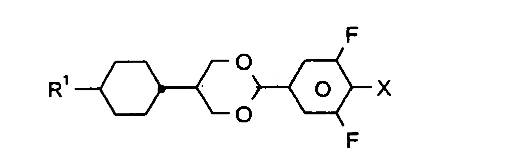 |
| **CCZU-n-X** | **CDU-n-X** |
| (X = F, Cl, -OCF3 = "OT") 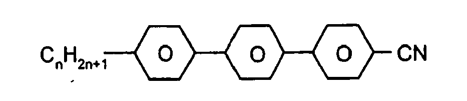 | (X = F, Cl, -OCF3 = "OT") 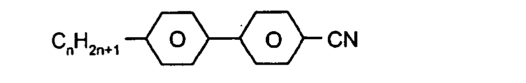 |
| **T3n** | **K3n** |
| 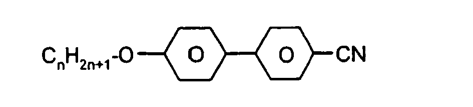 | 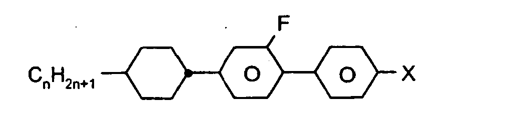 |
| **M3n** | **CGP-n-X** |
| 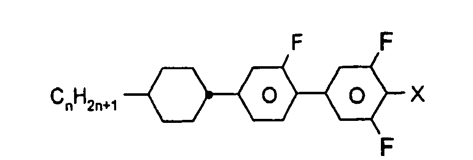 | (X = F, Cl, -OCF3 = "OT") 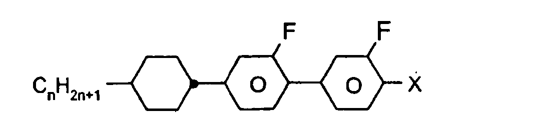 |
| **CGU-n-X** | **CGG-n-X** |
| (X = F, Cl, -OCF3 = "OT") | (X = F, Cl, -OCF3 = "OT") |
| 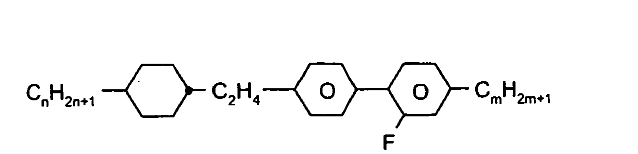 | |
| **Inm** | |
| | 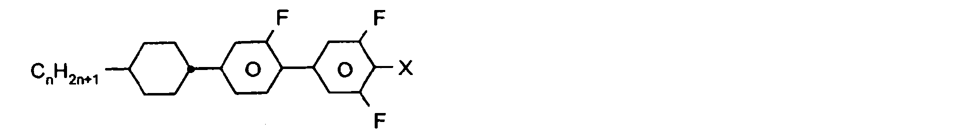 |
| **CGU-n-X** | |
| **(X = F, Cl, -OCF3 = "OT")** | |
| 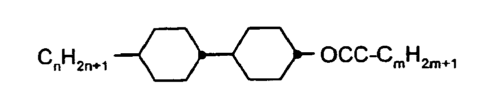 | 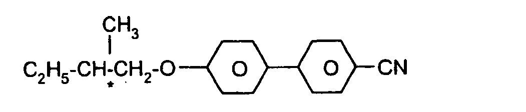 |
| | |
| **C-nm** | **C15** |
| | 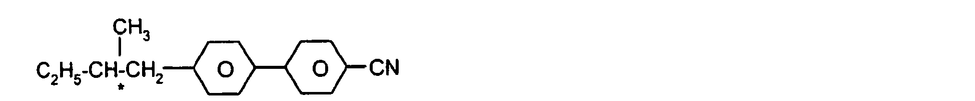 |
| **CB15** | |
| 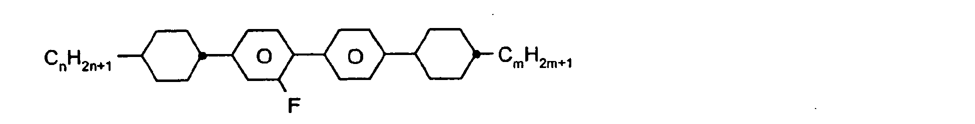 | |
| **CBC-nmF** | |
| 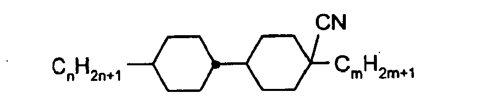 | 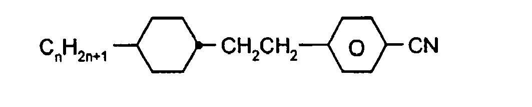 |
| | |
| **CCN-nm** | **G3n** |
| 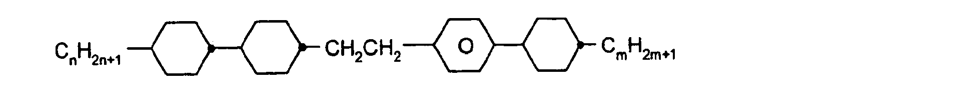 | |
| **CCEPC-nm** | |
| 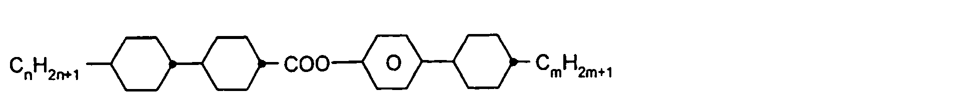 | |
| **CCPC-nm** | |
| 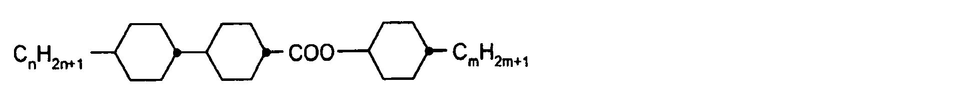 | |
| **CH-nm** | |
| 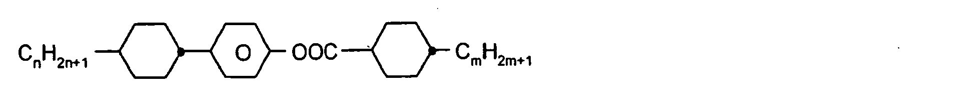 | |
| **HD-nm** | |
| 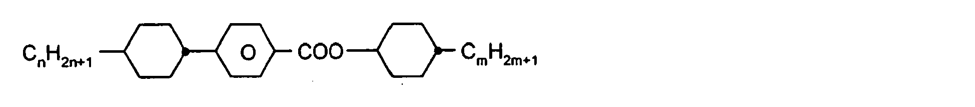 | |
| **HH-nm** | |
| 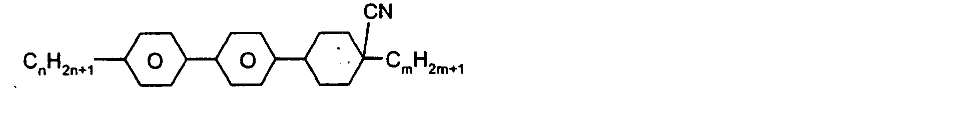 | |
| **NCB-nm** | |
| 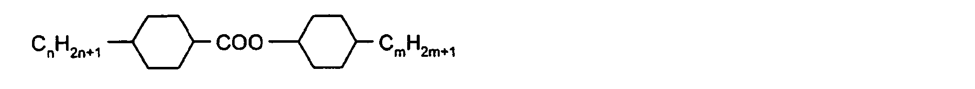 | |
| **OS-nm** | |
| 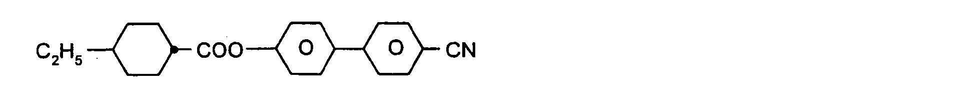 | |
| **CHE** | |
| 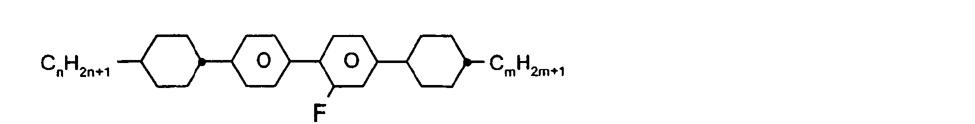 | |
| **CBC-nmF** | |
| 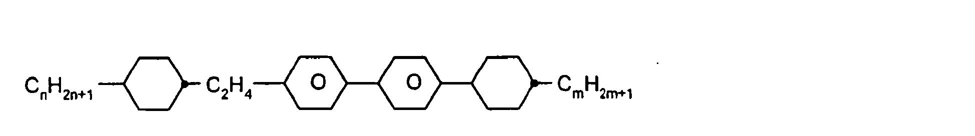 | |
| **ECBC-nm** | |
| 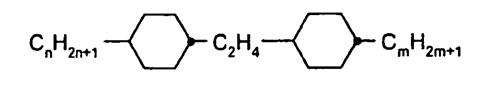 | 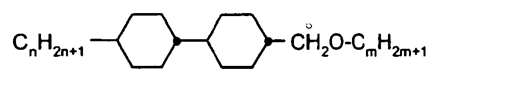 |
| **ECCH-nm** | **CCH-n1EM** |
| 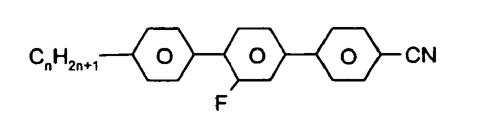 | 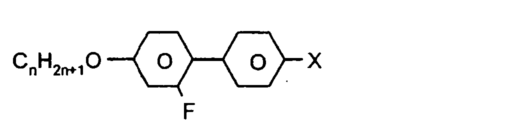 |
| **T-nFN** | **GP-nO-X** |
| | **(X = F, Cl, -OCF3 = "OT")** |
| 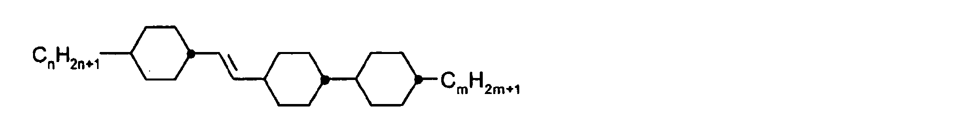 | |
| **CVCC-n-m** | |
| 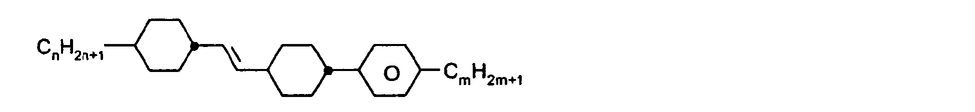 | |
| **CVCP-n-m** | |
| 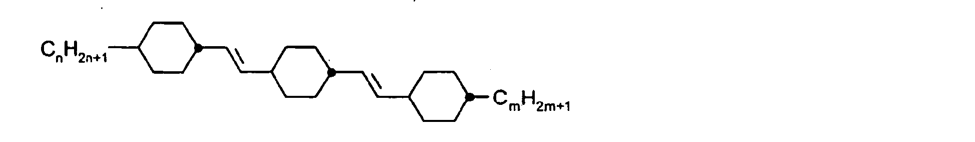 | |
| **CVCVC-n-m** | |
| 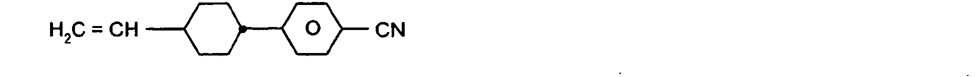 | |
| **CP-V-N** | |
| 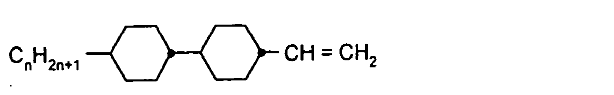 | |
| **CC-n-V** | |
| 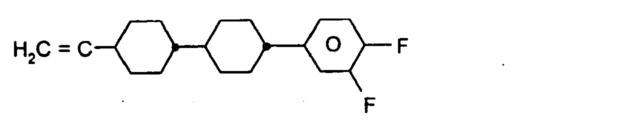 | |
| **CCG-V-F** | |
| 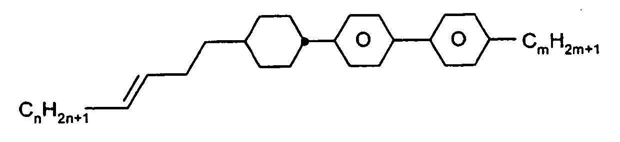 | |
| **CPP-nV2-m** | |
| 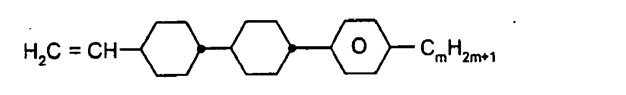 | |
| **CCP-V-m** | |
| 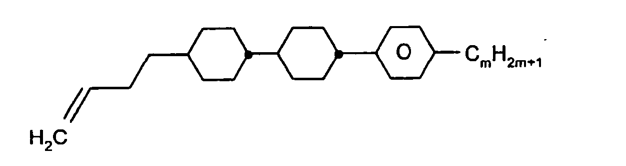 | |
| **CCP-V2-m** | |
| 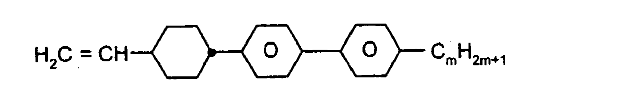 | |
| **CPP-V-m** | |
| 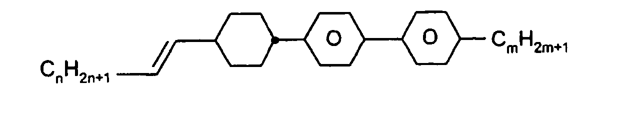 | |
| **CPP-nV-m** | |
| 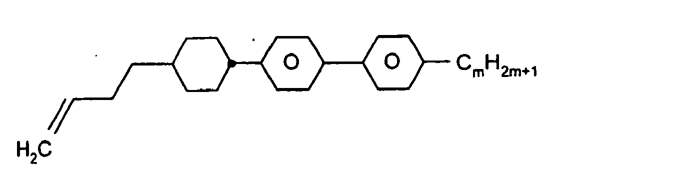 | |
| **CPP-V2-m** | |
| 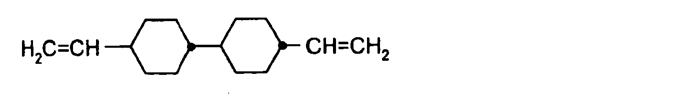 | |
| **CC-V-V** | |
| 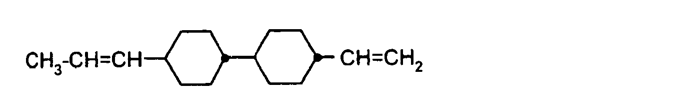 | |
| **CC-1V-V** | |
| 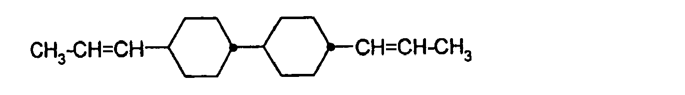 | |
| **CC-1V-V1** | |
| 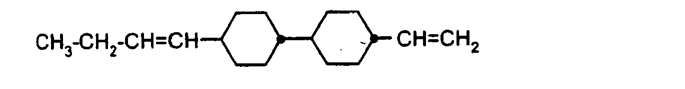 | |
| **CC-2V-V** | |
| 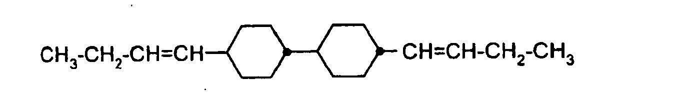 | |
| **CC-2V-V2** | |
| 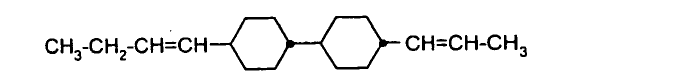 | |
| **CC-2V-V1** | |
| 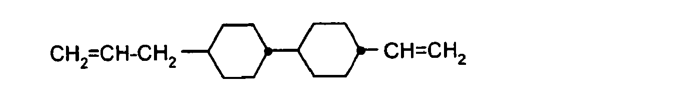 | |
| **CC-V1-V** | |
| 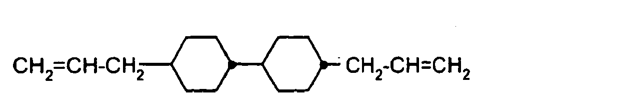 | |
| **CC-V1-1V** | |
| 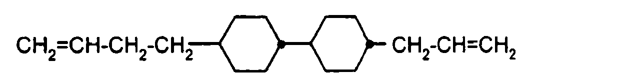 | |
| **CC-V2-1V** | |
| 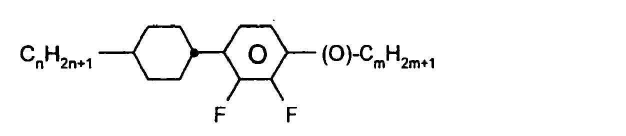 | |
| **PCH-n(O)mFF** | |
| 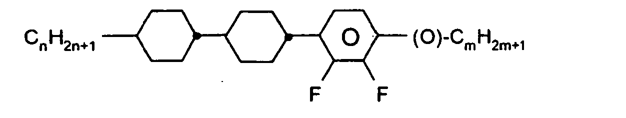 | |
| **CCP-n(O)mFF** | |
| 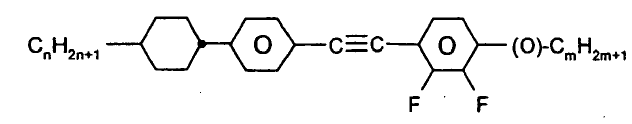 | |
| **CPTP-n(O)mFF** | |
| 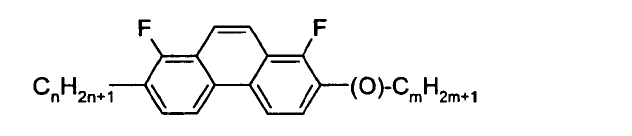 | |
| **Ph-n-(O)mFF** | |
| 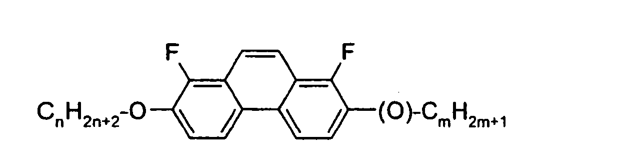 | |
| **Ph-nO-(O)mFF** | |
| 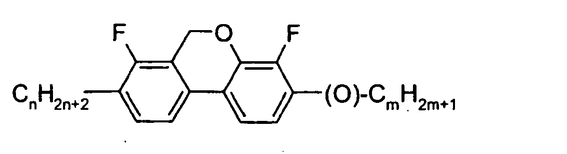 | |
| **BHHO-n-(O)mFF** | |
| 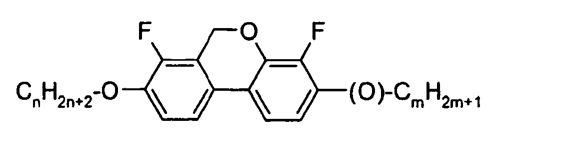 | |
| **BHHO-nO-(O)mFF** | |
| 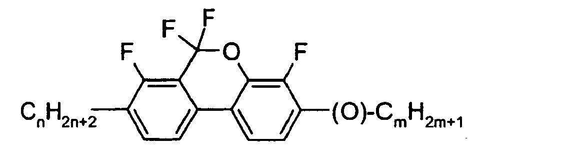 | |
| **BFFO-n-(O)mFF** | |
| 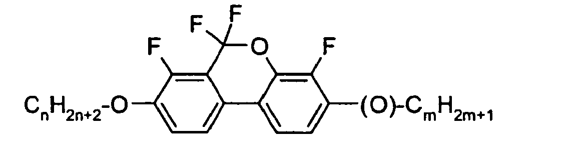 | |
| **BFFO-nO-(O)mFF** | |
| 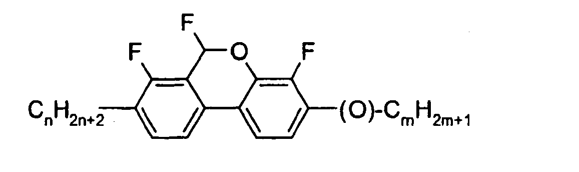 | |
| **BFO-n-(O)mFF** | |
| 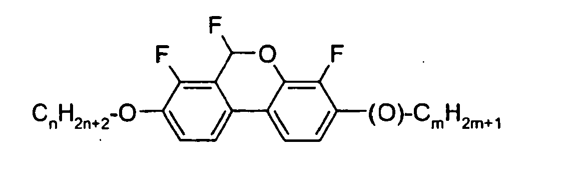 | |
| **BFO-nO-(O)mFF** | |
| 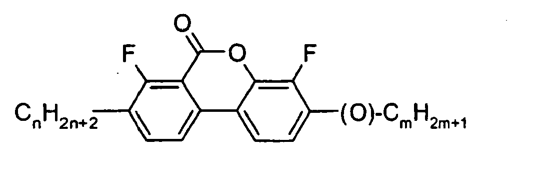 | |
| **BCOO-n-(O)mFF** | |
| 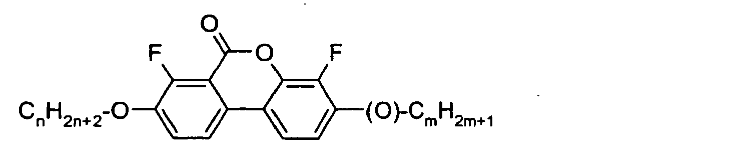 | |
| **BCOO-nO-(O)mFF** | |
| 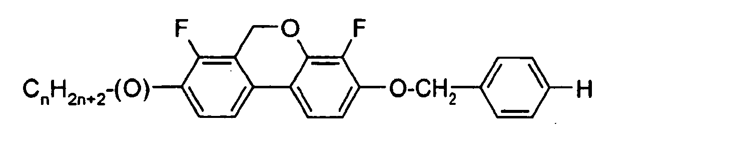 | |
| **BHHO-O1P-n(O)-HFF** | |
| 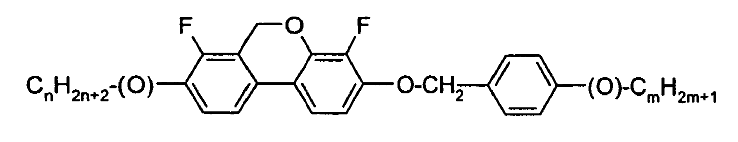 | |
| **BHHO-O1P-n(O)-(O)mFF** | |
| 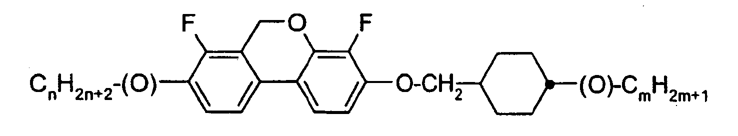 | |
| **BHHO-O1C-n(O)-(O)mFF** | |

### Beispiele

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Δn bedeutet optische Anisotropie (589 nm, 20 °C), Δε die dielektrische Anisotropie (1 kHz, 20 °C), H.R. die "voltage holding ratio" (bei 100 °C, nach 5 Minuten im Ofen, 1 V). V₁₀, V₅₀ und V₉₀ (die Schwellenspannung, Mittgrauspannung bzw. Sättigungsspannung) sowie V₀ (die kapazitive Schwellenspannung) wurden jeweils bei 20 °C bestimmt.

### Beispiel 1: 4,5-Difluor-2-(4-propyl-cyclohexyl)-8,9-dihydro-7H-furo[3,2-f]chromen

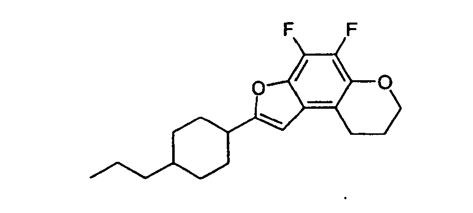

### 1.1. Herstellung von 1,2-Difluor-3-prop-2-inyloxy-benzol

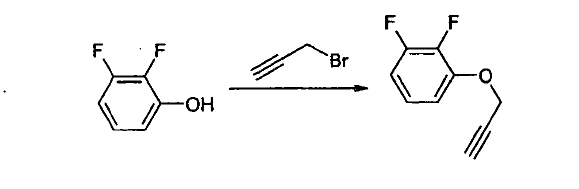

115,0 g (0.88 mol) 2,3-Difluorphenol werden zusammen mit 118,2 ml (17,7 mol) Propargylbromid (80% Lsg. in Toluol) und 146,6 g (138,2 mol) Kaliumcarbonat in 1,6 I Ethylmethylketon 3 h unter Rückfluss erhitzt. Der Ansatz wird filtriert, und der Filterrückstand wird mit MTBE gewaschen. Das Filtrat wird vollständig konzentriert, und der Rückstand wird säulenchromatographisch (SiO₂, *n-*Heptan : MTBE = 3 : 1) gereinigt.

### 1.2. Herstellung von 7,8-Difluor-2H-chromen

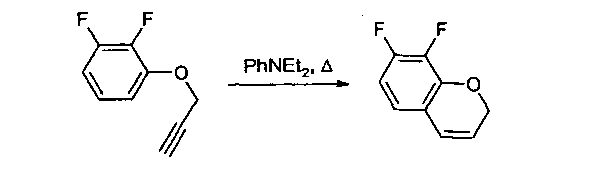

73,0 g (0,43 mol) 1,2-Difluor-3-prop-2-inyloxy-benzol werden in einem Autoklaven zusammen mit 126,0 g (2,17 mol) Kaliumfluorid in 650 ml *N,N-*Diethylanilin 3 h auf 200 °C erhitzt. Der Ansatz wird mit Wasser versetzt und mit 25 %-iger HCl sauer gestellt. Die Lösung wird mit MTBE extrahiert, und die vereinigten organischen Phasen werden mit ges. Natriumchloridlsg. gewaschen. Die Lösung wird mit Natriumsulfat getrocknet und vollständig konzentriert. Der Rückstand wird säulenchromatographisch (SiO₂, *n-*Heptan : MTBE = 10 : 1) gereinigt. Die Reaktion verläuft nicht vollständig. Auf diese Weise wird ein Gemisch aus 7,8-Difluor-2H-chromen und dem Ausgangsmaterial 1,2-Difluor-3-prop-2-inyloxy-benzol erhalten.

### 1.3. Herstellung von 7,8-Difluorchroman

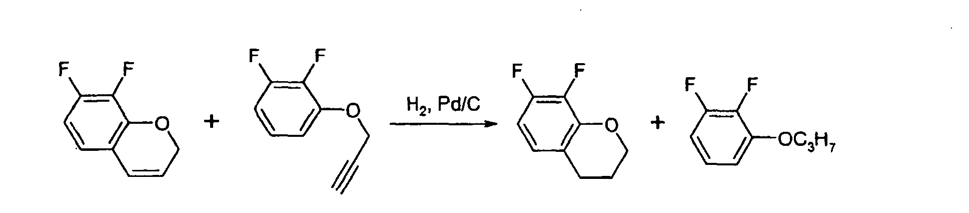

Ein Gemisch aus 7,8-Difluor-2H-chromen und 1,2-Difluor-3-prop-2-inyloxy-benzol (43,2 g) wird in 430 ml THF in Gegenwart von Pd/C (5 % Pd) 1 h bei 20°C hydriert. Der Ansatz wird vollständig konzentriert, und das Rohprodukt wird säulenchromatographisch (SiO₂, *n*-Pentan : 1-Chlorbutan = 4 : 1) gereinigt. Reines 7,8-Difluorchroman wird als leicht gelbliche Flüssigkeit erhalten.

### 1.4. Herstellung von 7,8-Difluor-chroman-6-ol

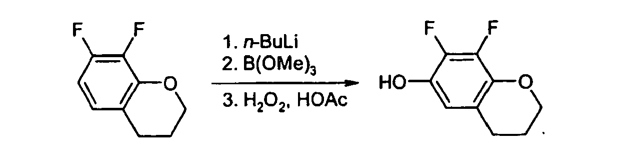

Eine Lsg. von 20,0 g (0,12 mol) 7,8-Difluorchroman in 400 ml THF wird bei -70°C mit 81,2 ml (0,13 mol) *n-*BuLi (15 % Lsg. in Hexan) versetzt. Nach 3 h bei dieser Temperatur werden 14,4 ml (0,13 mol) Trimethylborat zugetropft, und der Ansatz wird auf Raumtemperatur erwärmt. 30 ml verdünnte Essigsäure (ca. 30 %) werden zugeben, und der Ansatz wird vorsichtig mit 30 ml wäßr. Wasserstoffperoxidlsg. (35 %) versetzt. Nach beendeter Zugabe wird 17 h bei 20°C gerührt. Wasser wird zugeben und der Ansatz wird mit HCl sauer gestellt. Die Lösung wird mehrfach mit MTBE extrahiert, und die vereinigten organischen Phasen werden nacheinander mit Wasser, ges. Natriumchloridlsg. und Ammoniumeisen(II)-sulfatlsg. gewaschen. Die Lösung wird mit Natriumsulfat getrocknet und vollständig konzentriert. Das Rohprodukt wird säulenchromatographisch (SiO₂, *n-*Heptan : MTBE = 2 :1) gereinigt.

### 1.5. Herstellung von 7,8-Difluor-6-methoxymethoxy-chroman

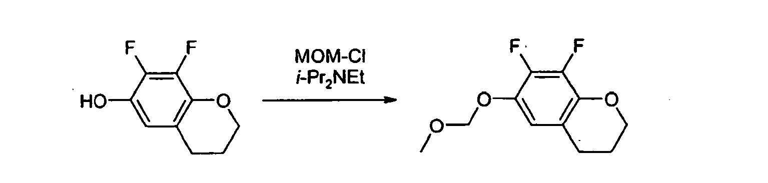

15,0 g (80,6 mmol) 7,8-Difluor-chroman-6-ol werden in 135 ml Dichlormethan gelöst, und 16,5 ml (94,2 mmol) *N*-Ethyldiisopropylamin werden unter Eiskühlung zugegeben. Nach 5 min werden 7,34 ml (97,0 mmol) Chlormethyl-methylether im Temperaturbereich von 15 bis 30°C zudosiert. Nach 18 h bei 20°C werden 50 ml Triethylamin zugegeben, und der Ansatz wird nochmals 18 h gerührt. Die Reaktionsmischung wird mit Wasser versetzt, und die organische Phase wird abgetrennt. Die wässrige Phase wird mit Dichlormethan extrahiert, und die vereinigten organischen Phasen werden nacheinander mit Wasser und ges. Natriumchloridlsg. gewaschen. Die Lösung wird mit Natriumsulfat getrocknet und vollständig konzentriert. Das Rohprodukt wird säulenchromatographisch (SiO₂, *n-*Heptan : MTBE = 2 : 1) gereinigt. Auf diese Weise wird 7,8-Difluor-6-methoxymethoxy-chroman als gelblicher Feststoff erhalten.

### 1.6. Herstellung von 7,8-Difluor-5-iod-6-methoxymethoxy-chroman

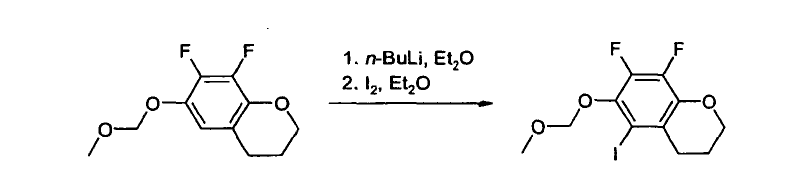

16,6 g (72,1 mmol) 7,8-Difluor-6-methoxymethoxy-chroman werden in 350 ml THF vorgelegt und bei -78°C mit 54,3 ml (86,5 mmol) *n-*BuLi (15 % Lsg. in Hexan) tropfenweise versetzt. Nach beendeter Zugabe wird 30 min bei dieser Temperatur und anschließend 1 h bei 20°C gerührt. Die Lösung wird erneut auf -78°C gekühlt, und eine Lösung von 22,0 g (86,5 mol) Iod in 100 ml THF wird zugetropft. Die Reaktionsmischung wird 1,5 h bei Raumtemperatur gerührt und anschließend mit MTBE verdünnt. Die Lösung wird mit Wasser und ges. Natriumchloridisg. gewaschen und mit Natriumsulfat getrocknet. Das nach dem Entfernen der Lösungsmittel verbleibende Rohprodukt wird säulenchromatographisch (SiO₂, *n*-Heptan : MTBE = 2 : 1) gereinigt. Auf diese Weise wird 7,8-Difluor-5-iod-6-methoxymethoxy-chroman als bräunliches Öl erhalten.

### 1.7. Herstellung von 7,8-Difluor-5-iod-chroman-6-ol

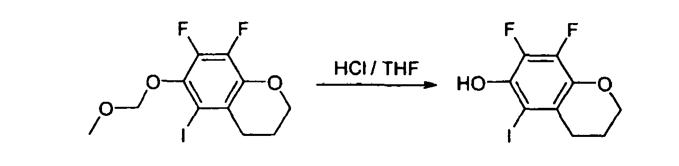

Eine Lösung von 20,2 g (56,7 mmol) 7,8-Difluor-5-iod-6-methoxymethoxychroman in 100 ml THF wird mit 10,4 ml konz. HCl versetzt, und die Mischung wird 18 h bei 20°C gerührt. Der Ansatz wird mit MTBE verdünnt, und die Lösung wird mit Wasser gewaschen. Die wässrige Phase wird mit MTBE extrahiert, und die vereinigten organischen Phasen werden mit Wasser und ges. Natriumchloridlsg. gewaschen. Die Lösung wird mit Natriumsulfat getrocknet und vollständig konzentriert. Das Rohprodukt wird säulenchromatographisch (SiO₂, *n-*Heptan : MTBE = 1 : 1) gereinigt. Auf diese Weise wird 7,8-Difluor-5-iod-chroman-6-ol als hellbrauner Feststoff erhalten.

### 1.8. Herstellung von 7,8-Difluor-5-(4-propyl-cyclohexylethinyl)-chroman-6-Ol

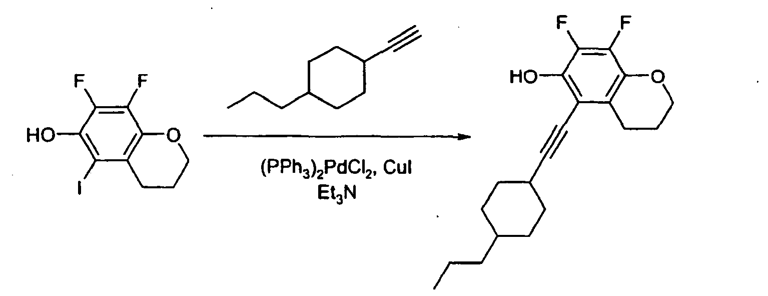

7,0 g (22,4 mmol) 7,8-Difluor-5-chroman-6-ol werden zusammmen mit 5,06 g (33,5 mmol) 1-Ethinyl-4-propyl-cyclohexan in Gegenwart von 472 mg (0,67 mmol) Bis(triphenylphosphin)palladium(II)-chlorid und 128 mg (0,67 mmol) Kupfer(1)-iodid in 90 ml Triethylamin 18 h bei 50 °C gerührt. Nach dem Abkühlen wird der Ansatz zu Eis / Wasser gegeben und mit Salzsäure sauer gestellt. Die Mischung wird mehrfach mit MTBE extrahiert, und die vereinigten Extrakte werden mit Wasser und ges. Natriumchloridlsg. gewaschen. Die Lösung wird mit Natriumsulfat getrocknet und vollständig konzentriert. Der Rückstand wird säulenchromatographisch (SiO₂, 1-Chlorbutan) gereinigt. Das erhaltene Öl wird direkt für die folgende Umsetzung verwendet.

### 1.9. Herstellung von 4,5-Difluor-2-(4-propyl-cyclohexyl)-8,9-dihydro-7H-furo[3,2-f]chromen

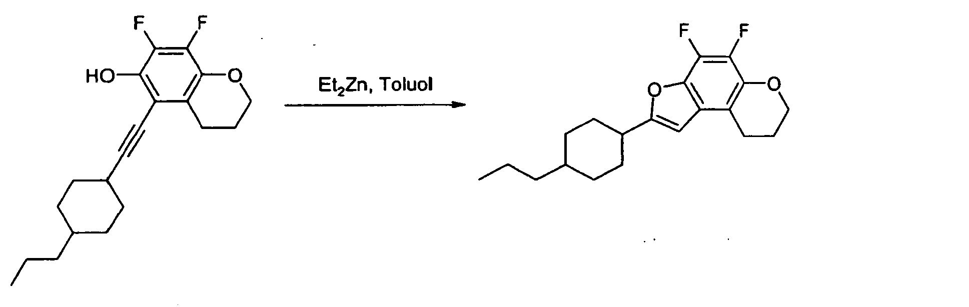

5,80 g (17,3 mmol) 7,8-Difluor-5-(4-propyl-cyclohexylethinyl)-chroman-6-ol werden zusammen mit 0,26 ml (1,73 mmol) TMEDA vorgelegt, und die Mischung wird bei 3 °C mit 9,6 ml (9,6 mmol) Diethylzink (1 M Lsg. in Heptan) versetzt. Nach dem Abklingen der Gasentwicklung werden 25 ml Toluol zugegeben, und der Ansatz wird 40 h zum Rückfluss erhitzt. Die Lösung wird zu ges. Ammoniumchloridlsg. gegeben, und das Gemisch wird mit Toluol extrahiert. Die vereinigten organischen Phasen werden mit Wasser und ges. Natriumchloridlsg. gewaschen und mit Natriumsulfat getrocknet. Die Lösung wird vollständig konzentriert, und der Rückstand wird säulenchromatographisch (SiO₂, 1-Chlorbutan : Pentan = 2 : 1) gereinigt. Weitere Reinigung erfolgt durch mehrfaches Umkristallisieren aus Ethanol bei erniedrigter Temperatur. 4,5-Difluor-2-(4-propylcyclohexyl)-8,9-dihydro-*7H*-furo[3,2-f]chromen wird als farbloser Feststoff mit einem Schmelzpunkt von 89°C erhalten (Δε = -5,0). 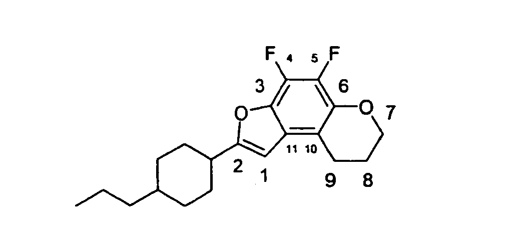 **¹H-NMR** (300 MHz, CHCl₃): δ = 6,23 (dd,1 H, *J* = 3,0 Hz, *J* = 1,0 Hz, 1-H), 4,27-4,24 (m, 2H, 7-H), 2,81 (td, 2H, *J* = 7,8 Hz, *J* = 1,5 Hz, 9-H), 2,67 (tm, 1 H, J = 12,0 Hz, H_{*aliph*.}), 2,18-2,04 (m, 4H, H_{*aliph*.}), 1,87 (dm, 2H, *J* = 12,0 Hz, H_{*aliph*.}), 1,54-1,43 (m, 2H, H*_{aliph.}*), 1,39-1,27 (m, 3H, H_{*aliph*.}), 1,26-1,27 (m, 2H, H_{*aliph*.}), 1,11-0,97 (m, 2H, H_{*aliph*.}), 0,90 (t, 3H, *J* = 7,1 Hz, CH₂CH₂**CH₃**).
**¹⁹F-NMR** (282 MHz, CHCl₃): δ = -162,7 (dm, 1 F, *J* = 19,5 Hz), -166,5 (d, 1 F, *J* = 19,5 Hz).
**MS** (EI): m/z(%) = 334 (100, M⁺), 249 (48).

### Beispiel 2: 4,5-Difluor-2-(4-propyl-cyclohexyl)-1,7,8,9-tetrahydro-2H-furo[3,2-f]-chromen

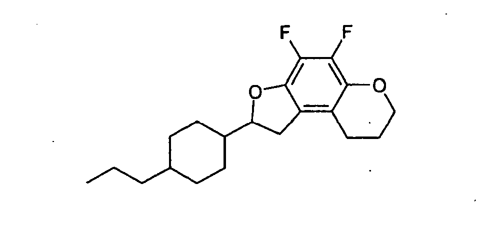

### 2.1. Herstellung von 4,5-Difluor-2-(4-propyl-cyclohexyl)-1,7,8,9-tetrahydro-2H-furo[3,2-f]-chromen

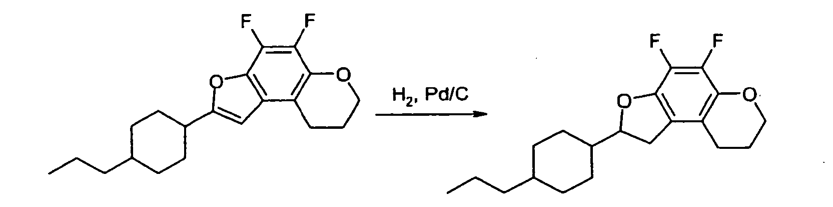

1,00 g (2,99 mmol) 4,5-Difluor-2-(4-propyl-cyclohexyl)-8,9-dihydro-*7H-*furo[3,2-f]chromen werden in THF in Gegenwart von Pd/C (5% Pd) 15 h mit elementarem Wasserstoff bei Raumtemperatur und Normaldruck hydriert. Die Reaktionslsg. wird vollständig konzentriert, und der Rückstand wird säulenchromatographisch (SiO₂, 1-Chlorbutan : Pentan = 2 : 1) gereinigt. Eine weitere Aufreinigung erfolgt durch Umkristallisieren aus Ethanol. 4,5-Difluor-2-(4-propyl-cyclohexyl)-1,7,8,9-tetrahydro-*2H*-furo[3,2-f]-chromen wird als farbloser Feststoff mit einem Schmp. von 96°C erhalten (Δε = -7,0). 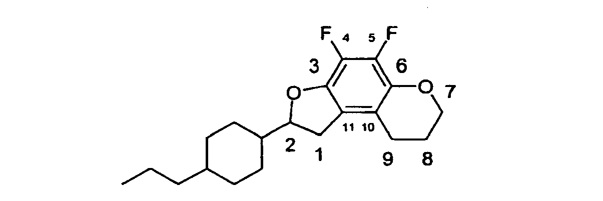 **¹H-NMR** (300 MHz, CHCl₃): δ = 4,61-4,53 (m,1 H, 2-H), 4,19-4,16 (m, 2H, 7-H), 3,04-2,96 (m, 1 H, 1-H), 2,84-2,75 (m, 1 H, 1-H), 2,60-2,54 (m, 2H, 9-H), 2,05-1,97 (m, 3H, Hₐₗᵢₚₕ.),1,81 (dm, 2H, *J* = 13,2 Hz, H_{*aliph*.}), 1,75-1,66 (m, 2H, H_{*aliph*.}), 1,64-1,56 (m, 1H, H*_{aliph.}*), 1,38-1,24 (m, 2H, H*_{aliph.}*), 1,22-1,01 (m, 4H, H*_{aliph.}*), 0,98-0,82 (m, 5H, H_{aliph.}).
**F-NMR** (282 MHz, CHCl₃): δ = -162,1 (d, 1 F, *J* = 19,5 Hz), -163,1 (d, 1 F, *J* = 19,5 Hz).
**MS** (EI): m/z(%) = 336 (82, M⁺), 199 (100).

### Beispiel 3: 4,5-Difluor-7-pentyl-2-(4-propyl-cyclohexyl)-8,9-dihydro-7H-furo[3,2-f]-chromen

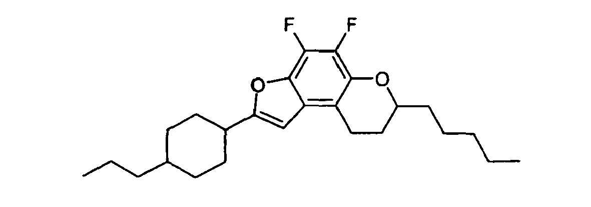

### 3.1. Herstellung von 1-(1-Ethinyl-hexyloxy)-2,3-difluor-benzol

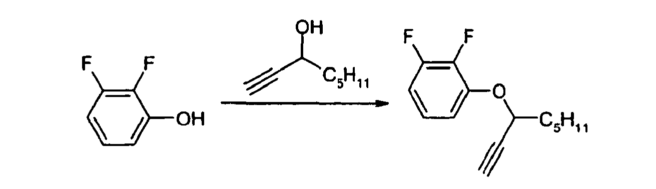

42,4 g (0,33 mol) 2,3-Difluorphenol werden zusammen mit 50,0 ml (0,34 mol) 1-Octin-3-ol und 94,1 g (0,36 mol) Triphenylphosphin in 1,2 I THF vorgelegt, und eine Lösung von 76,1 ml (0,39 mol) DIAD in 100 ml THF wird tropfenweise zugegeben. Nach 19 h bei 20°C wird mit MTBE verdünnt, und der Ansatz wird mit Wasser gewaschen. Die wässrige Phase wird mit MTBE extrahiert, und die vereinigten organischen Phasen werden mit ges. Natriumchloridlsg. gewaschen. Die Lösung wird mit Natriumsulfat getrocknet und vollständig konzentriert. Der Rückstand wird säulenchromatographisch (SiO₂, 1-Chlorbutan) gereinigt. 1-(1-Ethinyl-hexyloxy)-2,3-difluor-benzol wird als farbloses Öl erhalten.

### 3.2. Herstellung von 7,8-Difluor-2-pentyl-2H-chromen

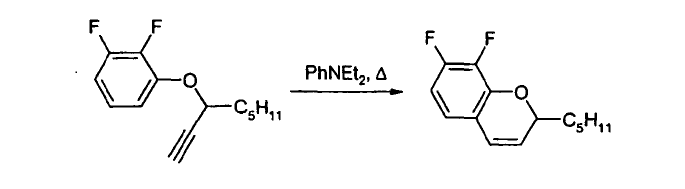

62,0 g (0,26 mol) 1-(1-Ethinyl-hexyloxy)-2,3-difluor-benzol werden in 390 ml *N,N*-Diethylanilin 2 h auf 195 °C erhitzt. Der Ansatz wird mit MTBE verdünnt und mehrfach mit 1 N HCl gewaschen. Die organische Phase wird mit Natriumsulfat getrocknet und vollständig konzentriert. Der Rückstand wird säulenchromatographisch (SiO₂, *n-*Pentan : 1-Chlorbutan = 5 : 1) gereinigt. 7,8-Difluor-2-pentyl-*2H*-chromen wird als braunes Öl erhalten.

### 3.3. Herstellung von 7,8-Difluor-2-pentyl-chroman

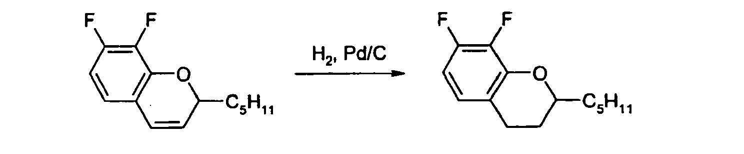

51,0 g (0,21 mol) 7,8-Difluor-2-pentyl-2H-chromen werden in 510 ml Toluol in Gegenwart von Pd/C (5 % Pd) 1 h bei Raumtemperatur hydriert. Der Ansatz wird vollständig konzentriert. Das Rohprodukt (gelbliche Flüssigkeit) kann direkt für die nächste Stufe verwendet werden.

### 3.4. Herstellung von 7,8-Difluor-2-pentyl-chroman-6-ol

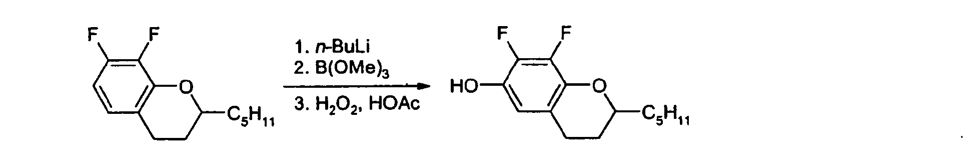

52,4 g rohes (ca. 0.22 mol) 7,8-Difluor-2-pentyl-chroman werden in 400 ml THF vorgelegt und bei -70°C mit 150,7 ml (0,24 mol) *n-*BuLi (15 % Lsg. in Hexan) versetzt. Nach 3 h bei dieser Temperatur werden 26,8 ml (0,24 mol) Trimethylborat zugetropft, und der Ansatz wird auf Raumtemperatur erwärmt. 55 ml verdünnte Essigsäure (ca. 30 %) werden zugeben, und der Ansatz wird vorsichtig mit 57 ml Wasserstoffperoxidlsg. (35 %) versetzt. Nach beendeter Zugabe wird 17 h bei Raumtemperatur gerührt. Wasser wird zugeben, und der Ansatz wird mit HCl sauer gestellt. Die Lösung wird mehrfach mit MTBE extrahiert, und die vereinigten organischen Phasen werden nacheinander mit Wasser, ges. Natriumchloridlsg. und Ammoniumeisen(II)-sulfatlsg. gewaschen. Die Lösung wird mit Natriumsulfat getrocknet und vollständig konzentriert. Das Rohprodukt wird säulenchromatographisch (SiO₂, *n-*Heptan : MTBE = 2 :1) gereinigt.

### 3.5. Herstellung von 7,8-Difluor-6-methoxymethoxy-2-pentyl-chroman

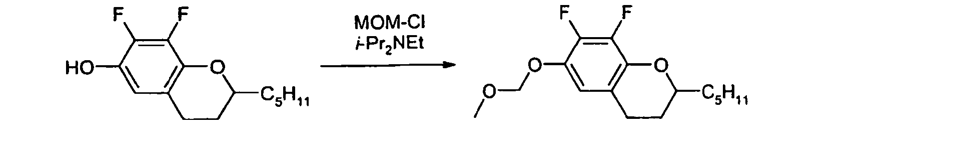

16,9 g (65,9 mmol) 7,8-Difluor-2-pentyl-chroman-6-ol werden in 110 ml Dichlormethan gelöst, und 13,5 ml (77,1 mmol) N-Ethyldiisopropylamin werden unter Eiskühlung zugegeben. Nach 5 min werden 6,0 ml (79,0 mmol) Chlormethyl-methylether im Temperaturbereich von 15 bis 30 °C zudosiert. Nach 16 h bei Raumtemperatur werden 50 ml Triethylamin zugegeben, und der Ansatz wird nochmals 24 h gerührt. Die Reaktionsmischung wird mit Wasser versetzt, und die organische Phase wird abgetrennt. Die wässrige Phase wird mit Dichlormethan extrahiert, und die vereinigten organischen Phasen werden nacheinander mit Wasser und ges. Natriumchloridlsg. gewaschen. Die Lösung wird mit Natriumsulfat getrocknet und vollständig konzentriert. Das Rohprodukt wird säulenchromatographisch (SiO₂, 1-Chlorbutan) gereinigt. Auf diese Weise wird 7,8-Difluor-6-methoxymethoxy-2-pentyl-chroman als gelbliches Öl erhalten.

### 3.6. Herstellung von 7,8-Difluor-5-iod-6-methoxymethoxy-2-pentyl-chroman

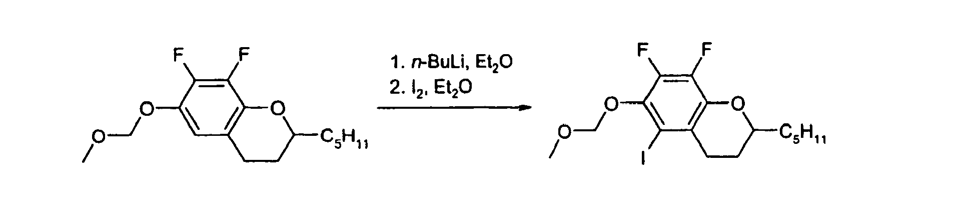

17,7 g (58,9 mmol) 7,8-Difluor-6-methoxymethoxy-2-pentyl-chroman werden in 370 ml THF vorgelegt und bei -78 °C mit 47,7 ml (75,9 mmol) *n-*BuLi (15% Lsg. in Hexan) tropfenweise versetzt. Nach beendeter Zugabe wird 30 min bei dieser Temperatur und anschließend 1 h bei Raumtemperatur gerührt. Die Lösung wird erneut auf -78 °C gekühlt, und eine Lösung von 17,9 g (70,7 mol) Iod in 100 ml THF wird zugetropft. Die Reaktionsmischung wird 90 min bei Raumtemperatur gerührt und anschließend mit MTBE verdünnt. Die Mischung wird mit ges. Natriumhydrogensulfitlsg. versetzt und anschließend mit Wasser und ges. Natriumchloridlsg. gewaschen. Das nach dem Trocknen mit Natriumsulfat und Entfernen der Lösungsmittel verbleibende Rohprodukt wird säulenchromatographisch (SiO₂, *n-*Heptan : MTBE = 3 : 1) gereinigt. Auf diese Weise wird 7,8-Difluor-5-iod-6-methoxymethoxy-2-pentyl-chroman als braunes Öl erhalten.

### 3.7. Herstellung von 7,8-Difluor-5-iod-2-pentyl-chroman-6-ol

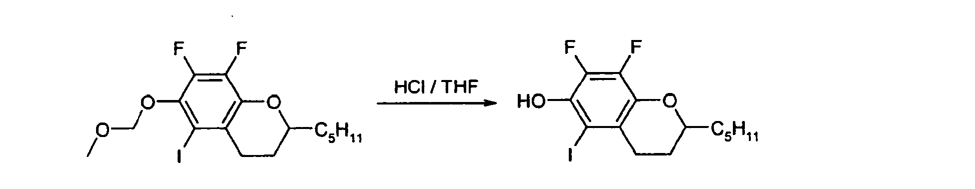

Eine Lösung von 21,4 g (50,2 mmol) 7,8-Difluor-5-iod-6-methoxymethoxy-2-pentyl-chroman in 90 ml THF wird mit 9,2 ml konz. HCI versetzt, und die Mischung wird 17 h bei Raumtemperatur gerührt. Der Ansatz wird mit MTBE verdünnt, und die Lösung wird mit Wasser gewaschen. Die wässrige Phase wird mit MTBE extrahiert, und die vereinigten organischen Phasen werden mit Wasser und ges. Natriumchloridlsg. gewaschen. Die Lösung wird mit Natriumsulfat getrocknet und vollständig konzentriert. Das Rohprodukt wird säulenchromatographisch (SiO₂, *n-*Heptan: MTBE = 1 : 1) gereinigt. Auf diese Weise wird 7,8-Difluor-5-iod-2-pentyl-chroman-6-ol als beige Feststoff erhalten.

### 3.8. Herstellung von 7,8-Difluor-2-pentyl-5-(4-propyl-cyclohexylethinyl)-chroman-6-ol

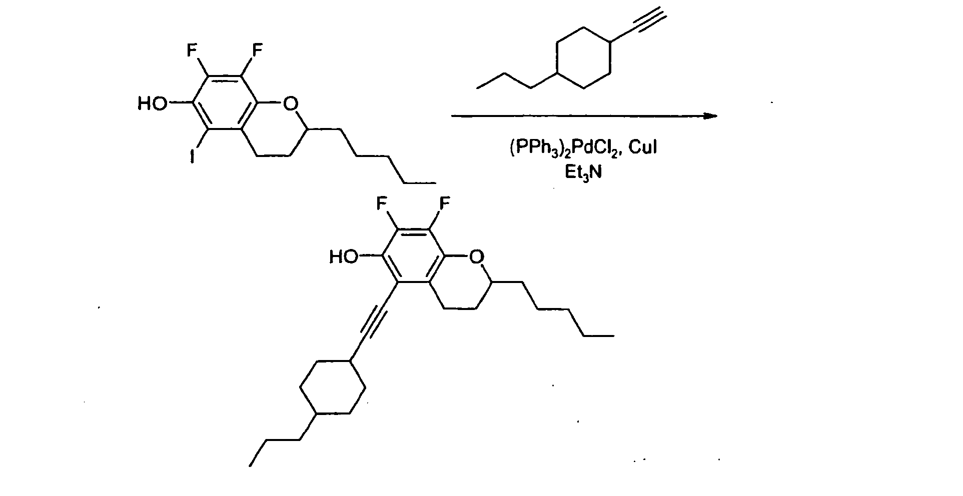

8,0 g (20,9 mmol) 7,8-Difluor-5-iod-2-pentyl-chroman-6-ol werden zusammmen mit 4,72 g (31,4 mmol) 1-Ethinyl-4-propyl-cyclohexan in Gegenwart von 441 mg (0,63 mmol) Bis(triphenylphosphin)palladium(II)-chlorid und 120 mg (0,63 mmol) Kupfer(I)-iodid in 90 ml Triethylamin 19 h bei 50 °C gerührt. Nach dem Abkühlen wird der Ansatz zu Eis / Wasser gegeben und mit Salzsäure sauer gestellt. Die Mischung wird mehrfach mit MTBE extrahiert, und die vereinigten Extrakte werden mit Wasser und ges. Natriumchloridlsg. gewaschen. Die Lösung wird mit Natriumsulfat getrocknet und vollständig konzentriert. Der Rückstand wird säulenchromatographisch (SiO₂, 1-Chlorbutan) gereinigt. Das erhaltene Phenol wird direkt für die folgende Umsetzung verwendet.

### 3.9. Herstellung von 4,5-Difluor-7-pentyl-2-(4-propyl-cyclohexyl)-8,9-dihydro-7H-furo[3,2-f]chromen

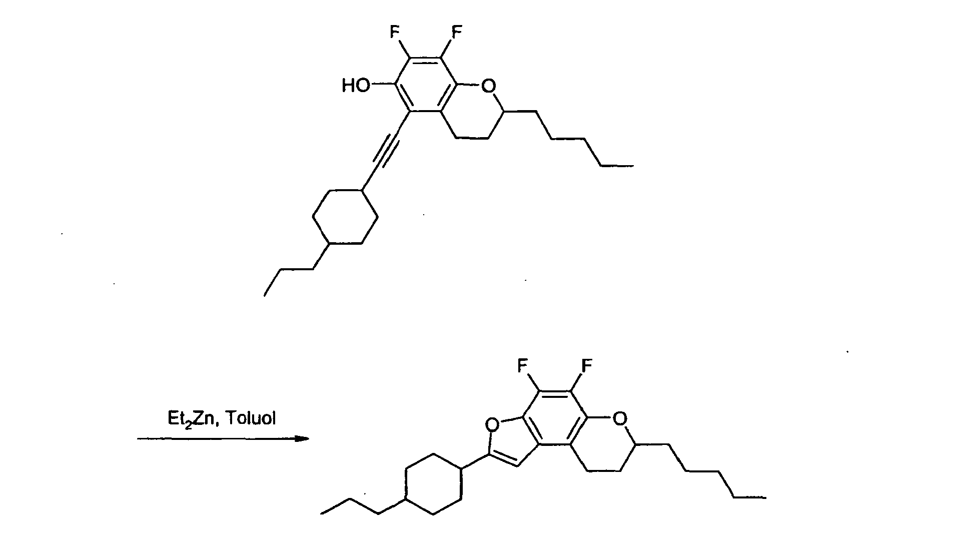

3,00 g (7,41 mmol) 7,8-Difluor-2-pentyl-5-(4-propyl-cyclohexylethinyl)-chroman-6-ol werden zusammen mit 0,11 ml (0,74 mmol) TMEDA vorgelegt, und die Mischung wird bei 3 °C mit 4,1 ml (4,1 mmol) Diethylzink (1 M Lsg. in Heptan) versetzt. Nach dem Abklingen der Gasentwicklung werden 11 ml Toluol zugegeben, und der Ansatz wird 40 h zum Rückfluss erhitzt. Die Lösung wird zu ges. Ammoniumchloridlsg. gegeben, und das Gemisch wird mit Toluol extrahiert. Die vereinigten organischen Phasen werden mit Wasser und ges. Natriumchloridlsg. gewaschen und mit Natriumsulfat getrocknet. Die Lösung wird vollständig konzentriert, und der Rückstand wird säulenchromatographisch (SiO₂, 1-Chlorbutan : Pentan = 2 : 1) gereinigt. Weitere Reinigung erfolgt durch mehrfaches Umkristallisieren aus Ethanol bei erniedrigter Temperatur. 4,5-Difluor-7-pentyl-2-(4-propyl-cyclohexyl)-8,9-dihydro-*7H*-furo[3,2-f]chromen wird als farbloser Feststoff mit der Phasensequenz K 56°C N 62°C I erhalten (Δε = -6,8). 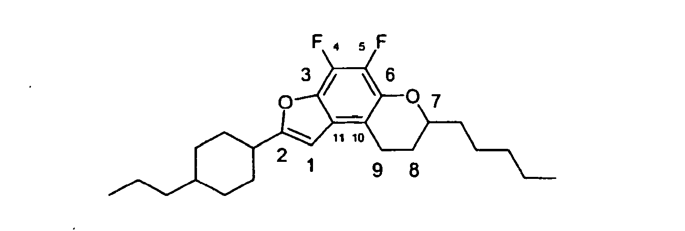 **¹H-NMR** (400 MHz, CHCl₃): δ = 6,22 (dd, 1H, *J =* 3,2 Hz, *J =* 0,8 Hz, 1-H), 4,05-3,99 (m, 1H, 7-H), 2,83-2,79 (m, 2H, 9-H), 2,67 (tm, 1H, J = 12,0 Hz, H_{aliph.}), 2,17-2,04 (m, 3H, *H_{aliph.}),* 1,90-1,75 (m, 4H, H*_{aliph.}*), 1,67-1,53 (m, 4H, H*_{aliph.}*), 1,50-1,40 (m, 2H, H*_{aliph.}*), 1,38-1,26 (m, 6H, *H*_{*a*/}*_{iph.}),* 1,25-1,18 (m, 2H, H*_{aliph.}*), 1,09-0,99 (m, 2H, *H_{aliph.}),* 0,93-0,88 (m, 6H, CH₂CH₃).
**¹⁹F-NMR** (376 MHz, CHCl₃): δ = -162,9 (dm, 1 F, *J* = 19,2 Hz), -166,2 (d, 1 F, *J* = 19,2 Hz).
**MS** (EI): m/z(%) = 404 (98, M⁺), 307 (100).

### Beispiel 4: 4,5-Difluor-7-pentyl-2-(4-propyl-cyclohexyl)-1,7,8,9-tetrahydro-2H-furo[3,2-f]chromen

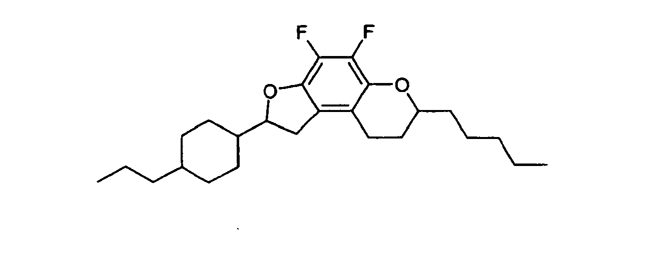

### 4.1. Herstellung von 4,5-Difluor-7-pentyl-2-(4-propyl-cyclohexyl)-1,7,8,9-tetrahydro-2H-furo[3,2-f]chromen

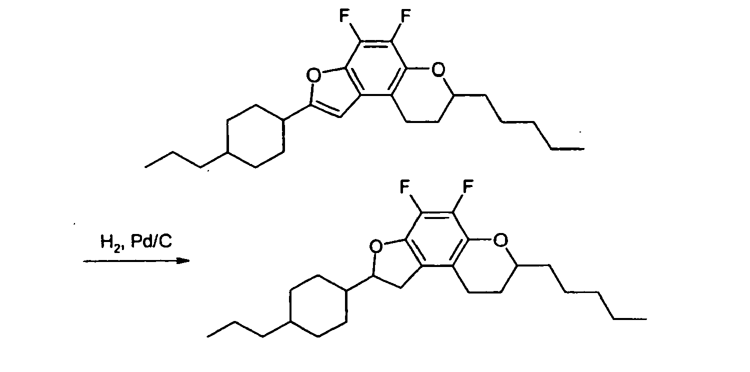

1,00 g (2,46 mmol) 4,5-Difluor-7-pentyl-2-(4-propyl-cyclohexyl)-8,9-dihydro-7H-furo[3,2-f]chromen werden in THF in Gegenwart von Pd/C (5% Pd) 19 h mit elementarem Wasserstoff bei Raumtemperatur und Normaldruck hydriert. Die Reaktionslsg. wird vollständig konzentriert, und der Rückstand wird säulenchromatographisch (SiO₂, 1-Chlorbutan : Pentan = 2 : 1) gereinigt. Eine weitere Aufreinigung erfolgt durch Umkristallisieren aus Ethanol. 4,5-Difluor-7-pentyl-2-(4-propyl-cyclohexyl)-8,9-dihydro-7H-furo[3,2-f]chromen wird als farbloser Feststoff mit einem Schmp. von 101°C erhalten (Δε = -10,5).
**¹H-NMR** (300 MHz, CHCl₃): δ = 4,61-4,52 (m,1H, 2-H), 3,97-3,88 (m, 1H, 7-H), 3,04-2,94 (m, 1H, 1-H), 2,84-2,74 (m, 1H, 1-H), 2,67-2,47 (m, 2H, 9-H), 2,05-1,96 (m, 2H, *H_{aliph.}*),1,85-1,66 (m, 4H, *H*_{*aliph*.}), 1,65-1,41 (m, 3H, *H_{aliph.}*), 1,39-1,26 (m, 5H, *H_{aliph.}*), 1,24-1,01 (m, 4H, H*_{aliph.}*), 0,98-0,82 (m, 5H, *H_{aliph.}*).
**¹⁹F-NMR** (282 MHz, CHCl₃): ö = -161,9 (dm, 1 F, *J =* 19,2 Hz), -163,8 (d, 1 F, *J* = 19,2 Hz).
**MS** (EI): m/z(%) = 406 (100, M⁺).

### Beispiel 5: 4,5-Difluor-2-methyl-7-propyl-8,9-dihydro-7H-furo[3,2-f]chromen

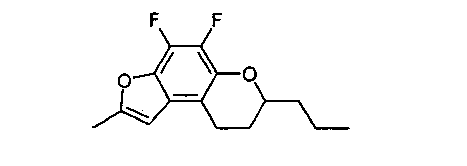

### 5.1. Herstellung von 5-Brom-1,2-difluor-3-prop-2-inyloxy-benzol

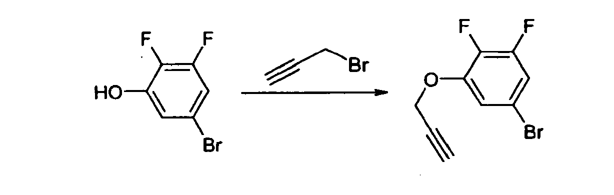

50,0 g (0,24 mol) 5-Brom-2,3-difluorphenol werden zusammen mit 32,0 ml (0,29 mol) Propargylbromid (80 % Lsg. in Toluol) und 39,7 g (0,29 mol) Kaliumcarbonat in 860 ml Ethylmethylketon 3 h unter Rückfluss erhitzt. Der Ansatz wird filtriert, und der Filterrückstand wird mit MTBE gewaschen. Das Filtrat wird vollständig konzentriert, und der Rückstand wird säulenchromatographisch (SiO₂, *n-*Heptan : MTBE = 3 : 1) gereinigt.

### 5.2. Herstellung von 4-Brom-6,7-difluor-2-methyl-benzofuran

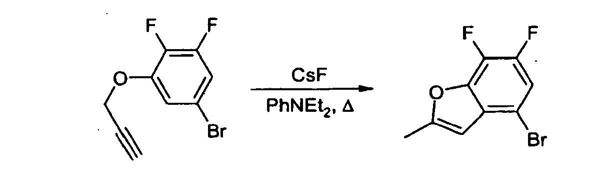

51,9 g (0,21 mol) 5-Brom-1,2-difluor-3-prop-2-inyloxy-benzol werden zusammen mit 20,7 g (0,14 mol) Cäsiumfluorid in 300 ml *N*,*N*-Diethylanilin 4 h auf 205°C erhitzt. Der Ansatz wird mit MTBE verdünnt und mehrfach mit 1 N HCI gewaschen. Die Lösung wird mit Natriumsulfat getrocknet und vollständig konzentriert. Der Rückstand wird säulenchromatographisch (SiO₂, *n-*Heptan : 1-Chlorbutan = 3 : 1) gereinigt. 4-Brom-6,7-difluor-2-methyl-benzofuran wird als gelber Feststoff erhalten.

### 5.3. Herstellung von 4-Brom-6,7-difluor-2-methyl-benzofuran-5-ol

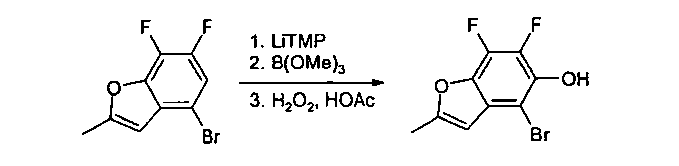

107,1 ml (0,17 mol) *n-*BuLi (15 % Lsg. in Hexan) werden bei -70°C in 150 ml THF vorgelegt und mit 29,0 ml (0,17 mol) 2,2,6,6-Tetramethylpiperidin versetzt. Nach 30 min bei dieser Temperatur wird eine Lösung von 38,3 g (0,16 mol) 4-Brom-6,7-difluor-2-methyl-benzofuran in 100 ml THF zudosiert. Nach 3 h bei dieser Temperatur werden 19,1 ml (0,17 mol) Trimethylborat zugetropft, und der Ansatz wird auf Raumtemperatur erwärmt. 40 ml verdünnte Essigsäure (ca. 30 %) werden zugeben, und der Ansatz wird vorsichtig mit 40 ml wässriger. Wasserstoffperoxidlsg. (35 %) versetzt. Nach beendeter Zugabe wird 18 h bei 20°C gerührt. Wasser wird zugeben, und der Ansatz wird mit HCl sauer gestellt. Die Lösung wird mehrfach mit MTBE extrahiert, und die vereinigten organischen Phasen werden nacheinander mit Wasser, ges. Natriumchloridlsg. und Ammoniumeisen(II)-sulfatlsg. gewaschen. Die Lösung wird mit Natriumsulfat getrocknet und vollständig konzentriert. Das Rohprodukt wird säulenchromatographisch (SiO₂, *n-*Heptan : 1-Chlorbutan = 2 :1) gereinigt. 4-Brom-6,7-difluor-2-methyl-benzofuran-5-ol wird als beige Feststoff erhalten.

### 5.4. Herstellung von 4-Brom-6,7-difluor-5-(2-methoxy-ethoxymethoxy)-2-methyl-benzofuran

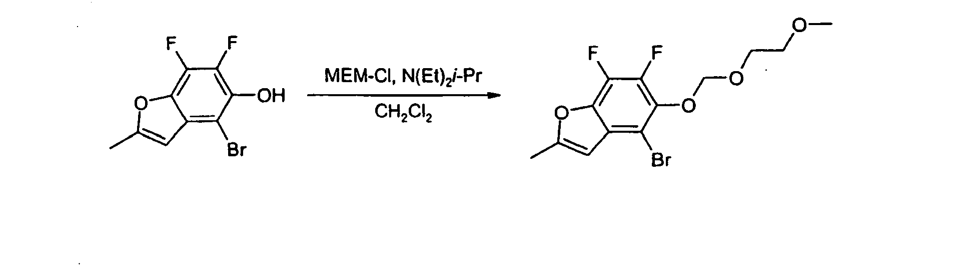

23,0 g (87,4 mmol) 4-Brom-6,7-difluor-2-methyl-benzofuran-5-ol werden bei 0°C in 120 ml Dichlormethan vorgelegt und nacheinander mit 17,9 ml (0,11 mol) *N*-Ethyldiisopropylamin und 11,9 ml (0,11 mol) MEMCI versetzt. Der Ansatz wird 16 h bei 20°C gerührt, und überschüssiges MEMCI wird mit Triethylamin gequencht. Wasser wird zugeben, und die organische Phase wird abgetrennt. Die wässrige Phase wird mit Dichlormethan extrahiert, und die vereinigten organischen Phasen werden mit Wasser und ges. Natriumchloridlsg. gewaschen. Die Lösung wird mit Natriumsulfat getrocknet und vollständig konzentriert. Das Rohprodukt wird säulenchromatographisch (SiO₂, *n-*Heptan : MTBE = 2 : 1) gereinigt.

### 5.5. Herstellung von 6,7-Difluor-5-(2-methoxy-ethoxymethoxy)-2-methyl-benzofuran-4-carbaldehyd

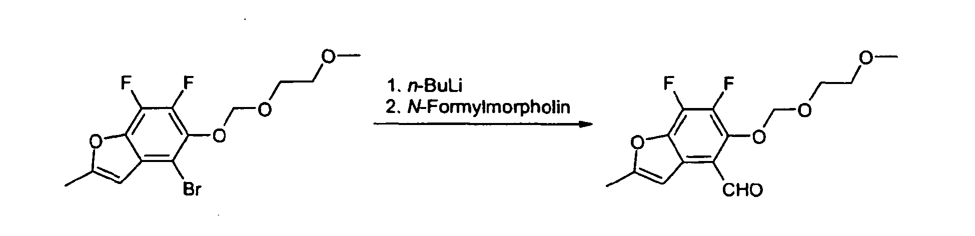

27,2 g (77,5 mmol) 4-Brom-6,7-difluor-5-(2-methoxy-ethoxymethoxy)-2-methyl-benzofuran werden bei -75°C in 500 ml THF vorgelegt, und 52,0 ml(85,2 mmol) *n-*BuLi (15 % Lsg. in Hexan) werden zugegeben. Nach 2 h bei dieser Temperatur werden 15,5 ml(155 mmol) *N-*Formylmorpholin zudosiert, und der Ansatz wird 2 h bei dieser Temperatur gerührt. Die Reaktionslsg. wird langsam auf -10°C erwärmt und mit verd. HCl hydrolysiert. Der Ansatz wird mit MTBE extrahiert, und die vereinigten organischen Phasen werden mit ges. Natriumchloridlsg. gewaschen. Die Lösung wird mit Natriumsulfat getrocknet und vollständig konzentriert. Der Rückstand wird säulenchromatographisch (SiO₂, *n-*Heptan : MTBE = 2 : 1 → *n*-Heptan : MTBE = 1 : 1) gereinigt.

### 5.6. Herstellung von 6,7-Difluor-5-hydroxy-2-methyl-benzofuran-4-carbaldehyd

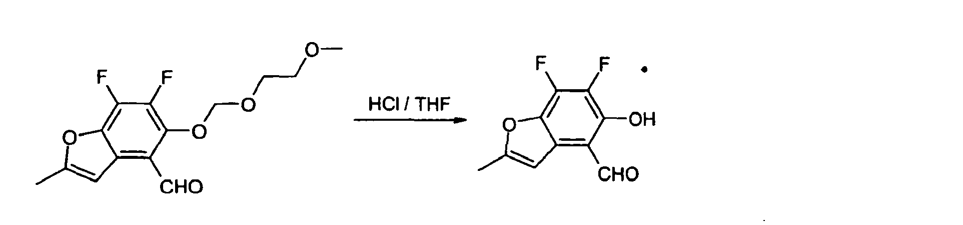

18,0 g (60,0 mmol) werden zusammen mit 11,0 ml konz. HCl in 100 ml THF 17 h bei 20°C gerührt. Die Reaktionslsg. wird mit MTBE verdünnt und mit Wasser gewaschen. Die wässrige Phase wird mit MTBE extrahiert, und die vereinigten organischen Phasen werden nacheinander mit Wasser und ges. Natriumchloridlsg. gewaschen. Die Lösung wird mit Natriumsulfat getrocknet und vollständig konzentriert. Das Rohprodukt wird säulenchromatographisch (SiO₂, 1-Chlorbutan) gereinigt. 6,7-Difluor-5-hydroxy-2-methyl-benzofuran-4-carbaldehyd wird als gelber kristalliner Feststoff erhalten.

### 5.7. Herstellung von 4,5-Difluor-2-methyl-7-propyl-7H-furo[3,2-f]chromen

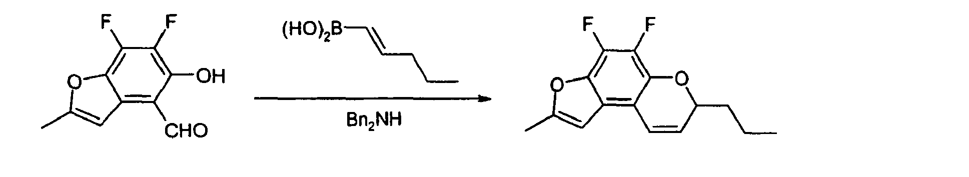

4,0 g (18,9 mmol) 6,7-Difluor-5-hydroxy-2-methyl-benzofuran-4-carbaldehyd werden zusammen mit 2,79 g (24,5 mmol) 1-Pentenboronsäure und 0,72 ml (3,73 mmol) Dibenzylamin in 95 ml 1,4-Dioxan 20 h bei 90°C gerührt. Die Reaktionsmischung wird mit Wasser versetzt und mit MTBE extrahiert. Die organische Phase wird abgetrennt, und die wässrige Phase wird mit MTBE extrahiert. Die vereinigten organischen Phasen werden mit Wasser und ges. Natriumchloridlsg. gewaschen. Die Lösung wird mit Natriumsulfat getrocknet und vollständig konzentriert. Das Rohprodukt wird zunächst säulenchromatographisch (SiO₂, 1-Chlorbutan) gereinigt und anschließend aus Methanol kristallisiert. 4,5-Difluor-2-methyl-7-propyl-*7H*-furo[3,2-f]chromen wird als gelber, kristalliner Feststoff erhalten.

### 5.8. Herstellung von 4,5-Difluor-2-methyl-7-propyl-8,9-dihydro-7H-furo[3,2-f]chromen

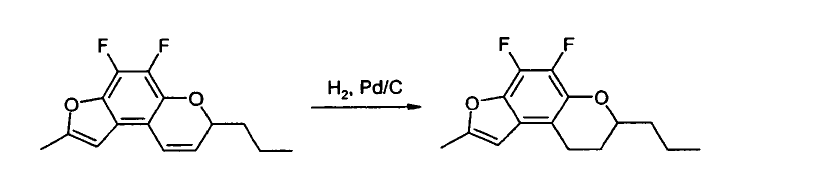

3,5 g (13,.2 mmol) 4,5-Difluor-2-methyl-7-propyl-*7H*-furo[3,2-f]chromen werden in Toluol in Gegenwart von Pd/C (5 % Pd) wenige Minuten mit elementarem Wasserstoff hydriert. Die Reaktionslsg. wird vollständig konzentriert, und der Rückstand wird säulenchromatographisch (SiO₂, *n-*Heptan : 1-Chlorbutan = 2 : 1) gereinigt. Die weitere Reinigung erfolgt durch Umkristallisation aus Ethanol und anschließende absorptive (SiO₂, *n-*Heptan : 1-Chlorbutan = 3 : 1) Filtration. 4,5-Difluor-2-methyl-7-propyl-8,9-dihydro-*7H*-furo[3,2-f]chromen wird als farbloser Feststoff mit einem Schmp. von 75°C erhalten (Δε = -7,8). 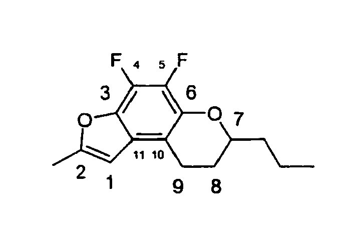 **¹H-NMR** (250 MHz, CHCl₃): δ = 6,26 (q, 1H, ⁴J = 1.0 Hz, 1-H), 4,09-4,00 (m, 1H, 7-H), 2,83-2,77 (m, 2-H, 9-H), 2,43 (s, 3H, 2-Me), 2,12-2,02 (m, 1H, 8-H), 1,86-1,70 (m, 2H, H*_{aliph.}),* 1,69-1,45 (m, 3H, 8-H, H*_{aliph.}*), 0,99 (t, 3H, CH₂CH₂CH₃).
**¹⁹F-NMR** (235 MHz, CHCl₃): δ = -164,2 (dm, 1 F, J = 19,5 Hz), -167,4 (d, 1F, J = 19,5 Hz).
**MS** (EI): m/z(%) = 266 (37, M⁺), 223 (6, [M - C₃H₇]⁺), 197 (100).

### Beispiel 6: 4,5-Difluor-2-methyl-7-propyl-1,7,8,9-tetrahydro-2H-furo[3,2-f]chromen

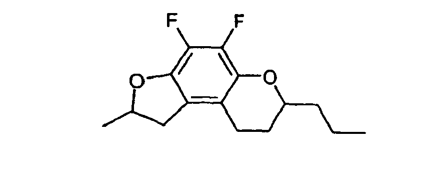

### 6.1. Herstellung von 4,5-Difluor-2-methyl-7-propyl-1,7,8,9-tetrahydro-2H-furo[3,2-f]chromen

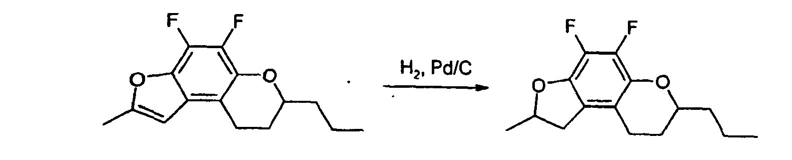

2,0 g (7,51 mmol) 4,5-Difluor-2-methyl-7-propyl-8,9-dihydro-*7H*-furo[3,2-f]chromen werden in THF in Gegenwart von Pd/C (5% Pd) 18 h mit elementarem Wasserstoff bei erhöhter Temperatur hydriert. Die Reaktionsisg. wird vollständig konzentriert, und der Rückstand wird säulenchromatographisch (SiO₂, 1-Chlorbutan) gereinigt. 4,5-Difluor-2-methyl-7-propyl-1,7,8,9-tetrahydro-*2H*-furo[3,2-f]chromen wird als farbloser Feststoff mit einem Schmp. von 87°C erhalten (Δε = -10,1). 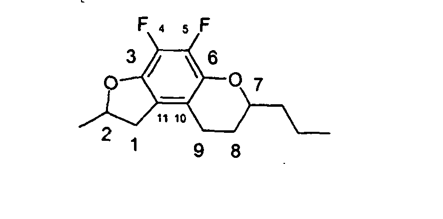 **¹H-NMR** (250 MHz, CHCl₃): δ = 5,06-4,92 (m, 1H, 2-H), 4,00-3,90 (m, 1H, 7-H), 3,21-3,09 (m, 1H, 1-H), 2,71-2,46 (m, 3H, 1-H, 9-H), 2,06-1,95 (m, 1H, 8-H), 1,80-1,53 (m, 5H, 8-H, **CH₂CH₂**CH₃), 1,48 (d, 3H, J = 6.3 Hz, 2-Me), 0,97 (t, 3H, J = 7,0 Hz, CH₂CH₂**CH₃**).
**¹⁹F-NMR** (235 MHz, CHCl₃): δ = -160,6 (dm, 1 F, J = 19,5 Hz), -162,8 (d, 1F, J = 19,5 Hz).
**MS** (EI): m/z(%) = 268 (89, M⁺), 225 (6, [M - C₃H₇]⁺), 199 (100).

### Beispiel 7: 4,5-Difluor-2-methyl-7-pentyl-8,9-dihydro-7H-furo[3,2-f]chromen

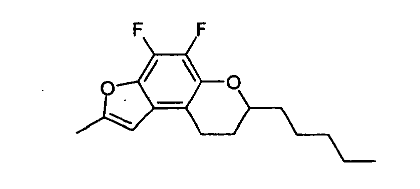

### 7.1. Herstellung von 4,5-Difluor-2-methyl-7-pentyl-7H-furo[3,2-f]chromen

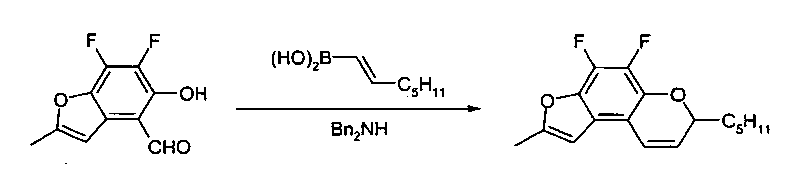

4,0 g (18,6 mmol) 6,7-Difluor-5-hydroxy-2-methyl-benzofuran-4-carbaldehyd werden zusammen mit 3,21 g (22,6 mmol) 1-Heptenboronsäure und 0,72 ml (3,73 mmol) Dibenzylamin in 95 ml 1,4-Dioxan 20 h bei 90°C gerührt. Die Reaktionsmischung wird mit Wasser versetzt und mit MTBE extrahiert. Die organische Phase wird abgetrennt, und die wässrige Phase wird mit MTBE extrahiert. Die vereinigten organischen Phasen werden mit Wasser und ges. Natriumchloridlsg. gewaschen. Die Lösung wird mit Natriumsulfat getrocknet und vollständig konzentriert. Das Rohprodukt wird zunächst säulenchromatographisch (SiO₂, 1-Chlorbutan) gereinigt und anschließend aus Methanol kristallisiert. 4,5-Difluor-2-methyl-7-pentyl-7*H*-furo[3,2-f]chromen wird als gelber, kristalliner Feststoff erhalten.

### 7.2. Herstellung von 4,5-Difluor-2-methyl-7-pentyl-8,9-dihydro-7H-furo[3,2-f]chromen

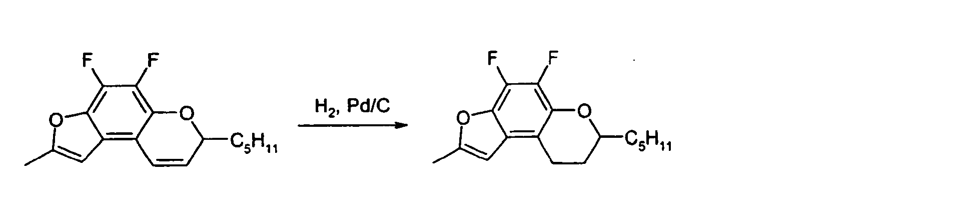

2,8 g (9,58 mmol) 4,5-Difluor-2-methyl-7-pentyl-7*H*-furo[3,2-f]chromen werden in Toluol in Gegenwart von Pd/C (5 % Pd) wenige Minuten mit elementarem Wasserstoff hydriert. Die Reaktionslsg. wird vollständig konzentriert, und der Rückstand wird säulenchromatographisch (SiO₂, *n-*Heptan : 1-Chlorbutan = 2 : 1) gereinigt. Die weitere Reinigung erfolgt durch Umkristallisation aus Ethanol. 4,5-Difluor-2-methyl-7-pentyl-8,9-dihydro-7*H*-furo[3,2-f]chromen wird als farbloser Feststoff mit einem Schmp. von 61 °C erhalten (Δε = -6,7). 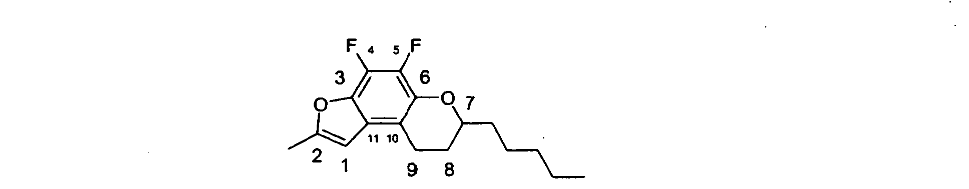 **¹H-NMR** (500 MHz, CHCl₃): δ = 6,27 (bs, 1H, 1-H), 4,06-4,01 (m, 1H, 7-H), 2,82-2,79 (m, 2H, 9-H), 2,44 (s, 3H, 2-Me), 2,10-2,05 (m, 1H, H_{*aliph*.}), 1,86-1,75 (m, 2H, *H_{aliph.}*), 1,67-1,60 (m, 1H, H*_{aliph.}*), 1,59-1,54 (m, 1H, *H_{aliph.}*), 1,51-1,43 (m, 1H, H*_{aliph.}*), 1,37-1,33 (m, 4H, H*_{aliph.}*), 0,91 (t, 3H, J = 7,0Hz, (CH₂)₄CH₃).
**¹⁹F-NMR** (235 MHz, CHCl₃): δ = -163,2 (dm, 1 F, J = 19,5 Hz), -166,4 (d, 1F, J = 19,5 Hz).
**MS** (EI): m/z(%) = 294 (27, M⁺), 223 (6, [M - C₅H₁₁]⁺), 197 (100).

### Beispiel 8: 4,5-Difluor-2-methyl-7-pentyl-1,7,8,9-tetrahydro-2H-furo[3,2-f]chromen

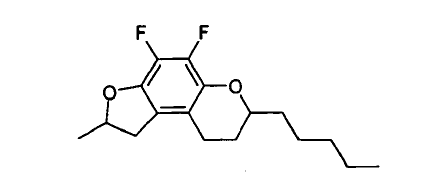

### 8.1. Herstellung von 4,5-Difluor-2-methyl-7-pentyl-1,7,8,9-tetrahydro-2H-furo[3,2-f]chromen

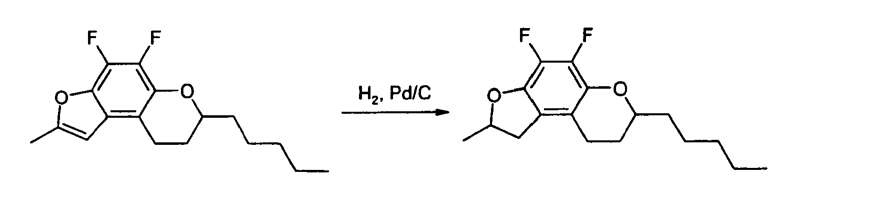

2,0 g (7,51 mmol) 4,5-Difluor-2-methyl-7-entyl-8,9-dihydro-*7H*-furo[3,2-f]chromen werden in THF in Gegenwart von Pd/C (5 % Pd) 18 h mit elementarem Wasserstoff bei erhöhter Temperatur hydriert. Die Reaktionslsg. wird vollständig konzentriert, und der Rückstand wird säulenchromatographisch (SiO₂, n-Heptan : MTBE = 4 : 1) gereinigt. Weitere Reinigung erfolgt durch Umkristallisation aus Ethanol bei 5 °C. 4,5-Difluor-2-methyl-7-pentyl-1,7,8,9-tetrahydro-2*H*-furo[3,2-f]chromen wird als farbloser Feststoff mit einem Schmp. von 76°C erhalten (Δε = -8,2). 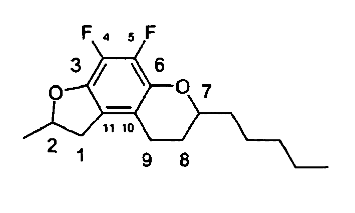 **¹H-NMR** (250 MHz, CHCl₃): δ = 5,07-4,92 (m, 1H, 2-H), 3,99-3,88 (m, 1H, 7-H), 3,22-3,09 (m, 1H, 1-H), 2,71-2,53 (m, 3H, 1-H, 9-H), 2,06-1,96 (m, 1H, 8-H), 1,80-1,51 (m, 5H, H*_{aliph.}*), 1,48 (d, 3H, J = 6.3 Hz, 2-Me), 1,36-1,32 (m, 4H, H_{*a*/*iPh.*}), 0,91 (t, 3H, J = 7.0 Hz, (CH₂)₄**CH₃**).
**F-NMR** (235 MHz, CHCl₃): δ = -162,1 (dm, 1 F, J = 19,5 Hz), -164,2 (d, 1F, J = 19,5 Hz).
**MS** (EI): m/z(%) = 296 (98, M⁺), 199 (100).

### Beispiel 9: 4,5-Difluor-7-pentyl-2-(4-propyl-p henyl)-8,9-dihydro-7H-furo[3,2-f]chromen

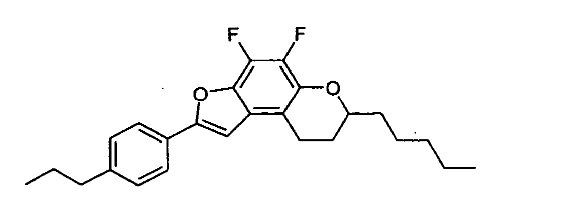

### 9.1. Herstellung von 4,5-Difluor-7-pentyl-2-(4-propyl-p henyl)-8,9-dihydro-7H-furo[3,2-f]chromen

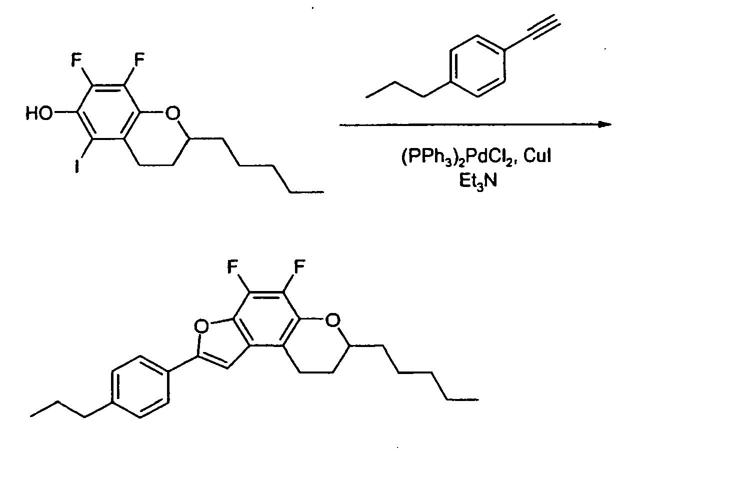

2,0 g (5,23 mmol) 7,8-Difluor-5-iod-2-pentyl-chroman-6-ol werden zusammmen mit 1,13 g (7,85 mmol) Ethinyl-*n-*propylbenzol in Gegenwart von 110 mg (0,16 mmol) Bis(triphenylphosphin)palladium(II)-chlorid und 30 mg (0,16 mmol) Kupfer(1)-iodid in 22 ml Triethylamin zunächst 6 h bei 60 °C gerührt. Die Mischung wird anschließend 18 h zum Rückfluss erwärmt. Nach dem Abkühlen wird der Ansatz zu Eis / Wasser gegeben und mit Salzsäure sauer gestellt. Die Mischung wird mehrfach mit MTBE extrahiert, und die vereinigten Extrakte werden mit Wasser und ges. Natriumchloridlsg. gewaschen. Die Lösung wird mit Natriumsulfat getrocknet und vollständig konzentriert. Der Rückstand wird säulenchromatographisch (SiO₂, Pentan : 1-Chlorbutan = 10 : 1) gereinigt. Weitere Aufreinigung erfolgt durch Umkristallisation aus Ethanol bei 5 °C. 4,5-Difluor-7-pentyl-2-(4-propyl-phenyl)-8,9-dihydro-7H-furo[3,2-f]chromen wird als Feststoff mit der Phasenfolge Tg -31 °C K 70°C N 79°C I erhalten (Δε = -7,2). 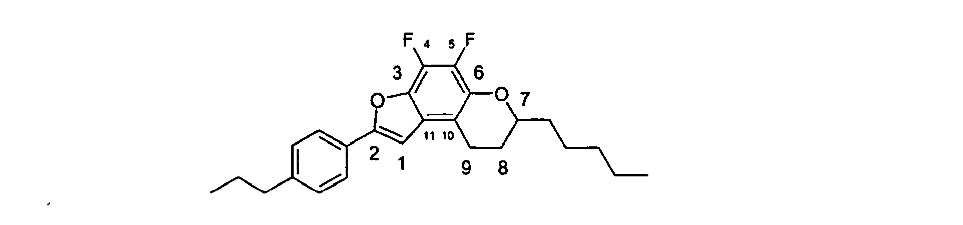 **¹H-NMR** (250 MHz, CHCl₃): δ = 7,72 (d, 2H, *J* = 8,3 Hz, *Hₐᵣₒₘ.),* 7,23 (d, 2H, *J* = 8,3 Hz, *H_{arom.}*), 6,81 (d, 1H, *J =* 2,7 Hz), 4,08-4,00 (m, 1H, 7-H), 2,88-2,83 (m, 2H, 9-H), 2,62 (t, 2H, J = 7,8 Hz, H*_{benzyl.}*), 2,14-2,05 (m, 1H, H*ₐₗᵢₚₕ*), 1,90-1,76 (m, 2H, H_{aliph.}), 1,73-1,61 (m, 4H, H*_{aliph.}*), 1,57-1,44 (m, 1H, H*_{aliph.}*), 1.39-1.33 (m, 4H, H_{aliph.}), 0,98-0.89 (m, 6H, *H_{aliph.}*).
**F-NMR** (282 MHz, CHCl₃): δ = -162,5 (dd, 1 F, J = 19,2 Hz, J = 1,9 Hz), - 164,3 (d, 1F, J = 19,2 Hz).
**MS** (EI): m/z(%) = 398 (100, M⁺), 301 (97).

### Beispiele 10 bis 86

Analog zu den Beispielen 1, 3 und 9 werden hergestellt

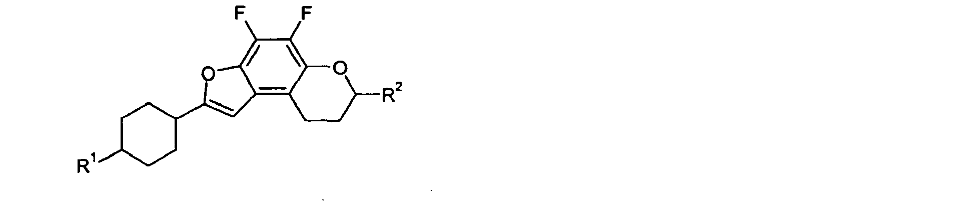

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 10 | CH₃ | CH₃ | |
| 11 | CH₃ | C₂H₅ | |
| 12 | CH₃ | *n-*C₃H₇ | |
| 13 | CH₃ | *n*-C₄H₉ | |
| 15 | CH₃ | *n-*C₅H₁₁ | |
| 16 | CH₃ | *n-*C₆H₁₃ | |
| 17 | CH₃ | *n-*C₇H₁₅ | |
| 18 | C₂H₅ | CH₃ | |
| 19 | C₂H₅ | C₂H₅ | |
| 20 | C₂H₅ | *n-*C₃H₇ | |
| 21 | C₂H₅ | *n-*C₄H₉ | |
| 22 | C₂H₅ | *n-*C₅H₁₁ | |
| 23 | C₂H₅ | *n-*C₆H₁₃ | |
| 24 | C₂H₅ | *n-*C₇H₁₅ | |
| 25 | *n-*C₃H₇ | CH₃ | |
| 26 | *n-*C₃H₇ | C₂H₅ | |
| 27 | *n-*C₃H₇ | *n-*C₃H₇ | |
| 28 | *n-*C₃H₇ | *n-*C₄H₉ | |
| 3 | *n-*C₃H₇ | *n-*C₅H₁₁ | K 56 N 62 I |
| 29 | *n-*C₃H₇ | *n-*C₆H₁₃ | |
| 30 | *n-*C₃H₇ | *n-*C₇H₁₅ | |
| 31 | *n*-C₄H₉ | CH₃ | |
| 32 | *n-*C₄H₉ | C₂H₅ | |
| 33 | *n-*C₄H₉ | *n-*C₃H₇ | |
| 34 | *n-*C₄H₉ | *n-*C₄H₉ | |
| 35 | *n-*C₄H₉ | *n-*C₅H₁₁ | |
| 36 | *n-*C₄H₉ | *n-*C₆H₁₃ | |
| 37 | *n-*C₄H₉ | *n-*C₇H₁₅ | |
| 38 | *n-*C₅H₁₁ | CH₃ | |
| 39 | *n-*C₅H₁₁ | C₂H₅ | |
| 40 | *n-*C₅H₁₁ | *n-*C₃H₇ | |
| 41 | *n-*C₅H₁₁ | *n-*C₄H₉ | |
| 42 | *n-*C₅H₁₁ | *n-*C₅H₁₁ | |
| 43 | *n-*C₅H₁₁ | *n-*C₆H₁₃ | |
| 44 | *n-*C₅H₁₁ *n-*C₅H₁₁ | *n-*C₇H₁₅ | |
| 45 | *n-*C₆H₁₃ | CH₃ | |
| 46 | *n-*C₆H₁₃ | C₂H₅ | |
| 47 | *n-*C₆H₁₃ | *n-*C₃H₇ | |
| 48 | *n-*C₆H₁₃ | *n-*C₄H₉ | |
| 49 | *n-*C₆H₁₃ | *n-*C₅H₁₁ | |
| 50 | *n-*C₆H₁₃ | *n-*C₆H₁₃ | |
| 51 | *n-*C₆H₁₃ | *n-*C₇H₁₅ | |
| 52 | *n-*C₇H₁₅ | CH₃ | |
| 53 | *n-*C₇H₁₅ | C₂H₅ | |
| 54 | *n-*C₇H₁₅ | *n-*C₃H₇ | |
| 55 | *n-*C₇H₁₅ | *n-*C₄H₉ | |
| 56 | *n-*C₇H₁₅ | *n-*C₅H₁₁ | |
| 57 | *n-*C₇H₁₅ | *n-*C₆H₁₃ | |
| 58 | *n-*C₇H₁₅ | *n-*C₇H₁₅ | |
| 59 | CH₃O | CH₃ | |
| 60 | CH₃O | C₂H₅ | |
| 61 | CH₃O | *n-*C₃H₇ | |
| 62 | CH₃O | *n-*C₄H₉ | |
| 63 | CH₃O | *n-*C₅H₁₁ | |
| 64 | CH₃O | *n-*C₆H₁₃ | |
| 65 | CH₃O | *n-*C₇H₁₅ | |
| 66 | C₂H₅O | CH₃ | |
| 67 | C₂H₅O | C₂H₅ | |
| 68 | C₂H₅O | *n-*C₃H₇ | |
| 69 | C₂H₅O | *n-*C₄H₉ | |
| 70 | C₂H₅O | *n-*C₅H₁₁ | |
| 71 | C₂H₅O | *n-*C₆H₁₃ | |
| 72 | C₂H₅O | *n-*C₇H₁₅ | |
| 73 | CH₂=CH | CH₃ | |
| 74 | CH₂=CH | C₂H₅ | |
| 75 | CH₂=CH | *n-*C₃H₇ | |
| 76 | CH₂=CH | *n-*C₄H₉ | |
| 77 | CH₂=CH | *n-*C₅H₁₁ | |
| 78 | CH₂=CH | n-C₆H₁₃ | |
| 79 | CH₂=CH | *n-*C₇H₁₅ | |
| 80 | CH₃-CH=CH | CH₃ | |
| 81 | CH₃-CH=CH | C₂H₅ | |
| 82 | CH₃-CH=CH | *n-*C₃H₇ | |
| 83 | CH₃-CH=CH | *n*-C₄H₉ | |
| 84 | CH₃-CH=CH | *n-*C₅H₁₁ | |
| 85 | CH₃-CH=CH | *n-*C₆H₁₃ | |
| 86 | CH₃-CH=CH | *n-*C₇H₁₅ | |

### Beispiele 87 bis 163

Analog zu den Beispielen 1, 3 und 9 werden hergestellt

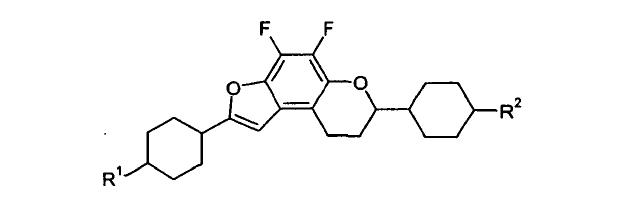

Bemerkung: * aus 10%-iger Lösung in ZLI-4792, bzw. ZLI-2857 (Δε) extrapolierte Werte.

| Nr. | R¹ | R² Phasensequenz T/°C |
|---|---|---|
| 87 | CH₃ | CH₃ |
| 88 | CH₃ | C₂H₅ |
| 89 | CH₃ | *n-*C₃H₇ |
| 90 | CH₃ | *n-*C₄H₉ |
| | | |
| 91 | CH₃ | *n-*C₅H₁₁ |
| 92 | CH₃ | *n-*C₆H₁₃ |
| 93 | CH₃ | *n-*C₇H₁₅ |
| 94 | C₂H₅ | CH₃ |
| 95 | C₂H₅ | C₂H₅ |
| 96 | C₂H₅ | *n-*C₃H₇ |
| 97 | C₂H₅ | *n-*C₄H₉ |
| 98 | C₂H₅ | *n-*C₅H₁₁ |
| 99 | C₂H₅ | *n-*C₆H₁₃ |
| 100 | C₂H₅ | *n-*C₇H₁₅ |
| 101 | *n-*C₃H₇ | CH₃ |
| 102 | *n-*C₃H₇ | C₂H₅ |
| 103 | *n-*C₃H₇ | *n-*C₃H₇ |
| 104 | *n-*C₃H₇ | *n-*C₄H₉ |
| 105 | *n-*C₃H₇ | *n-*C₅H₁₁ |
| 106 | *n-*C₃H₇ | *n-*C₆H₁₃ |
| 107 | *n-*C₃H₇ | *n-*C₇H₁₅ |
| 108 | *n-*C₄H₉ | CH₃ |
| 109 | *n-*C₄H₉ | C₂H₅ |
| 110 | *n-*C₄H₉ | *n-*C₃H₇ |
| 111 | *n-*C₄H₉ | *n-*C₄H₉ |
| 112 | *n-*C₄H₉ | *n-*C₅H₁₁ |
| 113 | *n-*C₄H₉ | *n-*C₆H₁₃ |
| 114 | *n-*C₄H₉ | *n-*C₇H₁₅ |
| 115 | *n-*C₅H₁₁ | CH₃ |
| 116 | *n-*C₅H₁₁ | C₂H₅ |
| 117 | *n-*C₅H₁₁ | *n-*C₃H₇ |
| 118 | *n-*C₅H₁₁ | *n-*C₄H₉ |
| 119 | *n-*C₅H₁₁ | *n-*C₅H₁₁ |
| 120 | *n-*C₅H₁₁ | *n-*C₆H₁₃ |
| 121 | *n-*C₅H₁₁ | *n-*C₇H₁₅ |
| 122 | *n-*C₆H₁₃ | CH₃ |
| 123 | *n-*C₆H₁₃ | C₂H₅ |
| 124 | *n-*C₆H₁₃ | *n-*C₃H₇ |
| 125 | *n-*C₆H₁₃ | *n-*C₄H₉ |
| 126 | *n*-C₆H₁₃ | *n-*C₅H₁₁ |
| 127 | *n-*C₆H₁₃ | *n-*C₆H₁₃ |
| 128 | *n-*C₆H₁₃ | *n-*C₇H₁₅ |
| 129 | *n-*C₇H₁₅ | CH₃ |
| 130 | *n-*C₇H₁₅ | C₂H₅ |
| 131 | *n-*C₇H₁₅ | *n-*C₃H₇ |
| 132 | *n-*C₇H₁₅ | *n-*C₄H₉ |
| 133 | *n-*C₇H₁₅ | *n-*C₅H₁₁ |
| 134 | *n-*C₇H₁₅ | *n*-C₆H₁₃ |
| 135 | *n-*C₇H₁₅ | *n-*C₇H₁₅ |
| 136 | CH₃O | CH₃ |
| 137 | CH₃O | C₂H₅ |
| 138 | CH₃O | *n-*C₃H₇ |
| 139 | CH₃O | *n-*C₄H₉ |
| 140 | CH₃O | *n-*C₅H₁₁ |
| 141 | CH₃O | *n-*C₆H₁₃ |
| 142 | CH₃O | *n-*C₇H₁₅ |
| 143 | C₂H₅O | CH₃ |
| 144 | C₂H₅O | C₂H₅ |
| 145 | C₂H₅O | *n-*C₃H₇ |
| 146 | C₂H₅O | *n-*C₄H₉ |
| 147 | C₂H₅O | *n-*C₅H₁₁ |
| 148 | C₂H₅O | *n-*C₆H₁₃ |
| 149 | C₂H₅O | *n-*C₇H₁₅ |
| 150 | CH₂=CH | CH₃ |
| 151 | CH₂=CH | C₂H₅ |
| 152 | CH₂=CH | *n-*C₃H₇ |
| 153 | CH₂=CH | *n-*C₄H₉ |
| 154 | CH₂=CH | *n-*C₅H₁₁ |
| 155 | CH₂=CH | *n-*C₆H₁₃ |
| 156 | CH₂=CH | *n*-C₇H₁₅ |
| 157 | CH₃-CH=CH | CH₃ |
| 158 | CH₃-CH=CH | C₂H₅ |
| 159 | CH₃-CH=CH | *n-*C₃H₇ |
| 160 | CH₃-CH=CH | *n-*C₄H₉ |
| 161 | CH₃-CH=CH | *n-*C₅H₁₁ |
| 162 | CH₃-CH=CH | *n-*C₆H₁₃ |
| 163 | CH₃-CH=CH | *n-*C₇H₁₅ |

### Beispiele 164 bis 239

Analog zu den Beispielen 2 und 4 werden hergestellt

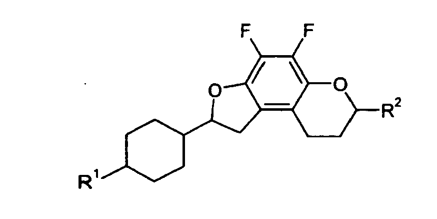

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 164 | CH₃ | CH₃ | |
| 165 | CH₃ | C₂H₅ | |
| 166 | CH₃ | *n-*C₃H₇ | |
| 167 | CH₃ | *n-*C₄H₉ | |
| 168 | CH₃ | *n-*C₅H₁₁ | |
| 169 | CH₃ | *n-*C₆H₁₃ | |
| 170 | CH₃ | *n-*C₇H₁₅ | |
| 171 | C₂H₅ | CH₃ | |
| 172 | C₂H₅ | C₂H₅ | |
| 173 | C₂H₅ | *n-*C₃H₇ | |
| 174 | C₂H₅ | *n-*C₄H₉ | |
| 175 | C₂H₅ | *n-*C₅H₁₁ | |
| 176 | C₂H₅ | *n-*C₆H₁₃ | |
| 177 | C₂H₅ | *n-*C₇H₁₅ | |
| 178 | *n-*C₃H₇ | CH₃ | |
| 179 | *n-*C₃H₇ | C₂H₅ | |
| 180 | *n*-C₃H₇ | *n-*C₃H₇ | |
| 181 | *n-*C₃H₇ | *n-*C₄H₉ | |
| 4 | *n-*C₃H₇ | *n-*C₅H₁₁ | K 101 I |
| 182 | *n-*C₃H₇ | *n*-C₆H₁₃ | |
| 183 | *n-*C₃H₇ | *n-*C₇H₁₅ | |
| 184 | *n-*C₄H₉ | CH₃ | |
| 185 | *n-*C₄H₉ | C₂H₅ | |
| 186 | *n-*C₄H₉ | *n-*C₃H₇ | |
| 187 | *n-*C₄H₉ | *n-*C₄H₉ | |
| 188 | *n-*C₄H₉ | *n-*C₅H₁₁ | |
| 189 | *n-*C₄H₉ | *n-*C₆H₁₃ | |
| 190 | *n-*C₄H₉ | *n-*C₇H₁₅ | |
| 191 | *n-*C₅H₁₁ | CH₃ | |
| 192 | *n-*C₅H₁₁ | C₂H₅ | |
| 193 | *n-*C₅H₁₁ | *n-*C₃H₇ | |
| 208 | *n-*C₅H₁₁ | *n-*C₄H₉ | |
| 209 | *n-*C₅H₁₁ | *n-*C₅H₁₁ | |
| 210 | *n-*C₅H₁₁ | *n-*C₆H₁₃ | |
| 211 | *n-*C₅H₁₁ | *n-*C₇H₁₅ | |
| 212 | *n-*C₆H₁₃ | CH₃ | |
| 213 | *n-*C₆H₁₃ | C₂H₅ | |
| 214 | *n-*C₆H₁₃ | *n-*C₃H₇ | |
| 215 | *n-*C₆H₁₃ | *n-*C₄H₉ | |
| 216 | *n-*C₆H₁₃ | *n-*C₅H₁₁ | |
| 217 | *n-*C₆H₁₃ | *n-*C₆H₁₃ | |
| 218. | *n-*C₆H₁₃ | *n-*C₇H₁₅ | |
| 219 | *n-*C₇H₁₅ | CH₃ | |
| 220 | *n-*C₇H₁₅ | C₂H₅ | |
| 221 | *n-*C₇H₁₅ | *n-*C₃H₇ | |
| 222 | *n-*C₇H₁₅ | *n-*C₄H₉ | |
| 223 | *n-*C₇H₁₅ | *n-*C₅H₁₁ | |
| 224 | *n-*C₇H₁₅ | *n-*C₆H₁₃ | |
| 225 | *n-*C₇H₁₅ | *n-*C₇H₁₅ | |
| 226 | CH₃O | CH₃ | |
| 227 | CH₃O | C₂H₅ | |
| 228 | CH₃O | *n-*C₃H₇ | |
| 229 | CH₃O | *n-*C₄H₉ | |
| 230 | CH₃O | *n-*C₅H₁₁ | |
| 231 | CH₃O | *n-*C₆H₁₃ | |
| 232 | CH₃O | *n*-C₇H₁₅ | |
| 233 | C₂H₅O | CH₃ | |
| 234 | C₂H₅O | C₂H₅ | |
| 235 | C₂H₅O | *n-*C₃H₇ | |
| 236 | C₂H₅O | *n-*C₄H₉ | |
| 237 | C₂H₅O | *n-*C₅H₁₁ | |
| 238 | C₂H₅O | *n-*C₆H₁₃ | |
| 239 | C₂H₅O | *n-*C₇H₁₅ | |

### Beispiele 240 bis 302

Analog zu den Beispielen 2 und 4 werden hergestellt

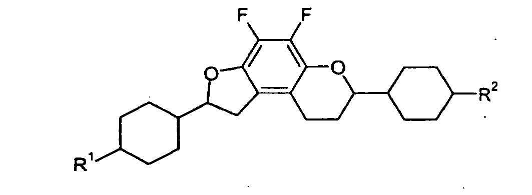

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 240 | CH₃ | CH₃ | |
| 241 | CH₃ | C₂H₅ | |
| 242 | CH₃ | *n-*C₃H₇ | |
| 243 | CH₃ | *n-*C₄H₉ | |
| 244 | CH₃ | *n-*C₅H₁₁ | |
| 245 | CH₃ | *n-*C₆H₁₃ | |
| 246 | CH₃ | *n-*C₇H₁₅ | |
| 247 | C₂H₅ | CH₃ | |
| 248 | C₂H₅ | C₂H₅ | |
| 249 | C₂H₅ | *n-*C₃H₇ | |
| 250 | C₂H₅ | *n-*C₄H₉ | |
| 251 | C₂H₅ | *n-*C₅H₁₁ | |
| 252 | C₂H₅ | *n-*C₆H₁₃ | |
| 253 | C₂H₅ | *n-*C₇H₁₅ | |
| 254 | *n-*C₃H₇ | CH₃ | |
| 255 | *n-*C₃H₇ | C₂H₅ | |
| 256 | *n-*C₃H₇ | *n-*C₃H₇ | |
| 257 | *n-*C₃H₇ | *n-*C₄H₉ | |
| 258 | *n-*C₃H₇ | *n-*C₅H₁₁ | |
| 259 | *n-*C₃H₇ | *n-*C₆H₁₃ | |
| 260 | *n-*C₃H₇ | *n-*C₇H₁₅ | |
| 261 | *n-*C₄H₉ | CH₃ | |
| 262 | *n-*C₄H₉ | C₂H₅ | |
| 263 | *n-*C₄H₉ | *n-*C₃H₇ | |
| 264 | *n-*C₄H₉ | *n-*C₄H₉ | |
| 265 | *n-*C₄H₉ | *n-*C₅H₁₁ | |
| 266 | *n-*C₄H₉ | *n-*C₆H₁₃ | |
| 267 | *n-*C₄H₉ | *n-*C₇H₁₅ | |
| 268 | *n-*C₅H₁₁ | CH₃ | |
| 269 | *n-*C₅H₁₁ | C₂H₅ | |
| 270 | *n-*C₅H₁₁ | *n-*C₃H₇ | |
| 271 | *n*-C₅H₁₁ | *n-*C₄H₉ | |
| 272 | *n-*C₅H₁₁ | *n-*C₅H₁₁ | |
| 273 | *n-*C₅H₁₁ | *n-*C₆H₁₃ | |
| 274 | *n-*C₅H₁₁ | *n-*C₇H₁₅ | |
| 275 | *n-*C₆H₁₃ | CH₃ | |
| 276 | *n-*C₆H₁₃ | C₂H₅ | |
| 277 | *n-*C₆H₁₃ | *n-*C₃H₇ | |
| 278 | *n-*C₆H₁₃ | *n-*C₄H₉ | |
| 279 | *n-*C₆H₁₃ | *n-*C₅H₁₁ | |
| 280 | *n-*C₆H₁₃ | *n-*C₆H₁₃ | |
| 281 | *n-*C₆H₁₃ | *n-*C₇H₁₅ | |
| 282 | *n-*C₇H₁₅ | CH₃ | |
| 283 | *n-*C₇H₁₅ | C₂H₅ | |
| 284 | *n-*C₇H₁₅ | *n-*C₃H₇ | |
| 285 | *n-*C₇H₁₅ | *n-*C₄H₉ | |
| 286 | *n-*C₇H₁₅ | *n-*C₅H₁₁ | |
| 287 | *n-*C₇H₁₅ | *n-*C₆H₁₃ | |
| 288 | *n-*C₇H₁₅ | *n-*C₇H₁₅ | |
| 289 | CH₃O | CH₃ | |
| 290 | CH₃O | C₂H₅ | |
| 291 | CH₃O | *n-*C₃H₇ | |
| 292 | CH₃O | *n-*C₄H₉ | |
| 293 | CH₃O | *n-*C₅H₁₁ | |
| 294 | CH₃O | *n-*C₆H₁₃ | |
| 295 | CH₃O | *n-*C₇H₁₅ | |
| 296 | C₂H₅O | CH₃ | |
| 297 | C₂H₅O | C₂H₅ | |
| 298 | C₂H₅O | *n-*C₃H₇ | |
| 299 | C₂H₅O | *n-*C₄H₉ | |
| 300 | C₂H₅O | *n-*C₅H₁₁ | |
| 300 | C₂H₅O | *n-*C₆H₁₃ | |
| 302 | C₂H₅O | *n-*C₇H₁₅ | |

### Beispiele 303 bis 307

Analog zu den Beispielen 5 und 7 werden hergestellt

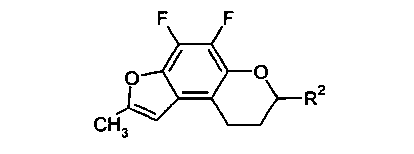

Bemerkung: * aus 10%-iger Lösung in ZLI-4792, bzw. ZLI-2857 (Δε) extrapolierte Werte.

| Nr. | R² | Phasensequenz T/°C |
|---|---|---|
| 303 | CH₃ | |
| 304 | C₂H₅ | |
| 5 | *n-*C₃H₇ | K 751 I |
| 305 | *n-*C₄H₉ | |
| 7 | *n-*C₅H₁₁ | K 61 I |
| 306 | *n-*C₆H₁₃ | |
| 307 | *n-*C₇H₁₅ | |

### Beispiele 308 bis 314

Analog zu den Beispielen 5 und 7 werden hergestellt

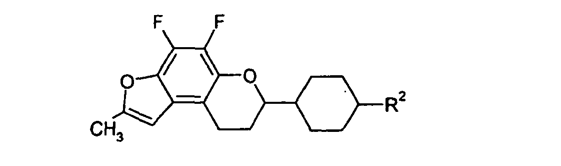

Bemerkung: * aus 10%-iger Lösung in ZLI-4792, bzw. ZLI-2857 (Δε) extrapolierte Werte.

| Nr. | R² | Phasensequenz T/°C |
|---|---|---|
| 308 | CH₃ | |
| 309 | C₂H₅ | |
| 310 | *n-*C₃H₇ | |
| 311 | *n-*C₄H₉ | |
| 312 | *n-*C₅H₁₁ | |
| 313 | *n-*C₆H₁₃ | |
| 314 | *n-*C₇H₁₅ | |

### Beispiele 315 bis 319

Analog zu den Beispielen 6 und 8 werden hergestellt

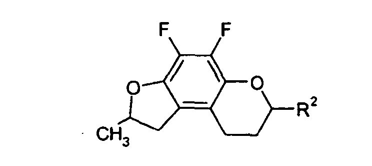

Bemerkung: * aus 10%-iger Lösung in ZLI-4792, bzw. ZLI-2857 (Δε) extrapolierte Werte.

| Nr. | R² | Phasensequenz T/°C |
|---|---|---|
| 315 | CH₃ | |
| 316 | C₂H₅ | |
| 6 | *n-*C₃H₇ | K 87 I |
| 317 | *n-*C₄H₉ | |
| 8 | *n-*C₅H₁₁ | K 76 I |
| 318 | *n-*C₆H₁₃ | |
| 319 | *n-*C₇H₁₅ | |

### Beispiele 320 bis 325

Analog zu den Beispielen 6 und 8 werden hergestellt

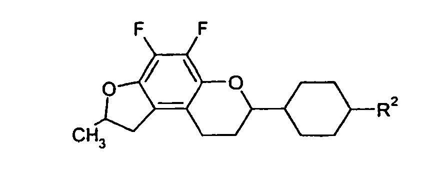

Bemerkung: * aus 10%-iger Lösung in ZLI-4792, bzw. ZLI-2857 (Δε) extrapolierte Werte.

| Nr. | R² | Phasensequenz T/°C |
|---|---|---|
| 320 | CH₃ | |
| 241 | C₂H₅ | |
| 321 | *n-*C₃H₇ | |
| 322 | *n-*C₄H₉ | |
| 323 | *n-*C₅H₁₁ | |
| 324 | *n-*C₆H₁₃ | |
| 325 | *n-*C₇H₁₅ | |

### Beispiele 326 bis 381

Analog zu den Beispielen 1, 3 und 9 werden hergestellt

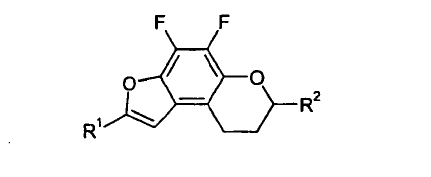

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 326 | C₂H₅ | CH₃ | |
| 327 | C₂H₅ | C₂H₅ | |
| 328 | C₂H₅ | *n-*C₃H₇ | |
| 329 | C₂H₅ | *n-*C₄H₉ | |
| 330 | C₂H₅ | *n-*C₅H₁₁ | |
| 331 | C₂H₅ | *n-*C₆H₁₃ | |
| 332 | C₂H₅ | *n-*C₇H₁₅ | |
| 333 | *n-*C₃H₇ | CH₃ | |
| 334 | *n-*C₃H₇ | C₂H₅ | |
| 335 | *n-*C₃H₇ | *n-*C₃H₇ | |
| 336 | *n-*C₃H₇ | *n-*C₄H₉ | |
| 337 | *n-*C₃H₇ | *n-*C₅H₁₁ | |
| 338 | *n-*C₃H₇ | *n-*C₆H₁₃ | |
| 339 | *n-*C₃H₇ | *n-*C₇H₁₅ | |
| 340 | *n-*C₄H₉ | CH₃ | |
| 341 | *n-*C₄H₉ | C₂H₅ | |
| 342 | *n-*C₄H₉ | *n-*C₃H₇ | |
| 343 | *n-*C₄H₉ | *n-*C₄H₉ | |
| 344 | *n-*C₄H₉ | *n-*C₅H₁₁ | |
| 345 | *n-*C₄H₉ | *n-*C₆H₁₃ | |
| 346 | *n-*C₄H₉ | *n-*C₇H₁₅ | |
| 347 | *n-*C₅H₁₁ | CH₃ | |
| 348 | *n-*C₅H₁₁ | C₂H₅ | |
| 349 | *n-*C₅H₁₁ | *n-*C₃H₇ | |
| 350 | *n-*C₅H₁₁ | *n-*C₄H₉ | |
| 351 | *n-*C₅H₁₁ | *n-*C₅H₁₁ | |
| 352 | *n-*C₅H₁₁ | *n-*C₆H₁₃ | |
| 353 | *n-*C₅H₁₁ | *n-*C₇H₁₅ | |
| 354 | *n-*C₆H₁₃ | CH₃ | |
| 355 | *n-*C₆H₁₃ | C₂H₅ | |
| 356 | n-C₆H₁₃ | *n-*C₃H₇ | |
| 357 | *n-*C₆H₁₃ | *n-*C₄H₉ | |
| 358 | *n-*C₆H₁₃ | *n*-C₅H₁₁ | |
| 359 | *n-*C₆H₁₃ | *n*-C₆H₁₃ | |
| 360 | *n-*C₆H₁₃ | *n-*C₇H₁₅ | |
| 361 | *n*-C₇H₁₅ | CH₃ | |
| 362 | *n-*C₇H₁₅ | C₂H₅ | |
| 363 | *n-*C₇H₁₅ | *n-*C₃H₇ | |
| 364 | *n-*C₇H₁₅ | *n-*C₄H₉ | |
| 365 | *n-*C₇H₁₅ | *n-*C₅H₁₁ | |
| 366 | *n-*C₇H₁₅ | *n-*C₆H₁₃ | |
| 367 | *n-*C₇H₁₅ | *n-*C₇H₁₅ | |
| 368 | CH₂=CH | CH₃ | |
| 369 | CH₂=CH | C₂H₅ | |
| 370 | CH₂=CH | *n-*C₃H₇ | |
| 371 | CH₂=CH | *n-*C₄H₉ | |
| 372 | CH₂=CH | *n-*C₅H₁₁ *n-*C₅H₁₁ | |
| 373 | CH₂=CH | *n-*C₆H₁₃ | |
| 374 | CH₂=CH | *n-*C₇H₁₅ | |
| 375 | CH₃-CH=CH | CH₃ | |
| 376 | CH₃-CH=CH | C₂H₅ | |
| 377 | CH₃-CH=CH | *n-*C₃H₇ | |
| 378 | CH₃-CH=CH | *n-*C₄H₉ | |
| 379 | CH₃-CH=CH | *n-*C₅H₁₁ | |
| 380 | CH₃-CH=CH | *n-*C₆H₁₃ | |
| 381 | CH₃-CH=CH | *n-*C₇H₁₅ | |

### Beispiele 382 bis 437

Analog zu den Beispielen 1, 3 und 9 werden hergestellt

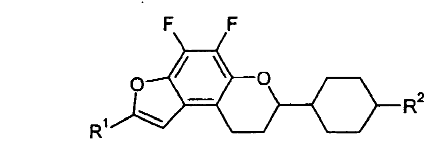

Bemerkung: * aus 10%-iger Lösung in ZLI-4792, bzw. ZLI-2857 (Δε) extrapolierte Werte.

| Nr. | R¹ | R² Phasensequenz T/°C |
|---|---|---|
| 382 | C₂H₅ | CH₃ |
| 383 | C₂H₅ | C₂H₅ |
| 394 | C₂H₅ | *n-*C₃H₇ |
| 395 | C₂H₅ | *n-*C₄H₉ |
| 396 | C₂H₅ | *n-*C₅H₁₁ |
| 397 | C₂H₅ | *n-*C₆H₁₃ |
| 398 | C₂H₅ | *n-*C₇H₁₅ |
| 399 | *n-*C₃H₇ | CH₃ |
| 390 | *n-*C₃H₇ | C₂H₅ |
| 391 | *n-*C₃H₇ | *n-*C₃H₇ |
| 392 | *n-*C₃H₇ | *n-*C₄H₉ |
| 393 | *n-*C₃H₇ | *n-*C₅H₁₁ |
| 394 | *n-*C₃H₇ | *n-*C₆H₁₃ |
| 395 | *n-*C₃H₇ | *n-*C₇H₁₅ |
| 396 | *n-*C₄H₉ | CH₃ |
| 397 | *n-*C₄H₉ | C₂H₅ |
| 398 | *n-*C₄H₉ | *n-*C₃H₇ |
| 399 | *n-*C₄H₉ | *n-*C₄H₉ |
| 400 | *n-*C₄H₉ | *n-*C₅H₁₁ |
| 401 | *n-*C₄H₉ | *n-*C₆H₁₃ |
| 402 | *n-*C₄H₉ | *n-*C₇H₁₅ |
| 403 | *n-*C₅H₁₁ | CH₃ |
| 404 | *n-*C₅H₁₁ | C₂H₅ |
| 405 | *n-*C₅H₁₁ | *n-*C₃H₇ |
| 406 | *n-*C₅H₁₁ | *n-*C₄H₉ |
| 407 | *n-*C₅H₁₁ | *n-*C₅H₁₁ |
| 408 | *n-*C₅H₁₁ | *n-*C₆H₁₃ |
| 409 | *n-*C₅H₁₁ | *n-*C₇H₁₅ |
| 410 | *n-*C₆H₁₃ | CH₃ |
| 411 | *n-*C₆H₁₃ | C₂H₅ |
| 412 | *n-*C₆H₁₃ | *n-*C₃H₇ |
| 413 | *n-*C₆H₁₃ | *n-*C₄H₉ |
| 414 | *n-*C₆H₁₃ | *n-*C₅H₁₁ |
| 415 | *n-*C₆H₁₃ | *n-*C₆H₁₃ |
| 416 | *n-*C₆H₁₃ | *n-*C₇H₁₅ |
| 417 | *n-*C₇H₁₅ | CH₃ |
| | | |
| 418 | *n-*C₇H₁₅ | C₂H₅ |
| 419 | *n-*C₇H₁₅ | *n-*C₃H₇ |
| 420 | *n-*C₇H₁₅ | *n-*C₄H₉ |
| 421 | *n-*C₇H₁₅ | *n-*C₅H₁₁ |
| 422 | *n-*C₇H₁₅ | *n-*C₆H₁₃ |
| 423 | *n-*C₇H₁₅ | *n-*C₇H₁₅ |
| 424 | CH₂=CH | CH₃ |
| 425 | CH₂=CH | C₂H₅ |
| 426 | CH₂=CH | *n-*C₃H₇ |
| 427 | CH₂=CH | *n-*C₄H₉ |
| 428 | CH₂=CH | *n-*C₅H₁₁ |
| 429 | CH₂=CH | *n-*C₆H₁₃ |
| 430 | CH₂=CH | *n-*C₇H₁₅ |
| 431 | CH₃-CH=CH | CH₃ |
| 432 | CH₃-CH=CH | C₂H₅ |
| 453 | CH₃-CH=CH | *n-*C₃H₇ |
| 434 | CH₃-CH=CH | *n-*C₄H₉ |
| 435 | CH₃-CH=CH | *n-*C₅H₁₁ |
| 436 | CH₃-CH=CH | *n-*C₆H₁₃ |
| 437 | CH₃-CH=CH | *n-*C₇H₁₅ |

### Beispiele 438 bis 480

Analog zu den Beispielen 2 und 4 werden hergestellt

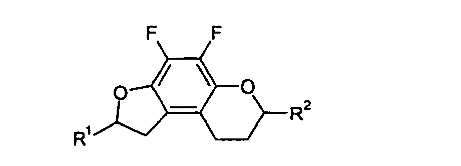

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 438 | CH₃ | *n-*C₇H₁₅ | |
| 439 | C₂H₅ | CH₃ | |
| 440 | C₂H₅ | C₂H₅ | |
| 441 | C₂H₅ | *n-*C₃H₇ | |
| 442 | C₂H₅ | *n-*C₄H₉ | |
| 443 | C₂H₅ | *n-*C₅H₁₁ | |
| 444 | C₂H₅ | *n-*C₆H₁₃ | |
| 445 | C₂H₅ | *n-*C₇H₁₅ | |
| 446 | *n-*C₃H₇ | CH₃ | |
| 447 | *n-*C₃H₇ | C₂H₅ | |
| 448 | *n-*C₃H₇ | *n-*C₃H₇ | |
| 449 | *n-*C₃H₇ | *n-*C₄H₉ | |
| 450 | *n-*C₃H₇ | *n-*C₅H₁₁ | |
| 451 | *n-*C₃H₇ | *n-*C₆H₁₃ | |
| 452 | *n-*C₃H₇ | *n-*C₇H₁₅ | |
| 453 | *n-*C₄H₉ | CH₃ | |
| 454 | *n-*C₄H₉ | C₂H₅ | |
| 455 | *n-*C₄H₉ | *n-*C₃H₇ | |
| 456 | *n-*C₄H₉ | *n-*C₄H₉ | |
| 457 | *n-*C₄H₉ | *n-*C₅H₁₁ | |
| 458 | *n-*C₄H₉ | *n-*C₆H₁₃ | |
| 459 | *n-*C₄H₉ | *n-*C₇H₁₅ | |
| 460 | *n-*C₅H₁₁ | CH₃ | |
| 461 | *n-*C₅H₁₁ | C₂H₅ | |
| 462 | *n-*C₅H₁₁ | *n-*C₃H₇ | |
| 463 | *n-*C₅H₁₁ | *n-*C₄H₉ | |
| 464 | *n-*C₅H₁₁ | *n-*C₅H₁₁ | |
| 465 | *n-*C₅H₁₁ | *n-*C₆H₁₃ | |
| 466 | *n-*C₅H₁₁ | *n-*C₇H₁₅ | |
| 467 | *n-*C₆H₁₃ | CH₃ | |
| 468 | *n-*C₆H₁₃ | C₂H₅ | |
| 469 | *n-*C₆H₁₃ | *n-*C₃H₇ | |
| 470 | *n-*C₆H₁₃ | *n-*C₄H₉ | |
| 471 | *n-*C₆H₁₃ | *n-*C₅H₁₁ | |
| 472 | *n-*C₆H₁₃ | *n-*C₆H₁₃ | |
| 473 | *n-*C₆H₁₃ | *n-*C₇H₁₅ | |
| 474 | *n-*C₇H₁₅ | CH₃ | |
| 475 | *n-*C₇H₁₅ | C₂H₅ | |
| 476 | *n-*C₇H₁₅ | *n-*C₃H₇ | |
| 477 | *n-*C₇H₁₅ | *n-*C₄H₉ | |
| 478 | *n-*C₇H₁₅ | *n-*C₅H₁₁ | |
| 479 | *n-*C₇H₁₅ | *n-*C₆H₁₃ | |
| 480 | *n-*C₇H₁₅ | *n-*C₇H₁₅ | |

### Beispiele 481 bis 523

Analog zu den Beispielen 2 und 4 werden hergestellt

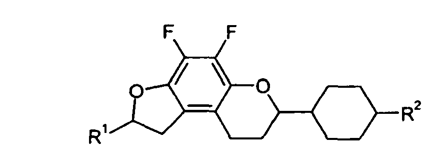

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 481 | CH₃ | *n-*C₇H₁₅ | |
| 482 | C₂H₅ | CH₃ | |
| 483 | C₂H₅ | C₂H₅ | |
| 484 | C₂H₅ | *n-*C₃H₇ | |
| 485 | C₂H₅ | *n-*C₄H₉ | |
| 486 | C₂H₅ | *n-*C₅H₁₁ | |
| 487 | C₂H₅ | *n-*C₆H₁₃ | |
| 488 | C₂H₅ | *n-*C₇H₁₅ | |
| 489 | *n-*C₃H₇ | CH₃ | |
| 490 | *n-*C₃H₇ | C₂H₅ | |
| 491 | n-C₃H₇ | *n-*C₃H₇ | |
| 492 | *n-*C₃H₇ | *n-*C₄H₉ | |
| 493 | *n-*C₃H₇ | *n-*C₅H₁₁ | |
| 494 | *n-*C₃H₇ | *n-*C₆H₁₃ | |
| 495 | *n-*C₃H₇ | *n-*C₇H₁₅ | |
| 496 | *n-*C₄H₉ | CH₃ | |
| 497 | *n-*C₄H₉ | C₂H₅ | |
| 498 | *n-*C₄H₉ | *n-*C₃H₇ | |
| 499 | *n-*C₄H₉ | *n-*C₄H₉ | |
| 500 | *n-*C₄H₉ | *n-*C₅H₁₁ | |
| 501 | *n-*C₄H₉ | *n-*C₆H₁₃ | |
| 502 | *n-*C₄H₉ | *n-*C₇H₁₅ | |
| 503 | *n-*C₅H₁₁ | CH₃ | |
| 504 | *n-*C₅H₁₁ | C₂H₅ | |
| 505 | *n-*C₅H₁₁ | *n-*C₃H₇ | |
| 586 | *n-*C₅H₁₁ | *n-*C₄H₉ | |
| 507 | *n-*C₅H₁₁ | *n-*C₅H₁₁ | |
| 508 | *n-*C₅H₁₁ | *n-*C₆H₁₃ | |
| 509 | *n-*C₅H₁₁ | *n-*C₇H₁₅ | |
| 510 | *n-*C₆H₁₃ | CH₃ | |
| 511 | *n-*C₆H₁₃ | C₂H₅ | |
| 512 | *n-*C₆H₁₃ | *n-*C₃H₇ | |
| 513 | *n-*C₆H₁₃ | *n-*C₄H₉ | |
| 514 | *n-*C₆H₁₃ | *n-*C₅H₁₁ | |
| 515 | *n-*C₆H₁₃ | *n-*C₆H₁₃ | |
| 516 | *n-*C₆H₁₃ | *n-*C₇H₁₅ | |
| 517 | *n-*C₇H₁₅ | CH₃ | |
| 518 | *n-*C₇H₁₅ | C₂H₅ | |
| 519 | *n-*C₇H₁₅ | *n-*C₃H₇ | |
| 520 | *n-*C₇H₁₅ | *n-*C₄H₉ | |
| 521 | *n-*C₇H₁₅ | *n-*C₅H₁₁ | |
| 522 | *n-*C₇H₁₅ | *n-*C₆H₁₃ | |
| 523 | *n-*C₇H₁₅ | *n-*C₇H₁₅ | |

### Beispiele 524 bis 599

Analog zu Beispiel 9 werden hergestellt

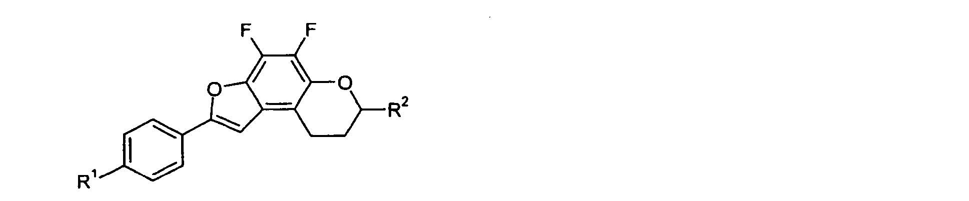

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 524 | CH₃ | CH₃ | |
| 525 | CH₃ | C₂H₅ | |
| 526 | CH₃ | *n-*C₃H₇ | |
| 527 | CH₃ | *n-*C₄H₉ | |
| 528 | CH₃ | *n-*C₅H₁₁ | |
| 529 | CH₃ | *n-*C₆H₁₃ | |
| 530 | CH₃ | *n-*C₇H₁₅ | |
| 531 | C₂H₅ | CH₃ | |
| 532 | C₂H₅ | C₂H₅ | |
| 533 | C₂H₅ | *n*-C₃H₇ | |
| 534 | C₂H₅ | *n-*C₄H₉ | |
| 535 | C₂H₅ | *n*-C₅H₁₁ | |
| 536 | C₂H₅ | *n-*C₆H₁₃ | |
| 537 | C₂H₅ | *n-*C₇H₁₅ | |
| 538 | *n-*C₃H₇ | CH₃ | |
| 539 | *n-*C₃H₇ | C₂H₅ | |
| 540 | *n-*C₃H₇ | *n-*C₃H₇ | |
| 541 | *n-*C₃H₇ | *n-*C₄H₉ | |
| 9 | *n-*C₃H₇ | *n-*C₅H₁₁ | T_{g} -31 K 70 N 79 I |
| 542 | *n-*C₃H₇ | *n-*C₆H₁₃ *n-*C₆H₁₃ | |
| 543 | *n-*C₃H₇ | *n-*C₇H₁₅ | |
| 544 | *n-*C₄H₉ | CH₃ | |
| 545 | *n-*C₄H₉ | C₂H₅ | |
| 546 | *n-*C₄H₉ | *n-*C₃H₇ | |
| 547 | *n-*C₄H₉ | *n-*C₄H₉ | |
| 548 | *n-*C₄H₉ | *n-*C₅H₁₁ | |
| 549 | *n-*C₄H₉ | *n-*C₆H₁₃ | |
| 550 | *n-*C₄H₉ | *n-*C₇H₁₅ | |
| 551 | *n-*C₅H₁₁ | CH₃ | |
| 552 | *n-*C₅H₁₁ | C₂H₅ | |
| 553 | *n-*C₅H₁₁ | *n-*C₃H₇ | |
| 554 | *n-*C₅H₁₁ | *n-*C₄H₉ | |
| 555 | *n-*C₅H₁₁ | *n-*C₅H₁₁ | |
| 556 | *n-*C₅H₁₁ | *n-*C₆H₁₃ | |
| 557 | *n-*C₅H₁₁ | *n-*C₇H₁₅ | |
| 558 | *n-*C₆H₁₃ | CH₃ | |
| 559 | *n-*C₆H₁₃ | C₂H₅ | |
| 560 | *n-*C₆H₁₃ | *n-*C₃H₇ | |
| 561 | *n-*C₆H₁₃ | *n-*C₄H₉ | |
| 562 | *n-*C₆H₁₃ | *n-*C₅H₁₁ | |
| 563 | *n-*C₆H₁₃ | *n-*C₆H₁₃ | |
| 564 | *n-*C₆H₁₃ | *n-*C₇H₁₅ | |
| 565 | *n-*C₇H₁₅ | CH₃ | |
| 566 | *n-*C₇H₁₅ | C₂H₅ | |
| 567 | *n-*C₇H₁₅ | *n-*C₃H₇ | |
| 568 | *n-*C₇H₁₅ | *n-*C₄H₉ | |
| 569 | *n-*C₇H₁₅ | *n-*C₅H₁₁ | |
| 570 | *n-*C₇H₁₅ | *n-*C₆H₁₃ | |
| 571 | *n-*C₇H₁₅ | *n-*C₇H₁₅ | |
| 572 | CH₃O | CH₃ | |
| 573 | CH₃O | C₂H₅ | |
| 574 | CH₃O | *n-*C₃H₇ | |
| 575 | CH₃O | *n-*C₄H₉ | |
| 576 | CH₃O | *n-*C₅H₁₁ | |
| 577 | CH₃O | *n-*C₆H₁₃ | |
| 578 | CH₃O | *n-*C₇H₁₅ | |
| 579 | C₂H₅O | CH₃ | |
| 580 | C₂H₅O | C₂H₅ | |
| 581 | C₂H₅O | *n-*C₃H₇ | |
| 582 | C₂H₅O | *n-*C₄H₉ | |
| 583 | C₂H₅O | *n-*C₅H₁₁ | |
| 584 | C₂H₅O | *n-*C₆H₁₃ | |
| 599 | C₂H₅O | *n-*C₇H₁₅ | |

### Beispiele 600 bis 676

Analog zu Beispiel 9 werden hergestellt

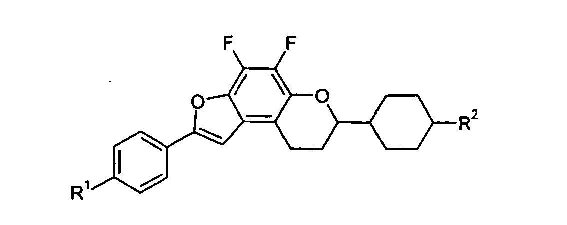

Bemerkung: * aus 10%-iger Lösung in ZLI-4792, bzw. ZLI-2857 (Δε) extrapolierte Werte.

| Nr. | R¹ | R² | Phasensequenz T/°C |
|---|---|---|---|
| 600 | CH₃ | CH₃ | |
| 601 | CH₃ | C₂H₅ | |
| 602 | CH₃ | *n-*C₃H₇ | |
| 603 | CH₃ | *n-*C₄H₉ | |
| 604 | CH₃ | *n-*C₅H₁₁ | |
| 605 | CH₃ | *n*-C₆H₁₃ | |
| 606 | CH₃ | *n-*C₇H₁₅ | |
| 607 | C₂H₅ | CH₃ | |
| 608 | C₂H₅ | C₂H₅ | |
| 609 | C₂H₅ | *n-*C₃H₇ | |
| 610 | C₂H₅ | *n-*C₄H₉ | |
| 611 | C₂H₅ | *n-*C₅H₁₁ | |
| 612 | C₂H₅ | *n-*C₆H₁₃ | |
| 613 | C₂H₅ | *n-*C₇H₁₅ | |
| 614 | *n-*C₃H₇ | CH₃ | |
| 615 | *n-*C₃H₇ | C₂H₅ | |
| 616 | *n-*C₃H₇ | *n-*C₃H₇ | |
| 617 | *n-*C₃H₇ | *n-*C₄H₉ | |
| 618 | *n-*C₃H₇ | *n-*C₅H₁₁ | |
| 619 | *n-*C₃H₇ | *n-*C₆H₁₃ | |
| 620 | *n-*C₃H₇ | *n-*C₇H₁₅ | |
| 621 | *n-*C₄H₉ | CH₃ | |
| 622 | *n-*C₄H₉ | C₂H₅ | |
| 623 | *n-*C₄H₉ | *n-*C₃H₇ | |
| 624 | *n-*C₄H₉ | *n-*C₄H₉ | |
| 625 | *n-*C₄H₉ | *n-*C₅H₁₁ | |
| 626 | *n-*C₄H₉ | *n-*C₆H₁₃ | |
| 627 | *n-*C₄H₉ | *n-*C₇H₁₅ | |
| 628 | *n-*C₅H₁₁ | CH₃ | |
| 629 | *n-*C₅H₁₁ | C₂H₅ | |
| 630 | *n-*C₅H₁₁ | *n-*C₃H₇ | |
| 631 | *n-*C₅H₁₁ | *n-*C₄H₉ | |
| 632 | *n-*C₅H₁₁ | *n-*C₅H₁₁ | |
| 633 | *n-*C₅H₁₁ | *n-*C₆H₁₃ | |
| 634 | *n-*C₅H₁₁ | *n-*C₇H₁₅ | |
| 635 | *n-*C₆H₁₃ | CH₃ | |
| 636 | *n-*C₆H₁₃ | C₂H₅ | |
| 637 | *n-*C₆H₁₃ | *n-*C₃H₇ | |
| 638 | *n-*C₆H₁₃ | *n-*C₄H₉ | |
| 639 | *n-*C₆H₁₃ | *n-*C₅H₁₁ | |
| 640 | *n-*C₆H₁₃ | *n-*C₆H₁₃ | |
| 641 | *n-*C₆H₁₃ | *n-*C₇H₁₅ | |
| 642 | *n-*C₇H₁₅ | CH₃ | |
| 643 | *n-*C₇H₁₅ | C₂H₅ | |
| 644 | *n-*C₇H₁₅ | *n-*C₃H₇ | |
| 645 | *n-*C₇H₁₅ | *n-*C₄H₉ | |
| 646 | *n-*C₇H₁₅ | *n-*C₅H₁₁ | |
| 677 | *n-*C₇H₁₅ | *n-*C₆H₁₃ | |
| 648 | *n-*C₇H₁₅ | *n-*C₇H₁₅ | |
| 649 | CH₃O | CH₃ | |
| 650 | CH₃O | C₂H₅ *n-*C₃H₇ | |
| 651 | CH₃O | | |
| 652 | CH₃O | *n-*C₄H₉ | |
| 653 | CH₃O | *n-*C₅H₁₁ | |
| 654 | CH₃O | *n-*C₆H₁₃ | |
| 669 | CH₃O | *n-*C₇H₁₅ | |
| 670 | C₂H₅O | CH₃ | |
| 671 | C₂H₅O | C₂H₅ | |
| 672 | C₂H₅O | *n-*C₃H₇ | |
| 673 | C₂H₅O | *n-*C₄H₉ | |
| 674 | C₂H₅O | *n-*C₅H₁₁ | |
| 675 | C₂H₅O | *n-*C₆H₁₃ | |
| 676 | C₂H₅O | *n*-C₇H₁₅ | |

## Patentansprüche

1. Verbindung der Formel I 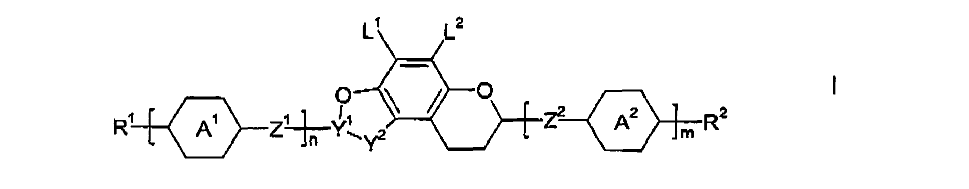 worin
R¹ und R² jeweils unabhängig voneinander H, Halogen, -CN, -SCN, -SF₅, -CF₃, -CHF₂, -CH₂F, -OCF₃, -OCHF₂ oder eine Alkylgruppe mit 1 bis 15 C-Atomen, die optional einfach durch CN oder CF₃ oder mindestens einfach durch Halogen substituiert sein kann, wobei jeweils auch eine oder mehrere CH₂-Gruppen, jeweils unabhängig voneinander, durch -O-, -S-, -CH=CH-, -CF=CF-, -CF=CH-, -CH=CF-, 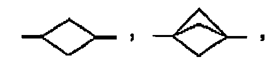 -CO-, -CO-O-, -O-CO- oder -O-CO-O- so ersetzt sein können, dass weder O-noch S-Atome direkt miteinander verknüpft sind,
>Y¹-Y²- >C=CH- oder >CH-CH₂-
L¹ und L² F, 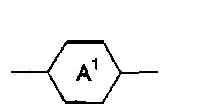
und 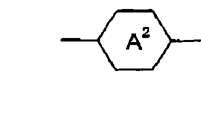 jeweils unabhängig voneinander und wenn mehrfach vorhanden auch diese unabhängig voneinander,
(a) einen trans-1,4-Cyclohexylenrest, worin auch eine oder zwei nicht benachbarte CH₂-Gruppen durch -O- und/oder -S-ersetzt sein können,
(b) einen 1,4-Cyclohexenylenrest,
(c) einen 1,4-Phenylenrest, worin auch eine oder zwei nicht benachbarte CH-Gruppen durch N ersetzt sein können,
(d) einen Rest ausgewählt aus der Gruppe Naphthalin-2,6-diyl, Decahydronaphthalin-2,6-diyl und 1,2,3,4-Tetrahydronaphthalin-2,6-diyl, oder
(e) einen Rest ausgewählt aus der Gruppe 1,4-Bicyclo-[2,2,2]-octylen, 1,3-Bicyclo-[1,1,1]-pentylen und Spiro-[3,3]-heptan-2,6-diyl
wobei in
(a) und (b) eine oder mehrere -CH₂- Gruppen, unabhängig voneinander, jeweils durch eine -CHF- oder eine -CF₂-Gruppe ersetzt sein können und in
(c) und (d) eine oder mehrere -CH= Gruppen, unabhängig voneinander, jeweils durch eine Gruppe ausgewählt aus der Gruppe -CF=, -CCI=, -CBr=, -C(CN)=, -C(CH₃)=, -C(CH₂F)=, -C(CHF₂)=, -C(OCH₃)=, -C(OCHF₂)= und -C(OCF₃)= ersetzt sein können
Z¹ und Z² jeweils unabhängig voneinander und, wenn mehrfach vorhanden, auch diese unabhängig voneinander, eine Einfachbindung, -CH₂-CH₂-, -CF₂-CH₂-, -CH₂-CF₂-, -CF₂-CF₂-, -CH=CH-, -CF=CF-, -CF=CH-, -CH=CF-, -C≡C-, -COO-, -OCO-, -CH₂O-, -OCH₂-, -CF₂O-, -OCF₂-, oder eine Kombination von zweien dieser Gruppen, wobei keine zwei O-Atome miteinander verbunden sind, und
n und m jeweils 0, 1, 2 oder 3, wobei
(n + m) 0, 1, 2 oder 3,
bedeuten.

2. Verbindung der Formel I nach Anspruch 1, ausgewählt aus der Gruppe der Verbindungen der Formeln IA und IB 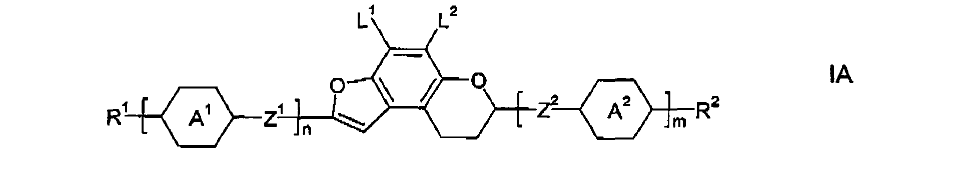 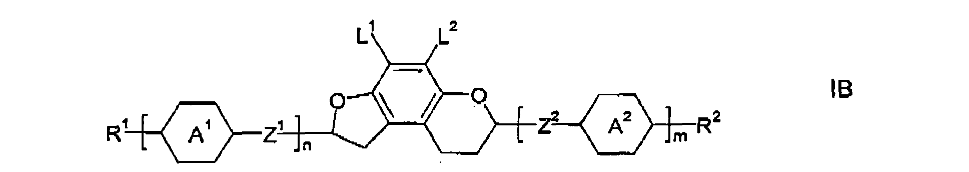 worin die Parameter die in Anspruch 1 gegebenen Bedeutungen haben.

3. Verbindung nach Anspruch 1 oder 2, **dadurch gekennzeichnet, dass** Z¹ und Z² eine Einfachbindung bedeuten.

4. Verbindung nach einem oder mehreren der Ansprüche 1 bis 3, **dadurch gekennzeichnet, dass** (m + n) 0 oder 1 bedeutet.

5. Flüssigkristallmedium, **dadurch gekennzeichnet, dass** es eine oder mehrere Verbindungen der Formel I, nach einem oder mehreren der Ansprüche 1 bis 4, enthält.

6. Flüssigkristallmedium nach Anspruch 5, **dadurch gekennzeichnet, dass** es eine nematische Phase aufweist.

7. Flüssigkristallmedium nach Anspruch 5 oder 6, **dadurch gekennzeichnet, dass** es eine oder mehrere dielektrisch negative Verbindungen der Formel II 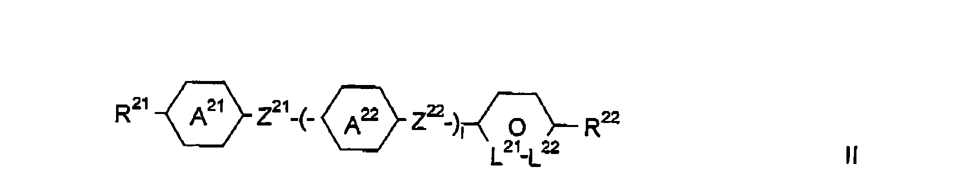 worin
R²¹ und R²² jeweils unabhängig voneinander die in Anspruch 1 bei Formel I für R¹ gegebene Bedeutung haben,
Z²¹ und Z²² jeweils unabhängig voneinander die in Anspruch 1 bei Formel I für Z¹ gegebene Bedeutung haben, 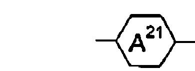
und 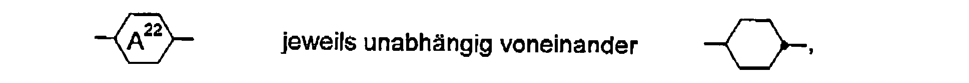 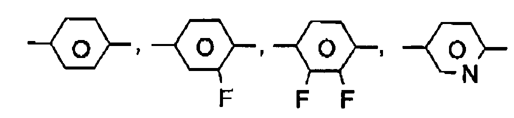 oder 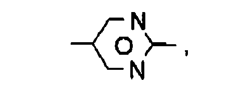
L²¹ und L²² beide C-F oder eines von beiden N und das andere C-F, und
I 0 oder 1
bedeuten, enthält.

8. Flüssigkristallmedium nach einem oder mehreren der Ansprüche 5 bis 7, **dadurch gekennzeichnet, dass** es eine oder mehrere Verbindungen der Formel II-1 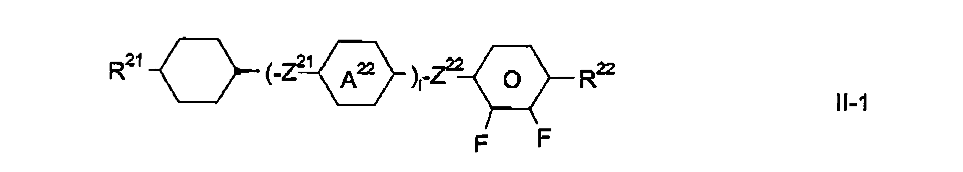 worin R²¹, R²², Z²¹, Z²², 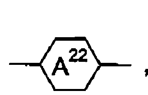 und I die in Anspruch 8 gegebenen Bedeutungen haben, enthält.

9. Verwendung eines Flüssigkristallmediums nach einem oder mehreren der Ansprüche 5 bis 8 in einer elektrooptischen Anzeige.

10. Elektrooptische Anzeige enthaltend ein Flüssigkristallmedium nach einem oder mehreren der Ansprüche 5 bis 8.

11. Anzeige nach Anspruch 10, **dadurch gekennzeichnet, dass** es sich um ein VAN LCD handelt.

12. Verfahren zur Herstellung eines Flüssigkristallmediums nach einem oder mehreren der Ansprüche 5 bis 8, **dadurch gekennzeichnet, dass** eine oder mehrere Verbindungen der Formel I, nach einem oder mehreren der Ansprüche 1 bis 4, mit einer oder mehreren weiteren Verbindungen gemischt wird.

13. Verfahren zur Herstellung einer elektrooptischen Anzeige, **dadurch gekennzeichnet, dass** ein Flüssigkristallmedium wie in einem oder mehreren der Ansprüche der Ansprüche 5 bis 8 definiert zwischen zwei Substrate eingebracht wird.

## Claims

1. Compound of the formula I 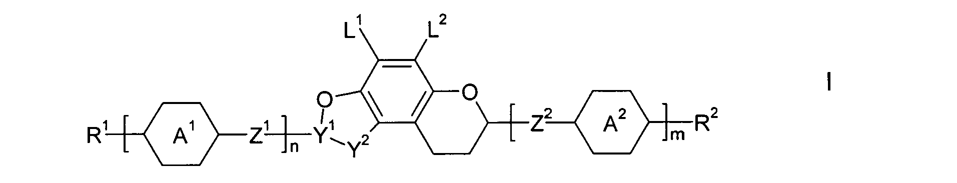 in which
R¹ and R² each, independently of one another, denote H, halo- gen, -CN, -SCN, -SF₅, -CF₃, -CHF₂, -CH₂F, -OCF₃, -OCHF₂ or an alkyl group having 1 to 15 C atoms, which may optionally be monosubstituted by CN or CF₃ or at least monosubstituted by halogen and in which one or more CH₂ groups, in each case inde- pendently of one another, may each be replaced by -O-, -S-, -CH=CH-, -CF=CF-, -CF=CH-, -CH=CF-, 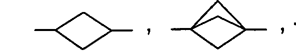 -CO-, -CO-O-, -O-CO- or -O-CO-O- in such a way that neither O nor S atoms are linked directly to one another,
>Y¹-Y²- denotes >C=CH- or >CH-CH₂-,
L¹ and L² denote F, 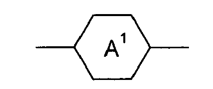
and 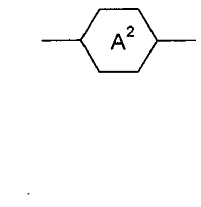 each, independently of one another, and, if present more than once, these also independently of one another, denote
(a) a trans-1,4-cyclohexylene radical, in which, in addition, one or two non-adjacent CH₂ groups may be replaced by -O- and/or -S-,
(b) a 1,4-cyclohexenylene radical,
(c) a 1,4-phenylene radical, in which, in addition, one or two non-adjacent CH groups may be replaced by N,
(d) a radical selected from the group naphthalene-2,6-diyl, decahydronaphthalene-2,6-diyl and 1,2,3,4-tetrahydronaphthalene-2,6-diyl, or
(e) a radical selected from the group 1,4-bicyclo[2.2.2]octylene, 1,3-bicyclo[1.1.1]pentylene and spiro[3.3]heptane-2,6-diyl, where in
(a) and (b), one or more -CH₂- groups, independently of one another, may each be replaced by a -CHF- or a -CF₂- group, and in
(c) and (d), one or more -CH= groups, independently of one another, may each be replaced by a group selected from the group -CF=, -CCl=, -CBr=, -C(CN)=, -C(CH₃)=, -C(CH₂F)=, -C(CHF₂)=, -C(OCH₃)=, -C(OCHF₂)= and -C(OCF₃)=,
Z¹ and Z² each, independently of one another, and, if present more than once, these also independently of one another, denote a single bond, -CH₂-CH₂-, -CF₂-CH₂-, -CH₂-CF₂-, -CF₂-CF₂-, -CH=CH-, -CF=CF-, -CF=CH-, -CH=CF-, -C=C-, -COO-, -OCO-, -CH₂O-, -OCH₂-, -CF₂O-, -OCF₂-, or a combination of two of these groups, where no two O atoms are bonded to one another, and
n and m each denote 0, 1, 2 or 3, where
(n + m) denotes 0, 1, 2 or 3.

2. Compound of the formula I according to Claim 1, selected from the group of the compounds of the formulae IA and IB 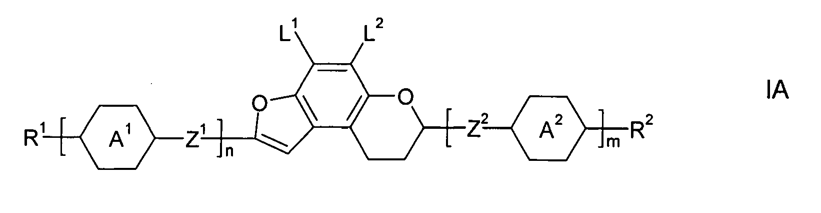 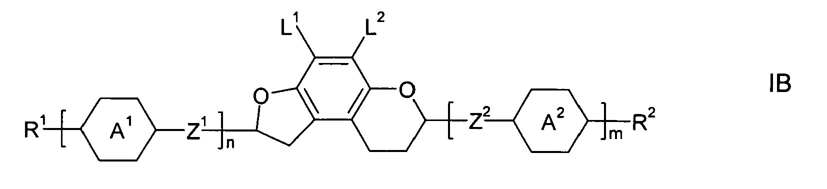 in which the parameters have the meanings given in Claim 1.

3. Compound according to Claim 1 or 2, **characterised in that** Z¹ and Z² denote a single bond.

4. Compound according to one or more of Claims 1 to 3, **characterised in that** (m + n) denotes 0 or 1.

5. Liquid-crystal medium, **characterised in that** it comprises one or more compounds of the formula I according to one or more of Claims 1 to 4.

6. Liquid-crystal medium according to Claim 5, **characterised in that** it has a nematic phase.

7. Liquid-crystal medium according to Claim 5 or 6, **characterised in that** it comprises one or more dielectrically negative compounds of the formula II 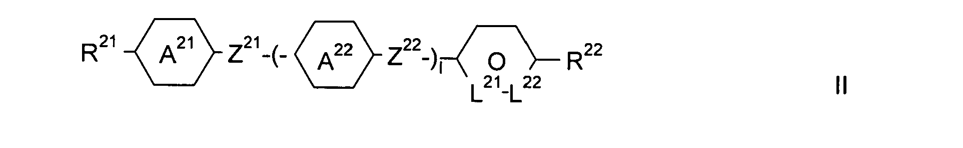 in which
R²¹ and R²² each, independently of one another, have the mean- ing given for R¹ under formula I in Claim 1,
Z²¹ and Z²² each, independently of one another, have the mean- ing given for Z¹ under formula I in Claim 1, 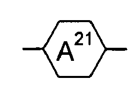 and 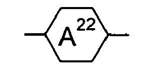 each, independently of one another, denote 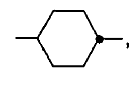 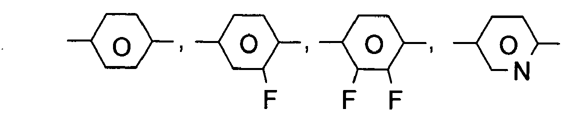 or 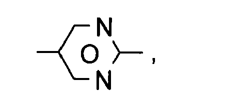
L²¹ and L²² both denote C-F or one of the two denotes N and the other denotes C-F, and
I denotes 0 or 1.

8. Liquid-crystal medium according to one or more of Claims 5 to 7, **characterised in that** it comprises one or more compounds of the formula II-1 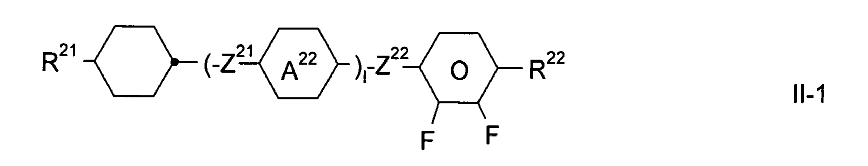 in which R²¹, R²², Z²¹, Z²², 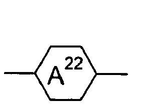 and I have the meanings given in Claim 7.

9. Use of a liquid-crystal medium according to one or more of Claims 5 to 8 in an electro-optical display.

10. Electro-optical display containing a liquid-crystal medium according to one or more of Claims 5 to 8.

11. Display according to Claim 10, **characterised in that** it is a VAN LCD.

12. Process for the preparation of a liquid-crystal medium according to one or more of Claims 5 to 8, **characterised in that** one or more compounds of the formula I according to one or more of Claims 1 to 4 are mixed with one or more further compounds.

13. Process for the production of an electro-optical display, **characterised in that** a liquid-crystal medium as defined in one or more of Claims 5 to 8 is introduced between two substrates.

## Revendications

1. Composé de la formule I 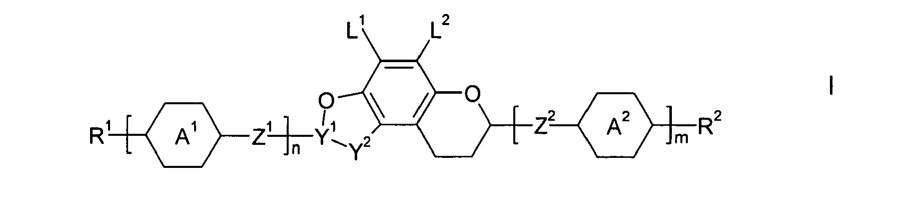 dans laquelle
R¹ et R² représentent, chacun indépendamment de l'autre, H, halogène, -CN, -SCN, -SF₅, -CF₃, -CHF₂, -CH₂F, -OCF₃, -OCHF₂ ou un groupe alkyle comportant de 1 à 15 atomes de C, lequel peut en option être mono- substitué par CN ou CF₃ ou au moins monosubstitué par halogène et où un ou plusieurs groupes CH₂, dans chaque cas indépendamment les uns des autres, peuvent chacun être remplacés par -O-, -S-, -CH=CH-, -CF=CF-, -CF=CH-, -CH=CF-, 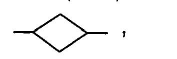 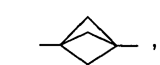 -CO-, -CO-O-, -O-CO- ou -O-CO-O- de telle sorte que ni des atomes de O, ni des atomes de S ne soient liés directement les uns aux autres,
>Y¹-Y²- représente >C=CH- ou >CH-CH₂-,
L¹ et L² représentent F, 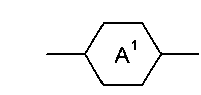
et 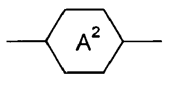 représentent, chacun indépendamment de l'autre, et, si présents plus d'une fois, ceux-ci également indépendamment les unes des autres représentent :
(a) un radical trans-1,4-cyclohexylène, où, en addition, un ou deux groupes CH₂ non adjacents peuvent être remplacés par -O- et/ou -S-,
(b) un radical 1,4-cyclohexénylène,
(c) un radical 1,4-phénylène, où, en addition, un ou deux groupes CH non adjacents peuvent être remplacés par N,
(d) un radical choisi parmi le groupe naphtalène-2,6-diyle, décahydronaphtalène-2,6-diyle et 1,2,3,4-tétrahydronaphtalène-2,6-diyle, ou
(e) un radical choisi parmi le groupe 1,4-bicyclo[2.2.2]octylène, 1,3-bicyclo[1.1.1]pentylène et spiro[3.3]heptane-2,6-diyle, dans lequel dans :
a) et (b), un ou plusieurs groupes -CH₂-, indépendamment les uns des autres, peuvent chacun être remplacés par un groupe -CHF- ou un groupe -CF₂-, et dans :
(c) et (d), un ou plusieurs groupes -CH=, indépendamment les uns des autres, peuvent chacun être remplacés par un groupe choisi parmi le groupe -CF=, -CCI=, -CBr=, -C(CN)=, -C(CH₃)=, -C(CH₂F)=, -C(CHF₂)=, -C(OCH₃)=, -C(OCHF₂)= et -C(OCF₃)=,
Z¹ et Z² représentent, chacun indépendamment de l'autre, et, si présents plus d'une fois, ceux-ci représentent, également indépendamment les unes des autres, une liaison simple, -CH₂-CH₂-, -CF₂-CH₂-, -CH₂-CF₂-, -CF₂-CF₂-, -CH=CH-, -CF=CF-, -CF=CH-, -CH=CF-, -C=C-, -COO-, -OCO-, -CH₂O-, -OCH₂-, -CF₂O-, -OCF₂-, ou une combinaison de deux de ces groupes, où deux atomes de O ne sont pas liés l'un à l'autre, et
n et m représentent chacun 0, 1, 2 ou 3, où
(n + m) représente 0, 1, 2 ou 3.

2. Composé de la formule I selon la revendication 1, choisi parmi le groupe des composés des formules IA et IB 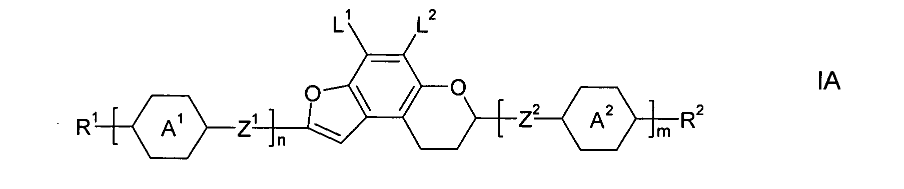 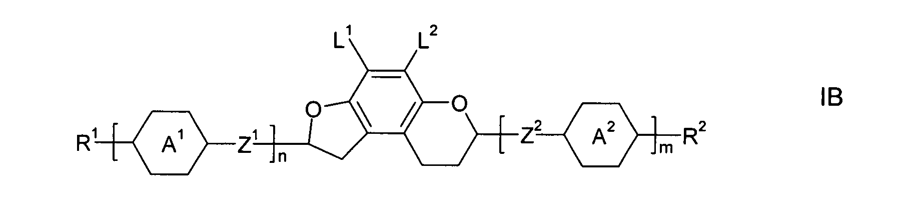 dans lesquelles les paramètres présentent les significations données dans la revendication 1.

3. Composé selon la revendication 1 ou 2, **caractérisé en ce que** Z¹ et Z² représentent une liaison simple.

4. Composé selon une ou plusieurs des revendications 1 à 3, **caractérisé en ce que** (m + n) représente 0 ou 1.

5. Milieu cristallin liquide, **caractérisé en ce qu'**il comprend un ou plusieurs composés de la formule I selon une ou plusieurs des revendications 1 à 4.

6. Milieu cristallin liquide selon la revendication 5, **caractérisé en ce qu'**il présente une phase nématique.

7. Milieu cristallin liquide selon la revendication 5 ou 6, **caractérisé en ce qu'**il comprend un ou plusieurs composés diélectriquement négatifs de la formule II 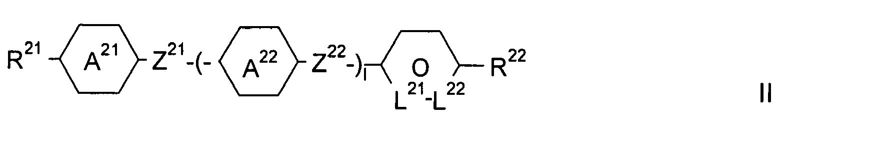 dans laquelle
R²¹ et R²² présentent, chacun indépendamment de l'autre, la signification donnée pour R¹ sous la formule I selon la revendication 1,
Z²¹ et Z²² présentent, chacun indépendamment de l'autre, la signification donnée pour Z¹ sous la formule I selon la revendication 1, 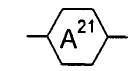 and 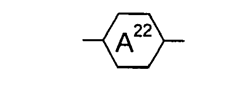 représentent, chacun indépendamment de l'autre, 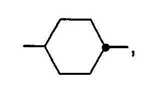 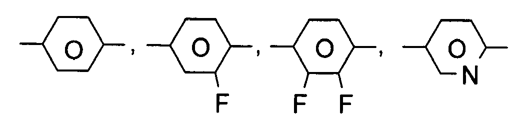 or 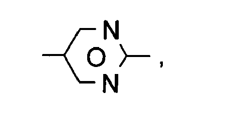
L²¹ et L²² représentent tous deux C-F ou l'un des deux repré- sente N et l'autre représente C-F, et
I représente 0 ou 1.

8. Milieu cristallin liquide selon une ou plusieurs des revendications 5 à 7, **caractérisé en ce qu'**il comprend un ou plusieurs composés de la formule II-1 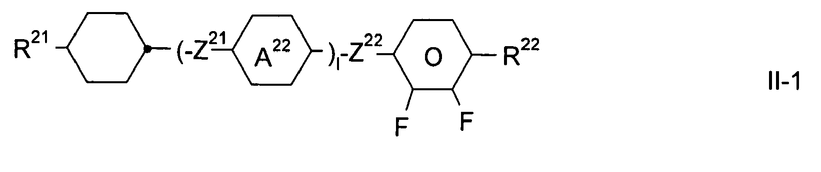 dans laquelle R²¹, R²², Z²¹, Z²², 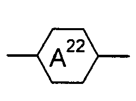 et I présentent les significations données dans la revendication 7.

9. Utilisation d'un milieu cristallin liquide selon une ou plusieurs des revendications 5 à 8 dans un affichage électro-optique.

10. Affichage électro-optique contenant un milieu cristallin liquide selon une ou plusieurs des revendications 5 à 8.

11. Affichage selon la revendication 10, **caractérisé en ce qu'**il s'agit d'un VAN LCD.

12. Procédé pour la préparation d'un milieu cristallin liquide selon une ou plusieurs des revendications 5 à 8, **caractérisé en ce qu'**un ou plusieurs composés de la formule I selon une ou plusieurs des revendications 1 à 4 sont mélangés avec un ou plusieurs autres composés.

13. Procédé pour la production d'un affichage électro-optique, **caractérisé en ce qu'**un milieu cristallin liquide comme défini selon une ou plusieurs des revendications 5 à 8 est introduit entre deux substrats.
